# EUROPEAN PATENT APPLICATION

(11) **EP 4 227 309 A1**
(43) Date of publication of application: **16.08.2023**
(21) Application number: 21879304.0
(22) Date of filing: 09.10.2021
(51) Int. Cl.: C07D 491/22, A61K 31/4375, A61K 47/68, A61P 35/00

(54) **DEUTERATED CAMPTOTHECIN DERIVATIVE AND ANTIBODY-DRUG CONJUGATE THEREOF**

(30) Priority: 12.10.2020 CN 202011086104
(71) Applicant: Sichuan Baili Pharmaceutical Co. Ltd., Chengdu, Sichuan 611130 (CN); Baili-Bio (Chengdu) Pharmaceutical Co., Ltd., ChengDu, Sichuan 611130 (CN)
(72) Inventor: ZHU, Yi, Chengdu, Sichuan 611130 (CN); WAN, Weili, Chengdu, Sichuan 611130 (CN); ZHUO, Shi, Chengdu, Sichuan 611130 (CN); YU, Tianzi, Chengdu, Sichuan 611130 (CN); ZHU, Guili, Chengdu, Sichuan 611130 (CN); YANG, Xiujuan, Chengdu, Sichuan 611130 (CN)
(74) Representative: Haile, Alison Victoria
(86) International application number: PCT/CN2021/122813
(87) International publication number: WO 2022/078259

(57) **Abstract**

The invention discloses a deuterated camptothecin derivative and its antibody-drug conjugate (ADC), and combines the deuteration technology with camptothecin ADC to discover improved deuterated camptothecin ADC drug, so that it has higher safety and efficacy and can better meet the clinical challenge.

## Description

### Technical Field

The present invention relates to deuterated camptothecin derivatives and antibody-drug conjugates thereof.

### Background

As a novel type of targeted drug, an antibody-drug conjugate (ADC) generally comprises three parts: an antibody or antibody-like ligand, a small molecule drug, and a linking unit that connects the ligand and the drug. Antibody-drug conjugates use the specific recognition of antigens by antibodies, transport drug molecules to the vicinity of target cells and effectively release drug molecules to achieve therapeutic purposes. In August 2011, the U.S. Food and Drug Administration (FDA) approved ADECTEIS^{®}, a new ADC drug developed by Seattle Genetics for the treatment of Hodgkin lymphoma and recurrent degenerative large cell lymphoma (ALCL), and clinical application has demonstrated the safety and efficacy of such drugs.

Antibody-drug conjugate (ADC) drugs have the advantages of increasing water solubility, improving targeting, specific binding of antibodies to antigens, carrying the drug to the surrounding target cells, and effectively killing cells by releasing the drug near the target cells, reducing toxic side effects. Camptothecin drugs have considerable application prospects in ADC drugs. Currently, the antibody-conjugated drug trastuzumab deruxtecan (trade name Enhertu) with eatecan as the toxin has been approved for marketing by the US FDA on December 20, 2019, as the first marketed camptothecin-type ADC drug, it has well proved the drug-making ability and application prospect of this type of drug in the field of ADC.

The camptothecin family of drugs including irinotecan, eatecan, and SN38, etc., are known as antitumor small molecule compounds and inhibit DNA topoisomerase I to exhibit antitumor effects. Camptothecin drugs have been used widely in clinical practice, the main indications are bone cancer, prostate cancer, breast cancer, pancreatic cancer and so on. Unlike irinotecan, which is currently used clinically, eatecan does not require activation by utilizing enzymes. In addition, compared with SN-38, which is the pharmacodynamic body of irinotecan, and topotecan, which is also used in clinical practice, the inhibitory activity to topoisomerase I is stronger and there is more cell-wounding activity against a variety of cancer cells *in vitro.* In particular, it has also shown an effect on cancer cells that show resistance to SN-38 through the expression of P-glycoprotein. Eatecan has not been successfully marketed as a chemotherapy agent alone, due to its relative high cell activity, resulting in a narrow therapeutic window. At the same time, camptothecin drugs have a short half-life in plasma, and maintaining their efficacy in clinical use requires an increase in dosage or frequency of administration, which may cause tolerance problems for patients.

Deuterium is a stable non-radioactive isotope of hydrogen in nature. Due to its greater atomic mass than hydrogen, the C-D bond is more stable than the C-H bond (6-9 times). Replacing the hydrogen in the drug molecule with deuterium may block the metabolic site and reduce the generation of toxic metabolites. In addition, deuterated drugs can remain stable under a variety of metabolic enzymes, slow down the system clearance rate and prolong the half-life of drugs in the body. Therefore, deuterated drug treatment can achieve the goal of reducing the toxic and side effects of the drug without affecting the pharmacological activity, by reducing single dose amount. In 2017, the US FDA approved the first deuterium-substituted drug in history - AUSTEDO^{®} from Teva, which was approved for marketing in China on May 2020. AUSTEDO^{®} is a deuterated version of tetrabenazine. Its prototype drug, tetrabenazine, has been the mainstream drug for the treatment of Huntington's disease, but has defects such as short half-life and low patient dependence. Deuterated tetrabenazine replaces the hydrogen atoms in the two methoxy groups on the tetrabenazine benzene ring with deuterium atoms, which significantly reduces the speed of drug metabolism and increases the half-life of the drug, thereby reducing the dosage of the drug. At the same time, it also inhibits the withdrawal reaction caused by the decrease of drug blood concentration.

### Summary

The technical problem to be solved in this application is to combine deuterated technology with camptothecin ADC to provide better deuterated camptothecin ADC drug with higher safety, efficacy, and better satisfaction of clinical needs.

Based on the comprehensive understanding of ADC drugs, the inventors provided a series of deuterium atom-substituted camptothecin antibody-drug conjugates by introducing deuterium atoms into camptothecin-containing linker-drug compounds. It is found through extensive experimentation that the molecule exhibits high drug activity and safety *in vivo* and *in vitro,* and has achieved unexpected technical advantages.

The application provides a deuterated camptothecin derivative shown in Formula D or a pharmaceutically acceptable salt or solvate thereof: 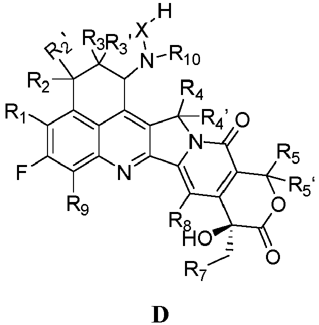 wherein:
R₁, R₇, R₁₀ are independently selected from hydrogen, deuterium, C₁₋₆ alkyl, one or more deuterated C₁₋₆ alkyl, substituted alkyl, aryl, one or more deuterated aryl groups, heteroaryl groups, and one or more deuterated heteroaryl group;
R₂, R₃, R₄, R₅, R₈, R₉ each are selected from hydrogen or deuterium;
X is selected from -C(O)-CR^{a}R^{b}-(CR^{c}R^{d})ₙ-O-, -C(O)-CR^{a}R^{b}-(CR^{c}R^{d})ₙ-NH- or -C(O)-CR^{a}R^{b}-(CR^{c}R^{d})ₙ-S-;
R^{a}, R^{b} are independently selected from a group consisting of hydrogen atom, deuterium atom, halogen, alkyl, deuterated alkyl, substituted alkyl, cycloalkyl, one or more deuterated cycloalkyl, cycloalkylalkyl, one or more deuterated cycloalkylalkyl, alkoxyalkyl, one or more deuterated alkoxyalkyl, heterocyclyl, aryl, substituted aryl and heteroaryl;
Alternatively, R^{a}, R^{b} together with their connected carbon atoms form C₃₋₆ cycloalkyl groups, or one or more deuterated C₃₋₆ cycloalkyl groups, cycloalkylalkyl groups, one or more deuterated cycloalkylalkyl groups, heterocyclic groups, and one or more deuterated heterocyclic group;
R^{c}, R^{d} are identical or different, and are independently selected from the group consisting of hydrogen, deuterium, halogen, C₁₋₆ alkyl, halogen alkyl, one or more deuterated C₁₋₆ alkyl, alkoxy, one or more deuterated alkoxy, hydroxyl, amino, cyanide, nitro, hydroxyalkyl, cycloalkyl, one or more deuterated cycloalkyl, heterocyclic, and one or more deuterated heterocyclic group;
Alternatively, R^{c}, R^{d} together with their connected carbon atoms form a C₃₋₆ cycloalkyl group, or one or more deuterated C₃₋₆ cycloalkyl groups, cycloalkylalkyl group, one or more deuterated cycloalkylalkyl groups, heterocyclic groups, and one or more deuterated heterocyclic group;
n is an integer from 0-4;
In a preferred embodiment, X is -C(O)-CR^{a}R^{b}-(CR³R⁴)ₙ-O-;
R^{a} is selected from hydrogen atom, deuterium atom, alkyl group, deuterated alkyl group, substituted alkyl group, cycloalkyl group, one or more deuterated cycloalkyl groups, cycloalkylalkyl group, one or more deuterated cycloalkylalkyl groups, alkoxyalkyl group, one or more deuterated alkoxyalkyl groups, heterocyclic group, aryl group, substituted aryl group or heteroaryl group;
R^{b} is selected from hydrogen atom, deuterium atom, alkyl group, deuterated alkyl group, substituted alkyl group, cycloalkyl group, one or more deuterated cycloalkyl groups, cycloalkylalkyl group, one or more deuterated cycloalkylalkyl groups, alkoxyalkyl group, one or more deuterated alkoxyalkyl groups, heterocyclic group, aryl group, substituted aryl group or heteroaryl group;
Alternatively, R^{a}, R^{b} together with their connected carbon atoms constitute a C₃₋₆ cycloalkyl group, one or more deuterated C₃₋₆ cycloalkyl groups, cycloalkylalkyl group, one or more deuterated cycloalkylalkyl groups or heterocyclic groups, or one or more deuterated heterocyclic group;
R^{c}, R^{d} are the identical or different, and are independently selected from the group consisting of hydrogen atom, deuterium atom, alkyl group, one or more deuterated alkyl groups, alkoxy groups, one or more deuterated alkoxy groups, hydroxyl groups, amino, cyano, nitro, hydroxyalkyl, cycloalkyl or heterocyclic group;
Alternatively, R^{c}, R^{d} together with their connected carbon atoms form C₃₋₆ cycloalkyl, one or more deuterated C₃₋₆ cycloalkyl, cycloalkylalkyl, or one or more deuterated cycloalkylalkyl;
n is selected from 0 or 1.
R₁, R₂, R_{2'}, R₃, R_{3'}, R₄, R_{4'}, R₅, R_{5'}, R₇, R₈, R₉, R₁₀ and X contains at least one deuterium atom.

In a preferred embodiment, the camptothecin derivative comprises the structure shown in Formula **D₂**: 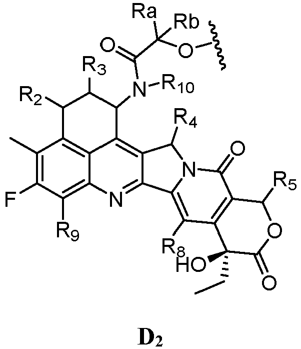 wherein:
R₁₀ is selected from hydrogen atom, or one or more deuterated C₁₋₆ alkyls;
R₂, R_{2'}, R₃, R_{3'}, R₄, R_{4'}, R₅, R_{5'}, R₈, R₉ are each selected from hydrogen or deuterium;
R^{a} is selected from hydrogen atom, deuterium atom, alkyl group, or one or more deuterated alkyl group;
R^{b} is selected from hydrogen atom, deuterium atom, alkyl group, or one or more deuterated alkyl group;
Alternatively, R^{a}, R^{b} together with their connected carbon atoms form C₃₋₆ cycloalkyl groups, one or more deuterated C₃₋₆ cycloalkyl groups, cycloalkylalkyl groups, one or more deuterated cycloalkylalkyl groups, heterocyclic groups, or one or more deuterated heterocyclic groups;
wherein, the wavy lines in Formula **D₂** represent either hydrogen atoms, or a covalent linkage linked either to a connector unit or to a ligand unit that binds to the antigen expressed by the target cell.

In a preferred embodiment, X is non-restrictively selected from:

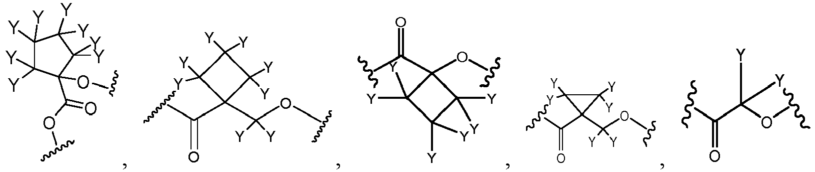

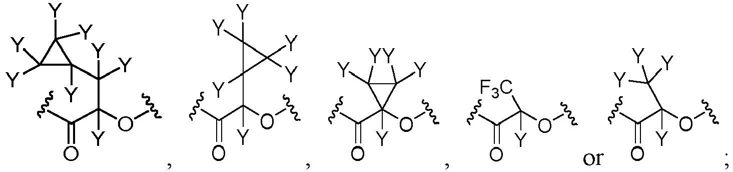

Wherein Y is selected from hydrogen or deuterium atoms.

In a preferred embodiment, the tautomers, mesoforms, racemates, enantiomers, diastereoisomers or mixtures thereof are included and disclosed.

In a preferred embodiment, compounds shown below or their pharmaceutically acceptable salts or solvates are non-restrictively selected.

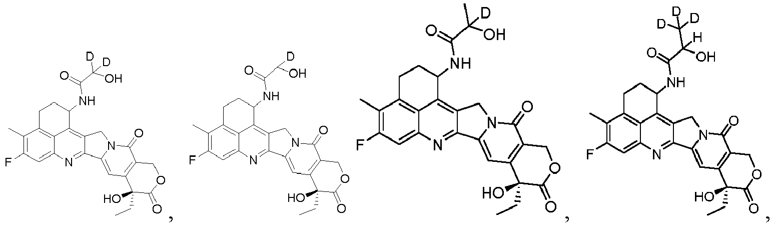

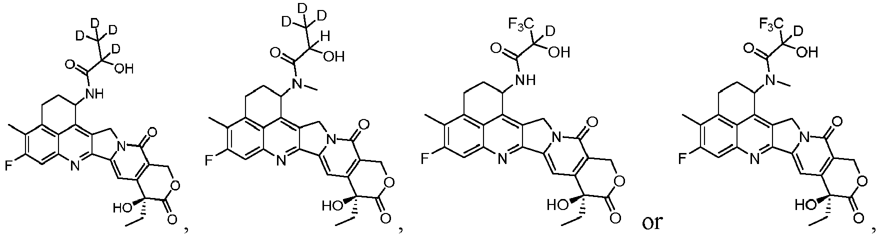

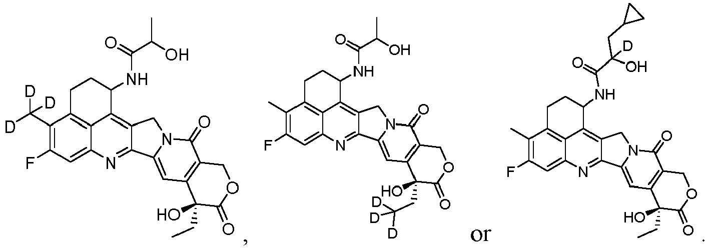

The invention also disclosed a drug-linker compound as shown in the Formula (-L-X-D₂,) or a pharmaceutically acceptable salt or solvate thereof 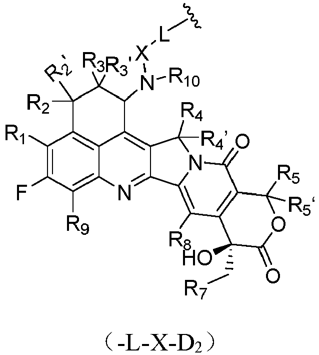 wherein:
R₁, R₇, R₁₀ are selected from the group consisting of hydrogen, deuterium, one or more deuterated C₁₋₆ alkyls, C₁₋₆ alkyl, substituted alkyl, aryl, one or more deuterated aryl, heteroaryl, and one or more deuterated heteroaryl groups;
R₂, R_{2'}, R₃, R_{3'}, R₄, R_{4'}, R₅, R_{5'}, R₈, and R₉ are each selected from hydrogen or deuterium;
X is -C(O)-CR^{a}R^{b}-(CR^{c}R^{d})ₙ-O-;
R^{a} and R^{b} are independently selected from the group consisting of hydrogen, deuterium, halogens, alkyl, deuterated alkyl, substituted alkyl, cycloalkyl, one or more deuterated cycloalkyl alkyl, cycloalkylalkyl, one or more deuterated cycloalkylalkyls, alkoxy alkyl, one or more deuterated alkoxy alkyl, heterocyclic groups, one or more deuterated heterocyclic, aryl, one or more deuterated aryl, substituted aryl, heteroaryl, and one or more deuterated heteroaryl;
Alternatively, R^{a} and R^{b} together with connected carbon atoms form a group consisting of a C₃₋₆ cycloalkyl group, one or more deuterated C₃₋₆ cycloalkyl groups, cycloalkylalkyl group, one or more deuterated cycloalkylalkyl groups, heterocyclic, or one or more deuterated heterocyclic groups;
R^{c} and R^{d} are identical or different, and independently selected from the group consisting of hydrogen, deuterium, halogen, alkyl, haloalkyl, C₁₋₆ alkyl, one or more deuterated or fully deuterated C₁₋₆ alkyl, alkoxy, one or more deuterated alkoxy, hydroxyl, amino, cyano, nitro, hydroxyalkyl, cycloalkyl, one or more deuterated cycloalkyl, heterocyclic, and one or more deuterated heterocyclic groups;
Alternatively, R^{c} and R^{d} together with their connected carbon atoms form a group consisting of C₃₋₆ cycloalkyl, one or more deuterated C₃₋₆ cycloalkyl, cycloalkylalkyl, one or more deuterated cycloalkylalkyl, heterocyclic, and one or more deuterated heterocyclic;
n is selected from integers ranging from 0-4;
R₁, R₂, R_{2'}, R₃, R_{3'}, R₄, R_{4'}, R₅, R_{5'}, R₇, R₈, R₉, R₁₀ and X contains at least one deuterium atom;
L is a linker unit;
wherein the wavy lines in Formula -L-X-D₂ represents either a hydrogen atom or a covalent linkage to either a connector unit or an antibody that binds to an antigen expressed by target cells.

In a preferred embodiment, the O-terminal of X is connected to the linker unit L.

In a preferred embodiment, the linker unit -L- is -L1-L2-L3-L4-, wherein the L1 end is connected to an antibody and the L4 end is connected to the X;
wherein:
L₁ is selected from -(Succinimido-3-yl-*N*)-Y-C(O)-, -CH₂-C(O)-NR⁵-Y-C(O)- or -C(O)-Y-C(O)-,
wherein Y is selected from a group consisting of C₁₋₈ alkyl, C₁₋₈ alkyl-cycloalkyl and 1-8 atom linear or linear-cyclic heteralkyl; wherein the heteroalkyl group comprises 1-3 atoms selected from N, O or S, and wherein the C₁₋₈ alkyl, cycloalkyl, linear or linear-cyclic heteralkyl are independently substituted by one or more substituents selected from a group consisting of deuterated atom, halogen, hydroxyl, cyano, nitro, amino, alkyl, carboxy, heteralkyl, substituted alkyl, alkoxy and cycloalkyl group;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or a chemical bond, wherein p is selected from an integer from 0-20;
L₃ is selected from peptide residues composed of 2-7 amino acids, wherein the amino acids are further substituted by one or more substituents selected from the group consisting of deuterium, halogen, hydroxyl, cyano, amino, nitro, alkyl, substituted alkyl, alkoxy, cycloalkyl and substituted cycloalkyl;
L₄ is selected from -NR⁷(CR⁸R⁹)_{q}-, -C(O)NR⁷, -C(O)NR⁷(CH₂)_{q}- or chemical bond, and q is selected from an integer from 0-6;
R⁵, R⁶ and R⁷ are identical or different, and are independently selected from hydrogen atoms, deuterium atoms, halogens, alkyl groups, deuterated alkyl groups, haloalkyl groups, cycloalkyl groups, cycloalkylalkyl groups, alkoxyalkyl groups, heterocyclic groups, aryl groups, substituted aryl or heteroaryl groups; and
R⁸ and R⁹ are identical or different, and are independently selected from hydrogen atoms, deuterium atoms, halogens, alkyl groups, deuterated alkyl groups, haloalkyl groups, cycloalkyl groups, cycloalkyl alkyl groups, alkoxyalkyl groups, heterocyclic groups, aryl groups, substituted aryl or heteroaryl groups.

In still another preferred embodiment, L₁ is selected from -(Succinimido-3-yl-*N*)-Y-C(O)-, -CH₂-C(O)-NR⁵-Y-C(O)- or -C(O)-Y-C(O)-;
wherein Y is selected from C₁₋₈ alkyl, C₁₋₈ alkyl-cycloalkyl or 1-8 atom linear or linear-cyclic heteralkyl; wherein the heteroalkyl group comprises 1-3 atoms selected from N, O or S, and the C₁₋₈ alkyl, cycloalkyl group, linear or linear-cyclic heteryl group are substituted by one or more substituents selected independently from a group consisting of deuterated atom, halogen, hydroxyl, cyano, nitro, amino, alkyl, carboxy, heteralkyl, substituted alkyl, alkoxy and cycloalkyl group;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or chemical bond, wherein p is selected from an integer from 0-20.
L₃ is selected from peptides comprising amino acids phenylalanine (F), glycine (G), valine (V), lysine (K), citrulline, serine(S), glutamic acid (E) and aspartic acid (D); more preferably, L₃ is a peptide comprising one, two or more amino acids selected from phenylalanine and glycine; the most preferred is a tetrapeptide residue composed of glycine-glycine-phenylalanine-glycine.
L₄ is -NR⁷CR⁸R⁹-; in a preferred embodiment L₄ is -NHCH₂-;
R⁵, R⁶ and R⁷ are identical or different, and are independently selected from hydrogen, deuterium, halogen, alkyl, deuterated alkyl, haloalkyl, cycloalkyl, cycloalkylalkyl, alkoxyalkyl, heterocyclic group, aryl, substituted aryl or heteroaryl;
R⁸ and R⁹ are identical or different, and are independently selected from hydrogen, deuterium, halogen, alkyl, deuterated alkyl, haloalkyl, cycloalkyl, cycloalkylalkyl, alkoxyalkyl, heterocyclic group, aryl, substituted aryl or heteroaryl.

The invention provided a drug-linker compound as shown in the Formula L-X-D₂, or a pharmaceutically acceptable salt or solvate thereof; 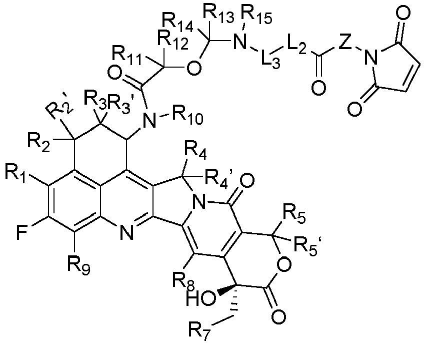 wherein:
Z is selected from -Y-C(O)-, -CH₂-C(O)-NR⁵-Y-C(O)- or -C(O)-Y-C(O)-, wherein Y is non-restrictively selected from C₁₋₈ alkyl, C₁₋₈ alkyl-cycloalkyl or 1-8 atom linear or linear-cyclic heteralkyl, wherein the heteralkyl group comprises 1-3 atoms selected from N, O or S, and the C₁₋₈ alkyl, cycloalkyl, linear or linear-cyclic heteryl group is independently substituted by one or more substituents selected from deuterium, halogen, hydroxyl, cyano, nitro, amino, alkyl, heteralkyl, substituted alkyl, alkoxy, carboxyl or cycloalkyl group;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or chemical bond, wherein p is selected from an integer from 0-20, wherein an alkyl group in the structure is replaced by one or more deuterium atoms;
L₃ is selected from peptide residues composed of 2-7 amino acids, wherein the amino acids are further substituted by one or more substituents selected from deuterium, halogen, hydroxyl, cyano, amino, nitro, alkyl, substituted alkyl, alkoxy and cycloalkyl and substituted cycloalkyl;
R is selected from deuterium atom, C₁₋₆ alkyl, substituted alkyl, aryl, substituted aryl or heteroaryl;
R₁₁ is selected from hydrogen atom, deuterium atom, halogen, alkyl group, deuterated alkyl group, haloalkyl group, cycloalkyl group cycloalkylalkyl group, heterocyclic group, aryl group, substituted aryl or heteroaryl group;
R₁₂ is selected from hydrogen atom, deuterium atom, halogen, alkyl group, haloalkyl group, deuterated alkyl group, cycloalkyl group, cycloalkylalkyl group, alkoxyalkyl group, heterocyclic group, aryl group, substituted aryl or heteroaryl group;
Alternatively, R₁₁, R₁₂ together with their connected carbon atoms form a C₃₋₆ cycloalkyl, cycloalkylalkyl or heterocyclic group;
R⁵, R⁶ are selected from hydrogen atom, deuterium atom, halogen, alkyl, deuterated alkyl, haloalkyl, cycloalkyl, cycloalkylalkyl, alkoxyalkyl, heterocyclyl, aryl, substituted aryl or heteroaryl;
R₁, R₇, R₁₀, R₁₅ are selected from hydrogen atoms, alkyl groups, or one or more deuterated C₁₋₄ alkyls;
R₂, R_{2'}, R₃, R_{3'}, R₄, R_{4'}, R₅, R_{5'}, R₈, R₉ are each selected from hydrogen or deuterium;
R₁, R₂, R_{2'}, R₃, R_{3'}, R₄, R_{4'}, R₅, R_{5'}, R₇, R₈, R₉, R₁₀, R₁₁ and R₁₂ contain at least one deuterium atom.

In a preferred embodiment, the compound according to Formula (L_{b}-X-D₂), or its pharmaceutically acceptable salt or solvate, is disclosed; 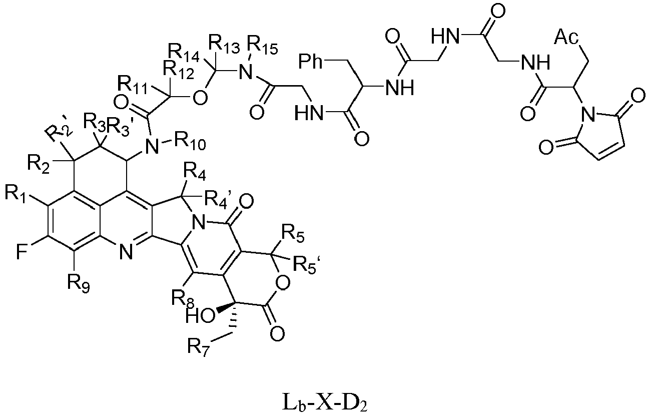 wherein:
R₁₁, R₁₂ are independently selected from hydrogen atoms, deuterium atoms, halogens, alkyl groups, deuterated alkyl groups, substituted alkyl groups, cycloalkyl groups, one or more deuterated cycloalkyl alkyl groups, cycloalkylalkyl groups, one or more deuterated cycloalkylalkyl groups, heterocyclic groups, one or more deuterated heterocyclic groups, aryl groups, one or more deuterated aryl groups, substituted aryl groups, heteroaryl groups, or one or more deuterated heteroaryl groups;
alternatively, R₁₁, R₁₂ together with the carbon atoms connected thereto form a C₃₋₆ cycloalkyl group, one or more deuterated C₃₋₆ cycloalkyl group, cycloalkylalkyl group, one or more deuterated cycloalkylalkyl groups, heterocyclic group, or one or more deuterated heterocyclic groups;
R₁, R₇, R₁₀, R₁₅ are selected from hydrogen atoms, alkyl groups, one or more deuterated or fully deuterated C₁₋₄ alkyl or substituted alkyls;
R₂, R_{2'}, R₃, R_{3'}, R₄, R_{4'}, R₅, R_{5'}, R₈, and R₉ are each selected from hydrogen or deuterium;
Ac is a hydrophilic structural unit having the structure of Formula c, containing both an amino group and a carboxyl group, X is a scaffold connecting the amino group and the carboxyl group and a C₁₋₁₀ subhydrocarbon group or substituted alkyton group, the subhydrocarbon group or substituted hydrocarbon group may be replaced by one or more deuterium atoms; Ac is connected to the 2-position methylene carbon marked in the structural formula L_{b}-X-D₂ through an amino functional group;

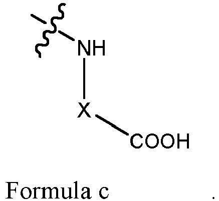

In a preferred embodiment, disclosed are tautomer, mesomer, racemate, enantiomer, diastereoisomer or a mixture thereof, wherein Ac is selected from a group consisting of glycine, α-alanine, β-alanine, and (D/L) glutamic acid.

In a preferred embodiment, deuterated camptothecin derivatives or their pharmaceutically acceptable salts or solvates are selected from the following structures: 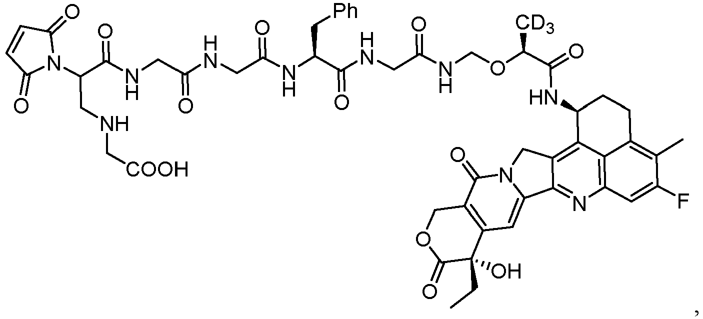 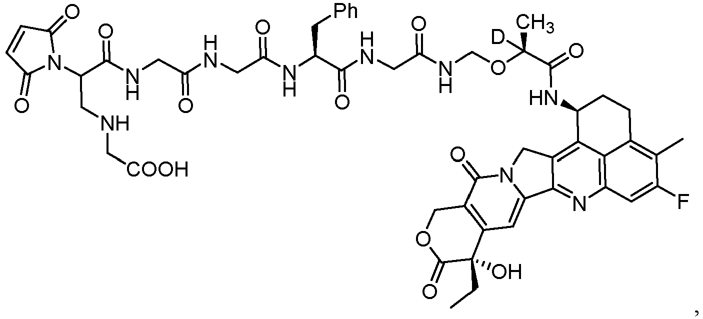 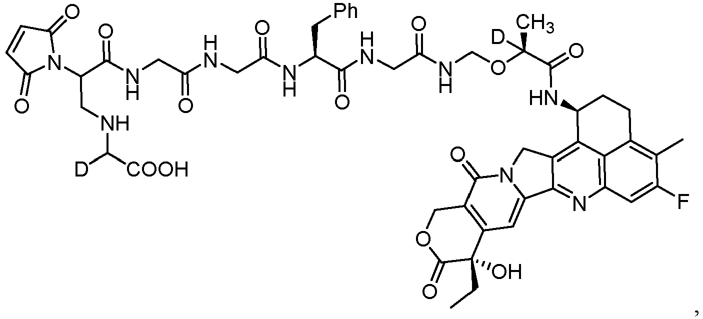 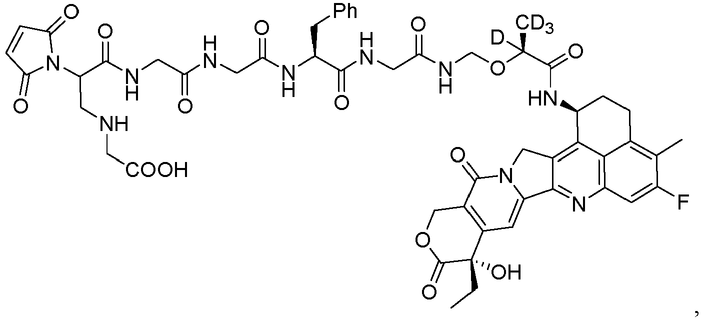 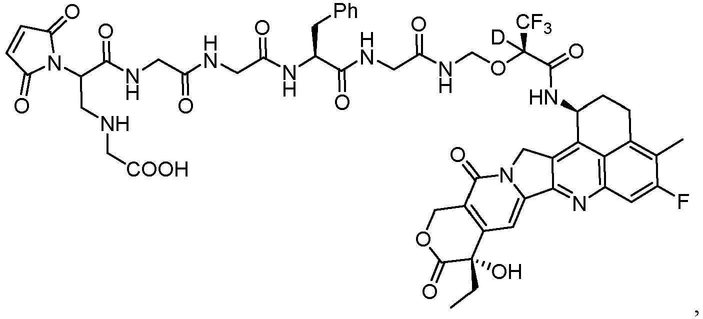 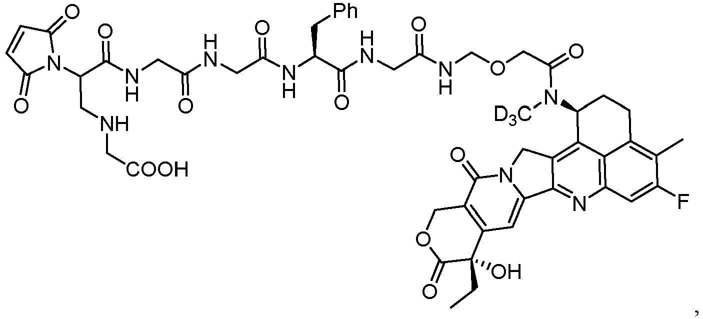 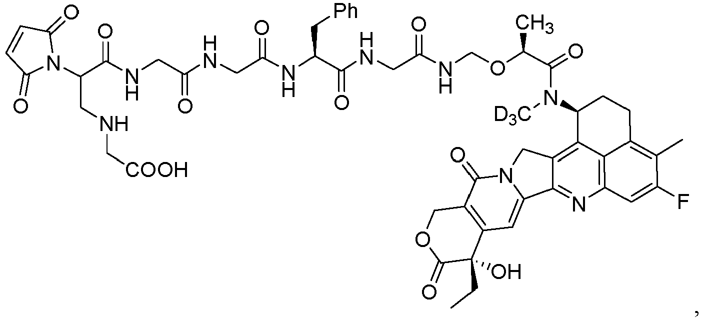 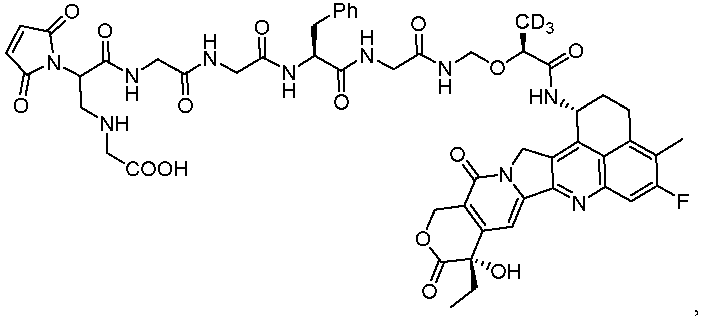 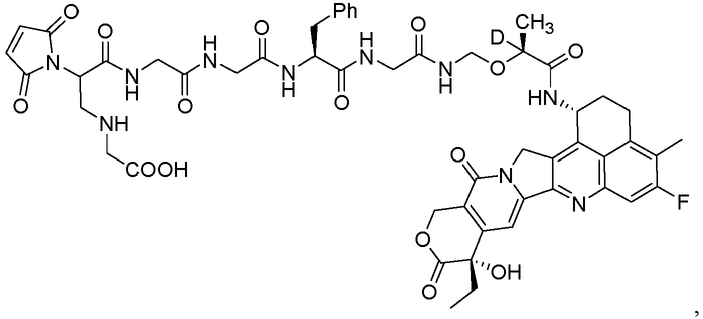 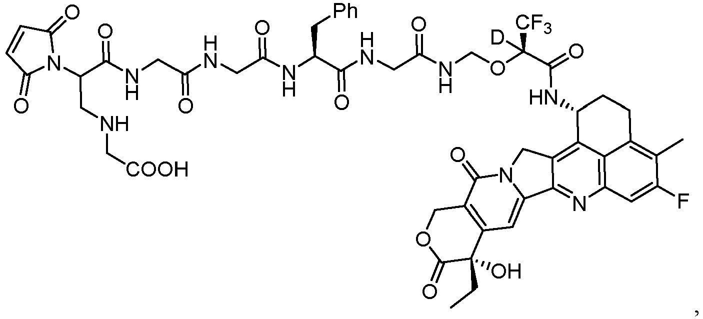 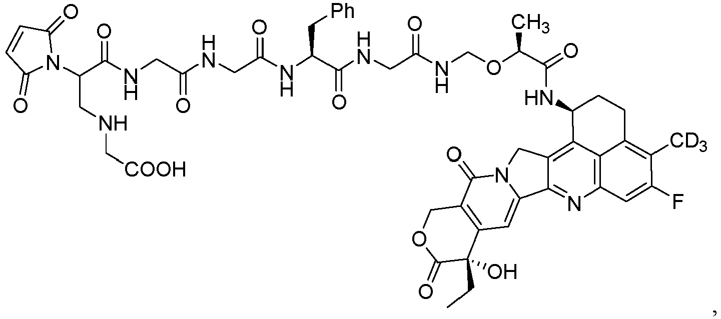 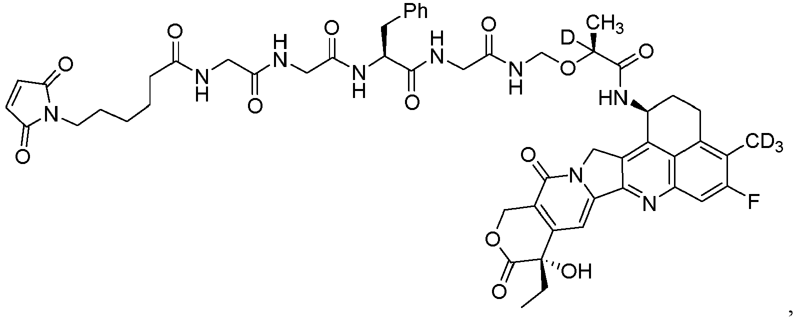 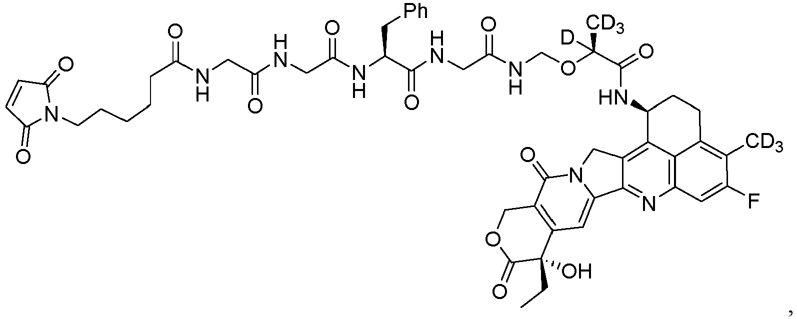 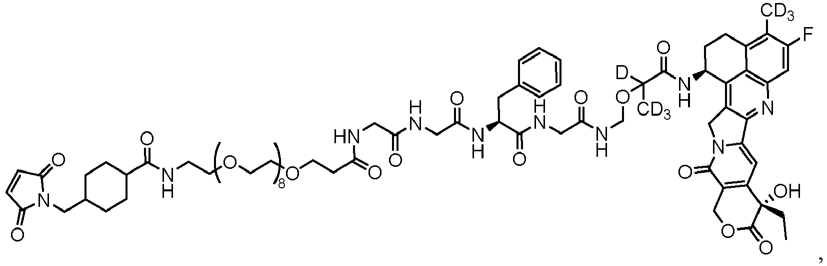 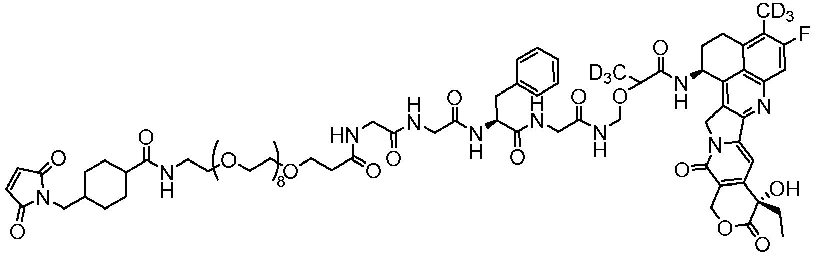 or

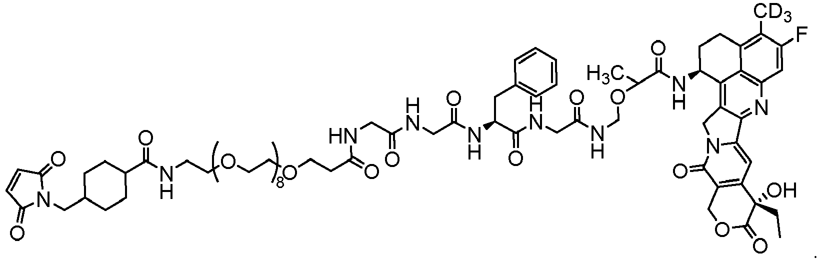

The present invention provides a camptothecin derivative or a camptothecin-linker compound, or a tautomer, mesoform, racemate, enantiomer, diastereoisomer or a mixture thereof, or a pharmaceutically acceptable salt or solvate thereof, which is linked to a ligand unit to form an antibody-drug conjugate as shown in the general formula (Ab-L-X-Dr) or a pharmaceutically acceptable salt or solvent. 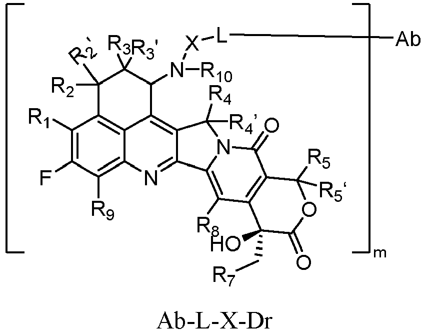 wherein:
R₁, R₇, R₁₀ are selected from hydrogen, deuterium, one or more deuterated C₁₋₆ alkyls, C₁₋₆ alkyl, substituted alkyl groups, aryl groups, one or more deuterated aryl groups, heteroaryl groups, or one or more deuterated heteroaryl groups;
R₂, R_{2'}, R₃, R_{3'}, R₄, R_{4'}, R₅, R_{5'}, R₈, R₉ are each selected from hydrogen or deuterium;
X is -C(O)-CR^{a}R^{b}-(CR^{c}R^{d})ₙ-O-;
R^{a}, R^{b} are independently selected from hydrogen atoms, deuterium, halogens, alkyl groups, deuterated alkyl groups, substituted alkyl groups, cycloalkyl groups, one or more deuterium atom substituted cycloalkyl group, cycloalkylalkyl groups, one or more deuterium atom substituted cycloalkylalkyl groups, alkoxyalkyl group, one or more deuterium atom substituted alkoxyalkyl groups, heterocyclic group, aryl group, substituted aryl or heteroaryl group;
Alternatively, R^{a}, R^{b} together with the carbon atoms connected thereto form a C₃₋₆ cycloalkyl, or one or more deuterium atoms substituted C₃₋₆ cycloalkyl, cycloalkylalkyls, one or more deuterium atoms substituted cycloalkylalkyls, heterocyclic groups, or one or more deuterated heterocyclic groups;
R^{c}, R^{d} are identical or different, and are independently selected from the group consisting of hydrogen, deuterium, halogen, C₁₋₆ alkyl, halogen alkyl, one or more deuterated C₁₋₆ alkyl, alkoxy, one or more deuterated alkoxy, hydroxyl, amino, cyanide, nitro, hydroxyalkyl, cycloalkyl, one or more deuterated cycloalkyl, heterocyclic, and one or more deuterated heterocyclic groups;
Alternatively, R^{c}, R^{d} together with their connected carbon atoms form a C₃₋₆ cycloalkyl group, one or more deuterated C₃₋₆ cycloalkyl group, cycloalkylalkyl group, one or more deuterated cycloalkylalkyl groups, heterocyclic groups, and one or more deuterated heterocyclic group;
n is an integer from 0-4;
L is a linker unit;
wherein the wavy lines in Formula -L-X-D₂ indicate either a hydrogen atom, or a covalent linkage linked to either a connector unit or to an antibody that binds to an antigen expressed in target cells.

In a preferred embodiment, wherein the linker unit -L- is -L₁-L₂-L₃-L₄-, wherein the L₁ end is connected to an antibody and the L₄ end is connected to the X;
wherein:
L₁ is selected from -(Succinimido-3-yl-*N*)-Y-C(O)-, -CH₂-C(O)-NR⁵-Y-C(O)- or -C(O)-Y-C(O)-,
wherein Y is selected from a group consisting of C₁₋₈ alkyl, C₁₋₈ alkyl-cycloalkyl or 1-8 atom linear and linear-cyclic heteralkyl; wherein the heteroalkyl group comprises 1-3 atoms selected from N, O or S, and wherein the C₁₋₈ alkyl, cycloalkyl, linear or linear-cyclic heteralkyl group are independently substituted by one or more substituents selected from a group consisting of deuterated atom, halogen, hydroxyl, cyano, nitro, amino, alkyl, carboxy, heteralkyl, substituted alkyl, alkoxy and cycloalkyl group;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or a chemical bond, wherein p is selected from an integer from 0-20;
L₃ is selected from peptide residues composed of 2-7 amino acids, wherein the amino acids are further substituted by one or more substituents selected from the group consisting of deuterium, halogen, hydroxyl, cyano, amino, nitro, alkyl, substituted alkyl, alkoxy, cycloalkyl and substituted cycloalkyl;
L₄ is selected from -NR⁷(CR⁸R⁹)_{q}-, -C(O)NR⁷, -C(O)NR⁷(CH₂)_{q}- or chemical bond, and q is selected from an integer from 0-6;
R⁵, R⁶ and R⁷ are identical or different, and are independently selected from hydrogen atoms, deuterium atoms, halogens, alkyl groups, deuterated alkyl groups, haloalkyl groups, cycloalkyl groups, cycloalkyl alkyl groups, alkoxyalkyl groups, heterocyclic groups, aryl groups, substituted aryl or heteroaryl groups;
R⁸ and R⁹ are identical or different, and are independently selected from hydrogen atoms, deuterium atoms, halogens, alkyl groups, deuterated alkyl groups, haloalkyl groups, cycloalkyl groups, cycloalkyl alkyl groups, alkoxyalkyl groups, heterocyclic groups, aryl groups, substituted aryl or heteroaryl groups.
m is selected from integers or decimals from 1-10;
Ab is an antibody, antibody fragment, target protein or Fc-fusion protein;
L is a linker unit.

In a preferred embodiment, Ab is an antibody that may form a linking bond with a linker unit through heteroatoms, the antibody is selected from murine antibodies, chimeric antibodies, humanized antibodies, fully human antibodies, antibody fragments, bispecific antibodies and multispecific antibodies.

In a more preferred embodiment, the antibody is selected from an antibody targeting one or more of the following targets: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MLTC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody or anti-CD123 antibody, and anti-CD47 antibody.

In a preferred embodiment, the antibody-drug conjugate or a pharmaceutically acceptable salt or solvate thereof are selected non-restrictively from the following: 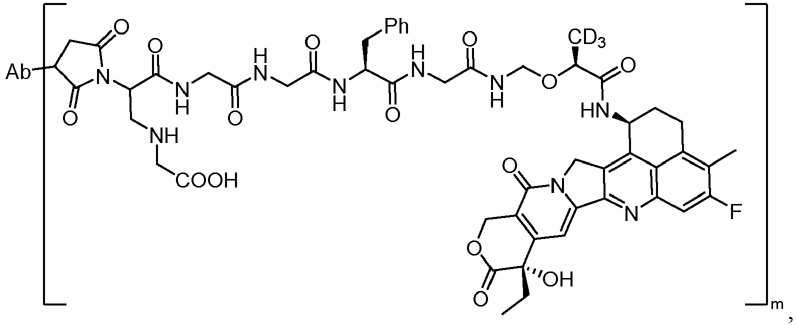 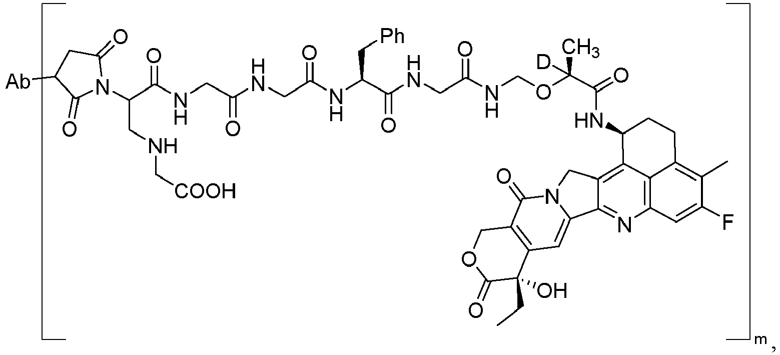 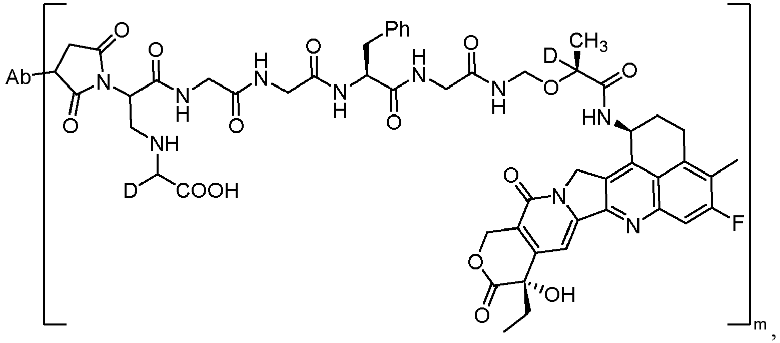 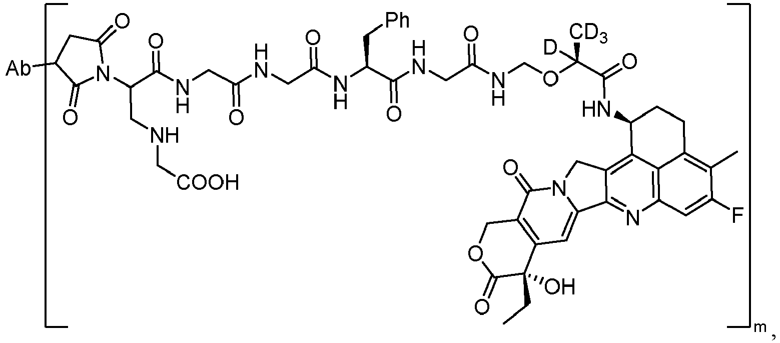 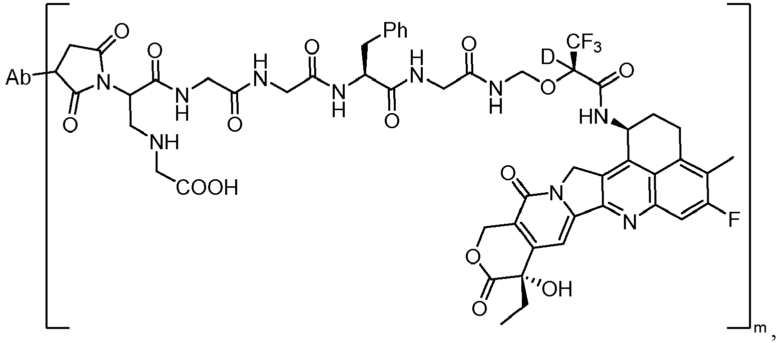 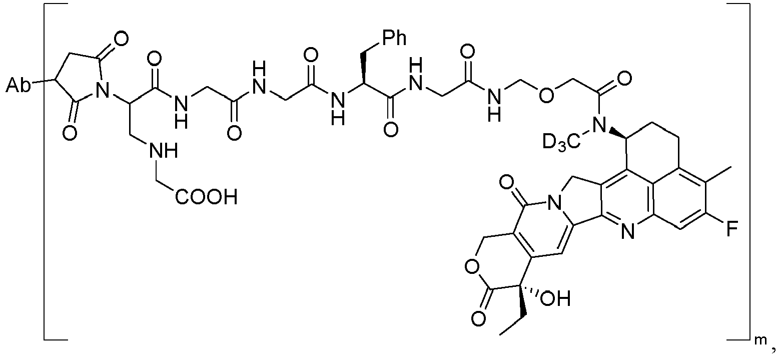 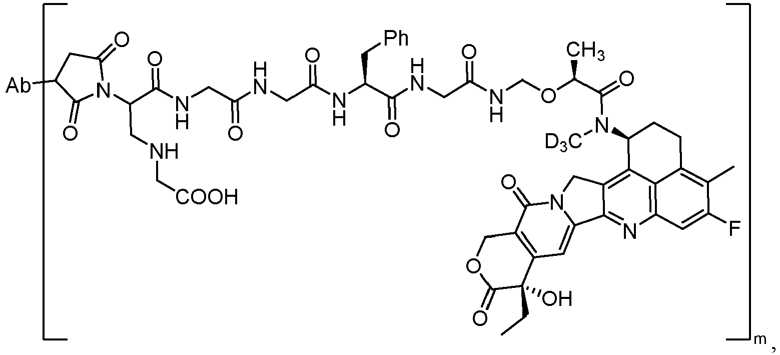 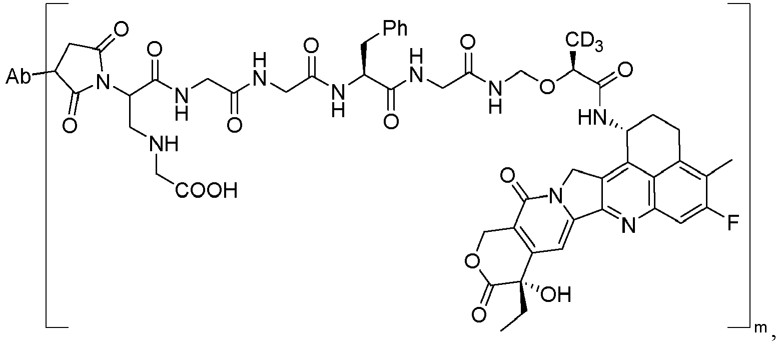 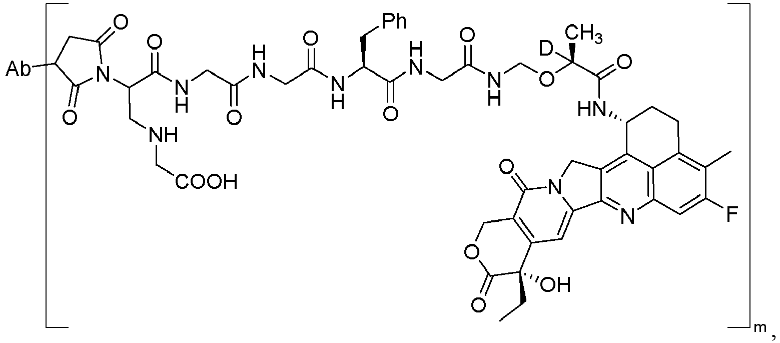 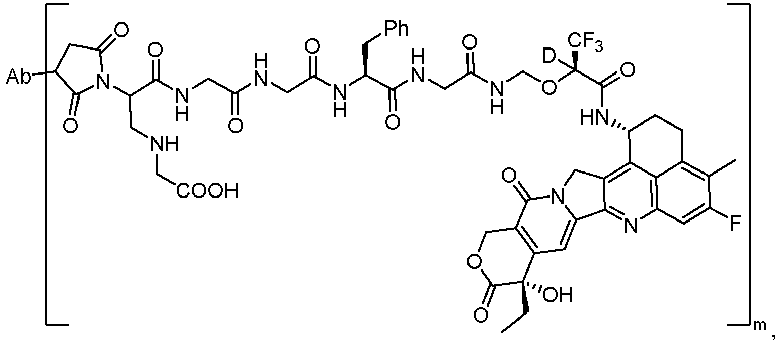 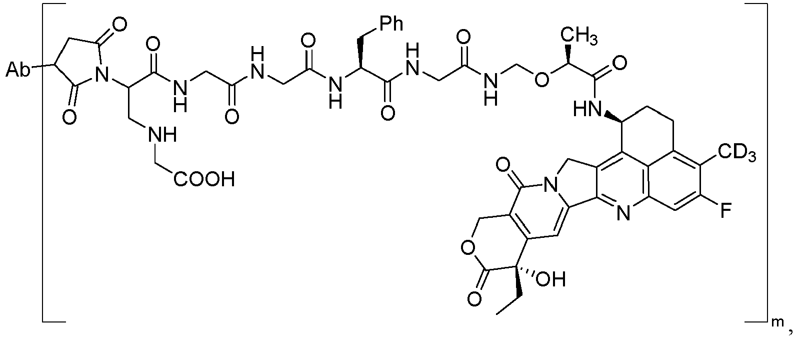 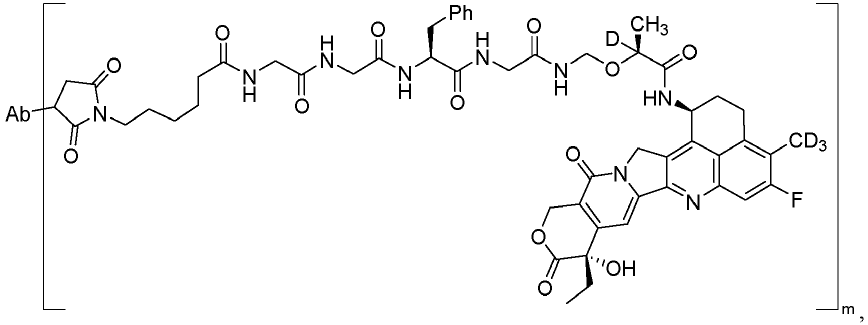 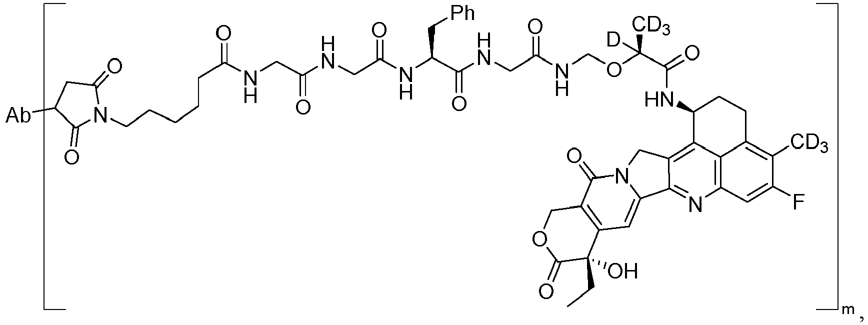 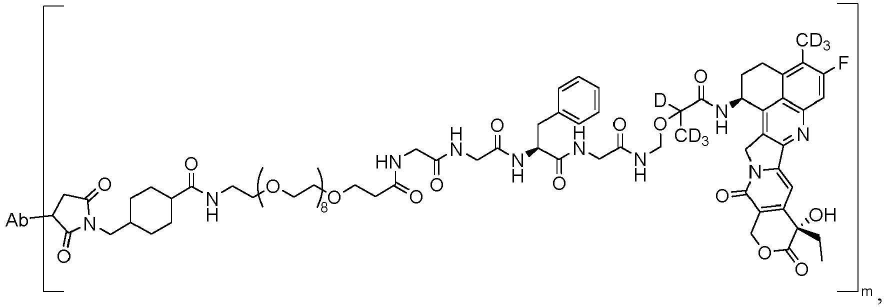 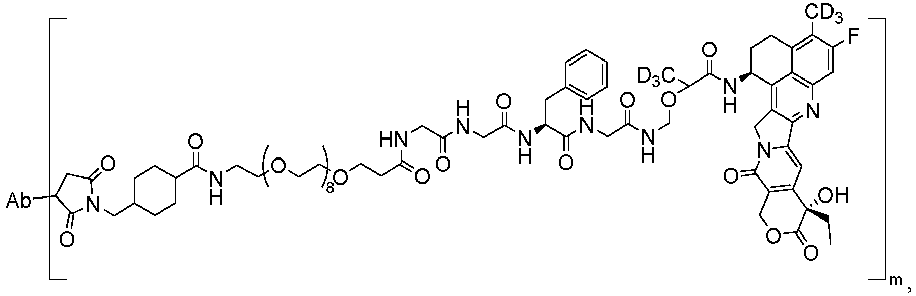 or 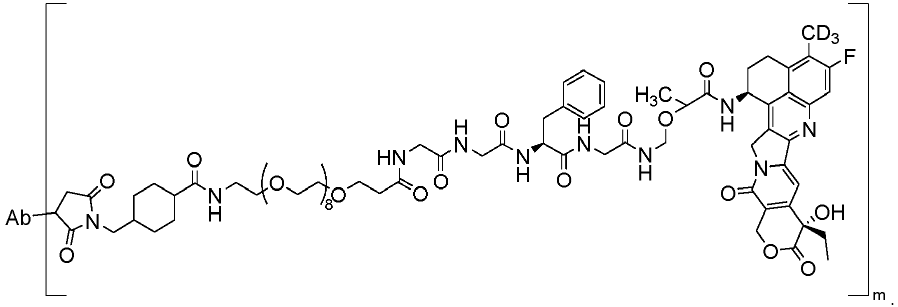 wherein:
m is selected from integers or decimals from 1-10;
Ab is an antibody, antibody fragment, targeted protein, or Fc-fusion protein.

The invention provides a method for preparing the compound (L-X-D₂), comprising the following synthesis steps.

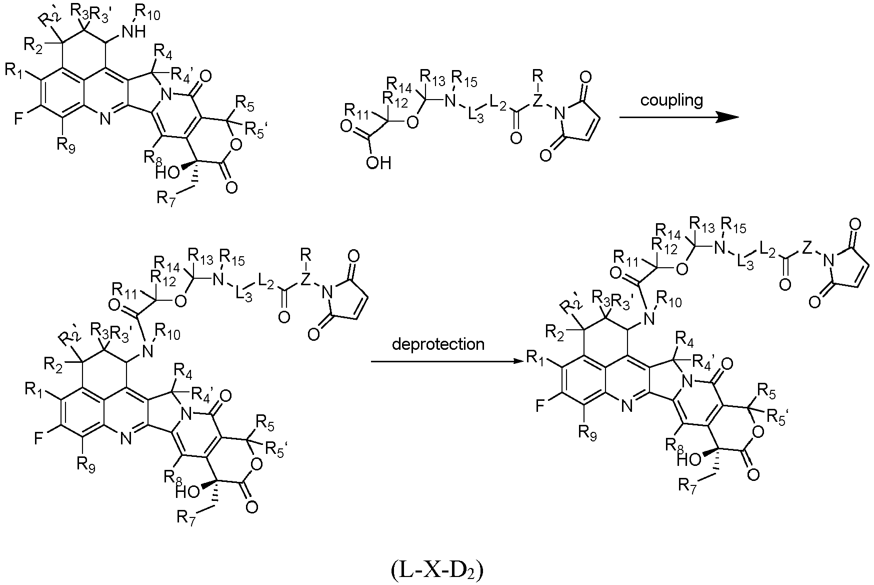

The application provides a method for preparing an antibody-drug conjugate with Formula (Ab-La-X-Dr) or a pharmaceutically acceptable salt or solvate thereof, comprising the following steps: : 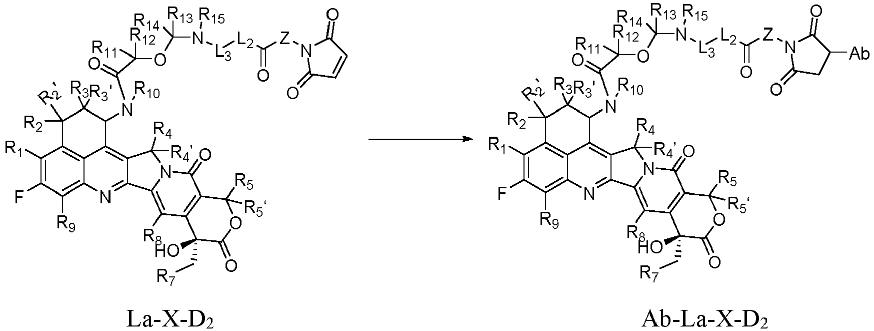 generate compound of Formula Ab-La-X-D₂ by conjugating antibodies, antibody fragments, targeted proteins, Fc-fusion proteins, etc. to compound of Formula (La-X-D₂).

The application provides a pharmaceutical composition comprising a therapeutically effective amount of the antibody-drug conjugate or a pharmaceutically acceptable salt or solvate, and a pharmaceutically acceptable carrier, diluent or excipient.

The application discloses the use, for purpose of preparing drugs for the treatment or prevention of tumors, of the camptothecin derivative or antibody-drug conjugate, or their tautomers, mesomers, racemates, enantiomers, diastereoisomers or a mixture thereof, or a pharmaceutically acceptable salt or solvent compound thereof, and a pharmaceutically acceptable carrier, diluent, or excipient.

In a preferred embodiment, the tumors are non-restrictedly selected from breast cancer, ovarian cancer, cervical cancer, uterine cancer, prostate cancer, kidney cancer, urinary tract cancer, bladder cancer, liver cancer, stomach cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma, glioblastoma, sarcoma and other solid tumors such as lymphoma and leukemia or blood tumors.

### Detailed Description

### Abbreviations and Definitions

Unless otherwise qualified, all technical and scientific terms used herein are consistent with the common understanding of those of ordinary skill in the art to which the present disclosure belongs. Although this disclosure may also be implemented or tested using any method and material similar or equivalent to the herein, the preferred method and material described herein is described herein. When describing and claiming protection of the invention, the following terms are used in accordance with the following definitions.

When a trade name is used in the present invention, the applicant intends to include the formulation of the trade name product, the generic drug and the active portion of the trade name product.

Unless otherwise indicated, the following terms and phrases as used herein are intended to have the following meanings. When a trademark is used herein, unless otherwise indicated in the context, the trademark includes the product formulation of the said trademark-associated product, generic drug and active pharmaceutical ingredient.

Unless stated to the contrary, terms used in the description and claims have the following meanings.

The term "ligand" refers to a macromolecular compound that recognizes and binds to antigens or receptors associated with target cells. The role of the ligands is to present drugs to target cell populations that bind to ligands, the ligands include but are not limited to protein hormones, lectins, growth factors, antibodies, or other molecules that bind to cells. In an embodiment of the present invention, the ligand is represented as Ab, and the ligand may form a bond with a linker by heteroatoms on the ligand. Preferably the ligand is an antibody or its antigen-binding fragment, the antibody is selected from a chimeric antibody, a humanized antibody, a fully human antibody or a murine antibody; preferably the Ab is monoclonal antibodies.

Ligand units refer to targeting agents that bind specifically to a target moiety. The ligand is capable of specifically binding to a cellular component or binding to a cellular component or binding to other target molecules of interest. The target portion or target is usually located on the cell surface. In some ways, the role of the ligand unit is to deliver a drug unit to the specific target cell population with which the ligand unit interacts. Ligands include, but are not limited to, proteins, polypeptides, and peptides, as well as non-proteins such as sugars. Suitable ligand units include, for example, antibodies, such as full-length (complete) antibodies or their antigen-binding fragments. In an embodiment in which the ligand unit is a non-antibody targeting reagent, it may be a peptide or polypeptide, or a non-protein molecule. Examples of such targeting agents include interferons, lymphokines, hormones, growth factors and colony-stimulating factors, vitamins, nutrient transport molecules, or any other cell-binding molecule or substance. In some embodiments, the linker is covalently linked to the sulfur atom of the ligand. In some embodiments, the sulfur atom is the sulfur atom of the cysteine residue that forms the interchain disulfide bond of the antibody. In some other embodiments, the sulfur atom is the sulfur atom of the cysteine residue that has been introduced into the ligand unit, which forms the interchain disulfide bond of the antibody. In some embodiments, the sulfur atoms are sulfur atoms that have been introduced into cysteine residues of ligand units (e.g., by site-directed mutagenesis or chemical reactions). In other respects, the linker-bound sulfur atom is selected from the cysteine residue that forms interchain disulfide bond of an antibody or the fronteceine residue that has been introduced into the ligand unit (e.g., by site-specific mutagenesis or chemical reaction). In some embodiments, EU index numbering system in Kabat (Kabat E.A. et al., (1991) (Sequences of Proteins of Immunological Interest, Fifth Edition, NIH Publication 91-3242) are used.

As used herein, "antibody" or "antibody unit" includes any part of the antibody structure to the extent to which it belongs. This unit can bind, reactively link, or form a complex to a receptor, antigen, or other receptor unit that the targeted cell population has. An antibody can be any protein or protein molecule that can bind, form a complex, or react with a portion of a cell population to be treated or biomodified. The antibody composed of an antibody drug conjugate in the present invention maintains its original antigen-binding ability in its original wild state. Therefore, the antibodies in the present invention can bind to antigens specifically. Antigens involved include, for example, tumor-associated antigens (TAAs), cell surface receptor proteins and other cell surface molecules, regulators of cell survival, regulators of cell proliferation, molecules associated with tissue growth and differentiation (such as known or predicted functional), lymphokines, cytokines, molecules involved in the regulation of the cellular cycle, molecules involved in angiogenesis, and molecules associated with angiogenesis (e.g., known or predicted functional). Tumor-associated factors can be clustered differentiating factors (e.g., CD protein).

Antibodies used in antibody-drug conjugates include, but are not limited to, antibodies against cell surface receptors and tumor-associated antigens. Such tumor-associated antigens are well known in the industry and can be prepared through well-known antibody preparation methods and information. To develop effective cell-level targets that can be used in cancer diagnosis and treatment, researchers seek transmembrane peptides or other tumor-associated peptides. These targets can be expressed specifically on the surface of one or more cancer cells and expressed little or no on the surface of one or more non-cancer cells. In general, such tumor-associated peptides tend to be overexpressed on the surface of cancer cells than on the surface of non-cancer cells. Identification of such tumor-associated factors can greatly improve the specific targeting characteristics of antibody-based treatment of cancer. For convenience, well-known antigen-related information is indicated below, including names, other names, and gene bank registry numbers. Nucleic acid and protein sequences corresponding to tumor-associated antioxidants can be found in public databases, such as Genbank. Tumor-associated antigens targeted by antibodies include all amino acid sequence variants and allogenes, have at least 70%, 80%, 85%, 90%, or 95% homology with sequences confirmed in the references, or have biological properties and characteristics that are completely consistent with the tumor-associated antigen sequences in the cited literature.

The term "inhibit" or "inhibition" refers to reducing the amount that can be detected, or preventing it altogether.

The term "cancer" refers to a physiological condition or disease characterized by dysregulated cell growth. "Tumor" includes cancer cells.

The term "autoimmune disease" is a disease or disorder that originates in targeting an individual's own tissues or proteins.

The term "drug" refers to a cytotoxic drug, expressed as D, a chemical molecule that can disrupt tumor cell normal growth when present within the tumor cell. In principle, cytotoxic drugs can kill tumor cells at high enough concentrations, but due to the lack of specificity, while killing tumor cells, a cytotoxic drug may also lead to apoptosis of normal cells, resulting in serious side effects. The term includes toxins such as small molecule toxins or enzyme-active toxins of bacterial, fungal, plant or animal origin, radioisotopes (e.g., At²¹¹, I¹³¹, I¹²⁵, Y⁹⁰, Re¹⁸⁶, Re¹⁸⁸, Sm¹⁵³, Bi²¹², P³² and Lu), toxic drugs, chemotherapy drugs, antibiotics and ribozin, preferably toxic drugs.

The term "linker" or "linker fragment" or "linker unit" refers to a chemical structure fragment or bond that is attached/connected/lined to a ligand at one end and to a drug at the other end, and can also be attached/connected/lined to other connectors and then connected to a drug.

Connectors, including extensions, spacers and amino acid units, can be synthesized by methods known in the art, such as those described in US2005-0238649A1. Adapters can be "cuttable junctions" that facilitate the release of drugs in the cell. For example, acid-labile (e.g., hydrazone), protease-sensitive (e.g., peptidase-sensitive) connector, photolabile connector, dimethyl connector, or disulfide-containing connector may be used (Chari et al. Cancer Research 52: 127-131, 1992); U.S. Patent No. 5, 208, 020.

Depending on the mechanism of drug release in the cell, as used herein, "linkers" or "linkers of antibody-drug conjugates" can be divided into two categories: non-breakable linkers and breakable linkers. For antibody drug conjugates containing non-breakable linkers, the drug release mechanism is: after the conjugates bind to antigens and are endocytosed by cells, the antibodies are enzymatically hydrolyzed in lysosomes, releasing active molecules composed of small molecule drugs, linkers, and antibody amino acid residues. The resulting changes in the molecular structure of the drug do not diminish its cytotoxicity, but because the active molecule is charged (amino acid residues), it cannot penetrate into neighboring cells. Therefore, such active drugs cannot kill tumor cells (bystander effect) that do not express a targeted antigen (antigen-negative cells) (Ducry et al., 2010, Bioconjugate Chem. 21: 5-13).

The term "ligand-drug conjugate" refers to the antibody being attached to a biologically active drug through a stable linker. In the present invention, "ligand-drug conjugate" is preferably an antibody drug conjugate (ADC), which refers to a monoclonal antibody or antibody fragment connected to a biologically active toxic drug through a stable linker unit.

The amino acid three-letter code and the single-letter code used in the present disclosure are such as J. boil. Chem. 1968, 243, 3558.

The term "alkyl" refers to a saturated aliphatic hydrocarbon group, which include a straight-chain or branched-chain group comprising 1 to 20 carbon atoms, preferably an alkyl group comprising 1 to 12 carbon atoms, more preferably an alkyl group containing 1 to 10 carbon atoms, more preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1, 1-dimethylpropyl, 1, 2-dimethylpropyl, 2, 2-dimethylpropyl, 1-ethylpropyl, 2-methylbutyl, 3-methylbutyl, n-hexyl, 1-ethyl-2-methylpropyl, 1, 1, 2-trimethylpropyl, 1, 1-dimethylbutyl, 1, 2-dimethylbutyl, 2, 2-dimethylbutyl, 1, 3-dimethylbutyl, 2- Ethyl butyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl,2,2-dimethylpentyl,3,3-dimethylpentyl,2-ethylpentyl,3-ethylpentyl,3-ethylpentyl,n-octyl,2,3-dimethylhexyl,2,4-dimethylhexyl,2,5-dimethylhexyl,2, 2-Dimethylhexyl, 3, 3-dimethylhexyl, 4, 4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2-diethylpentyl, n-decyl, 3, 3-diethylhexyl, 2, 2-diethylhexyl, and various branched-chain isomers. More preferably, a lower alkyl group comprising 1 to 6 carbon atoms, non-limiting embodiments comprising methyl, ethyl, n-propyl, isopropyl, n-butyl, isobutyl, tert-butyl, sec-butyl, n-pentyl, 1, 1-dimethylpropyl, 1, 2-dimethylpropyl, 2-dimethylpropyl, 1-ethylpropyl, 2-methylbutyl, 3-methylbutyl, n-hexyl, 1-ethyl-2-methylpropyl, 1, 1, 2-trimethylpropyl, 1, 1-dimethylbutyl, 1, 2-dimethylbutyl, 2, 2- Dimethylbutyl, 1,3-dimethylbutyl,2-ethylbutyl,2-methylpentyl,3-methylpentyl,4-methylpentyl group,2,3-dimethylbutyl,etc. Alkyl groups may be substituted or non-substituted, when substituted, the substituent may be substituted at any usable junction, the substituent is preferably one or more of the following groups, which are independently selected from alkyl groups, alkenyl groups, alkynyls, alkoxy, alkyl aminos, halogens, mercapto, hydroxyl, nitro, cyano, cycloalkyl, heteronyl, aryl, heteroaryl, cycloalkoxy, heteronic alkoxy, cycloalkylthio, heteronic thio, and oxo.

The term "substituted alkyl" refers to that hydrogen in an alkyl group is substituted by a substituent group, and unless otherwise stated in the text, the substituents of an alkyl group may be a variety of groups selected from the following group: -OR',-NR'R",-SR',-SiR'R"R"',-OC(O)R',-C(O)R',-CO₂R',-CONR'R",-OC(O)NR'R",-NR"C (O) R',-NR'-C(O)NR"R"',-NR"C(O)₂R',-NH-C(NH₂)=NH,-NR'C(NH₂)=NH,-NH-C(NH₂)=NR',-S(O)R',-S(O)₂R',-S(O)₂NR'R",-NR'S(O)₂R",-CN and -NO₂, with the number of substituents from 0 to (2m'+1), where m' is the total number of carbon atoms in the group. R',R"and R‴ each independently refer to hydrogen, unsubstituted C₁₋₈ alkyl, unsubstituted aryl, aryl group substituted by 1-3 halogens, unsubstituted C₁₋₈ alkyl, C₁₋₈ alkoxy or C₁₋₈ thioalkoxy, or unsubstituted aryl-C₁₋₄ alkyl. When R' and R" are attached to the same nitrogen atom, they can form a 3-, 4-, 5-, 6- or 7- ring with that nitrogen atom. For example, -NR'R" includes 1-pyrrolidinyl and 4-morpholinoyl.

The term "heteralkyl" refers to an alkyl group containing one or more heteroatoms selected from N, O or S, wherein the alkyl group is defined above.

The term "alkylene" refers to a saturated linear or branched aliphatic hydrocarbon group, which has 2 residues derived from the same carbon atom or two different carbon atoms of the parent alkane removed from two hydrogen atoms, which is a straight chain or branched chain group comprising 1 to 20 carbon atoms, preferably containing 1 to 12 carbon atoms, preferably an alkylene group containing 1 to 6 carbon atoms. Non-limiting examples of alkyl include, but are not limited to, methylene (-CH₂-,1, 1-ethylene (-CH(CH₃)-), 1, 2-ethylene (-CH₂CH₂)-, 1, 1-propylidene(-CH(CH₂CH₃)-), 1, 2-propylidene(-CH2CH(CH₃)-), 1, 3-propylidene(-CH₂CH₂CH₂-), 1, 4-butylidene(-CH₂CH₂CH₂CH₂-) and 1, 5-butylidene(- CH₂ CH₂CH₂CH₂CH₂-) and so on. Alkylene may be substituted or non-substituted, when substituted, the substituent may be substituted at any usable junction, the substituent preferably independently selected from one or more substituents in alkyl, alkenyl, alkyny, alkoxy, alkylamino, halogen, mercapto, hydroxyl, nitro, cyano, cycloalkyl, heterocyclic group, aryl, heteroarylaryl, cycloalkoxy, heterocycloalkoxy, heterocyclothio, cycloalkyl thio, heteronic sulfide and oxo.

The term "alkoxy" refers to -O- (alkyl) and -O- (cycloalkyl), where alkyl or cycloalkyl is defined as described above. Non-limiting examples of alkoxy groups include: methoxy, ethoxy, propoxy, butoxy, cyclopropoxy, cyclobutoxy, cyclopentoxy, cyclohexoxy. Alkoxy groups may be optionally substituted or non-substituted, when substituted, the substituents are preferably one or more of the following groups, which are independently selected from alkyl groups, alkenyl groups, alkynyls, alkyl aminos, halogens, mercapto, hydroxyl, nitro, cyano, cycloalkyl, heteronyl, aryl, heteroaryl, naphthenoxy, heteronic alkoxy, cycloalkylthio, heteronic thio, and heteronic sulfonyl.

The term "cycloalkyl" refers to saturated or partially unsaturated monocyclic or polycyclic hydrocarbon substituents, the cycloalkyl ring comprises 3 to 20 carbon atoms, preferably comprising 3 to 12 carbon atoms, more preferably comprising 3 to 10 carbon atoms, and most preferably comprising 3 to 8 carbon atoms. Non-limiting examples of monocyclocycloalkyl include cyclopropyl, cyclobutyl, cyclopentyl, cyclopentenyl, cyclohexenyl, cyclohexenyl, cyclohexadienyl, cycloheptyl, cycloheptyl, cycloheptyl, cycloheptyl, cyclooctyl, etc.; Polycyclic cycloalkyl groups include cycloalkyl groups of spirocyclical, polycyclic and bridge rings.

The term "spiroheterocyclic group" refers to a polycyclic heterocyclic group that shares an atom (called a spiro atom) between a single ring of 5 to 20 ring atoms, where one or more ring atoms are heteroatoms selected from nitrogen, oxygen or S (O) m (where m is integers 0 to 2), and the remaining ring atoms are carbon. It may contain one or more double bonds, but none of the rings have a fully conjugated vulon system. Preferably 6 to 14 ring atoms, more preferably 7 to 10 ring atoms. According to the number of spiral atoms shared between rings, spiral heterocyclic groups can be divided into single, double or multiple spiral heterocyclic groups, preferably single-spiral heterocyclic group and double-spiral heterocyclic group. More preferably 4 ring atoms/4 ring atoms, 4 ring atoms/5 ring atoms, 4 ring atoms/6 ring atoms, 5 ring atoms/5 ring atoms or 5 ring atoms/6 ring atoms single-spiral heterocyclic group.

The term "heterocyclic group" refers to a saturated or partially unsaturated monocyclic or polycyclic hydrocarbon substituent containing 3 to 20 ring atoms, of which one or more ring atoms are heteroatoms selected from nitrogen, oxygen, or S(O)m (where m is an integer from 0 to 2), but excluding the ring portion of -O-O-, -O-S- or -S-S-, and the remaining ring atoms are carbon. Preferably containing 3 to 12 ring atoms, of which 1~4 are heteroatoms; More preferably, the cycloalkyl ring comprises 3 to 10 ring atoms. Non-limiting examples of monocyclic heterocyclics include pyrrolidinyl, piperidinyl, piperazinyl, morpholinoyl, thiomorpholinoline, homopiperazinyl and the like. Polycyclic heterocyclic groups include heterocyclic groups of spirocyclic rings, poly rings, and bridge rings.

The term "cycloalkylalkyl" means that an alkyl group is replaced by one or more cycloalkyl groups, preferably by one, where an alkyl group is defined above, and where a cycloalkyl group is defined above.

The term "alkyl halide" refers to the substitution of alkyl by one or more halogens, of which alkyl is defined above.

The term "deuteryl alkyl" refers to the substitution of an alkyl group with one or more deuterium atoms, with alkyl groups as defined above.

The term "hydroxyl" refers to the -OH group.

The term "halogen" refers to fluorine, chlorine, bromine, or iodine.

The term amino refers to NH₂. The term "nitro" refers to -NO₂.

The term "amide group" means -C(O)N(alkyl) or (cycloalkyl), where alkyl, cycloalkyl, is defined above.

The term "carboxylate group" means -C(O)O(alkyl) or (cycloalkyl), where alkyl, cycloalkyl is defined above.

The invention also includes various forms of deuteration. Each available hydrogen atom attached to a carbon atom can be independently replaced by a deuterium atom. A person skilled in the art of this field can synthesize deuterated compounds by referring to relevant literature. In the preparation of deuterium compounds, commercially available deuterium initiation materials may be used, or they may be synthesized using conventional techniques using deuterium reagents. Unrestricted examples of deuterium reagents include: deuterium methanol, deuterium water, deuterium borane, trideuterium borane tetrahydrofuran solution, deuterium aluminum hydride, deuterium iodide ethane and deuterium iodide methane.

The term "antibody" refers to immunoglobulin, a tetrapeptide structure consisting of two identical heavy chains and two identical light chains connected by interchain disulfide bonds. The amino acid composition and sequence of the heavy chain constant region of immunoglobulin are different, so its antigenicity is also different. On this basis, immunoglobulins can be divided into five classes, or isotypes of immunoglobulins, namely IgM, IgD, IgG, IgA, and IgE, with their corresponding heavy chains being µ, δ, γ, α, and ε chain. Ig can be divided into different subgroups according to the amino acid composition of hinge region and the number and location of heavy chain disulfide bonds. For example, IgG can be divided into IgG1, IgG2, IgG3, and IgG4. The light chain is divided into κ chain or λ chain through the difference of the constant region. Each of the five types of Ig can have a κ chain or a λ chain. The antibody of the invention is preferably a specific antibody against the cell surface antigen on the target cell, and the non-restrictive embodiments are the following: Anti-egfrviii antibody, anti-DLL-3 antibody, anti-Psma antibody, anti-CD70 antibody, anti-MUC16 antibody, anti-ENPP3 antibody, anti-tDGF1 antibody, anti-ETbr antibody, anti-MSLn antibody, anti-Tim-1 antibody, anti-LrRC15 antibody, anti-Liv-1 antibody, anti-Canag /AFP antibody , anti-cladin 18.2 Antibody, anti-Mesothelin antibody, anti-HER2 (ErbB2) antibody, anti-EGFR antibody, anti-C-Met antibody, anti-SlitrK6 antibody, anti-Kit /CD117 antibody, anti-Steap1 antibody, Anti-SlamF7 /CS1 antibody, anti-Napi2b /SLC34A2 antibody, Anti- GPNMB antibody, anti-HER3 (ErbB3) antibody, anti-MLTC1 /CD227 antibody, anti-Axl antibody, anti-CD166 antibody, anti-B7-H3 (CD276) antibody, anti-PTK7 /CCK4 antibody, anti-PRLR antibody, anti-EFna4 antibody, anti-5T4 antibody, anti-NotCH3 antibody, anti-NEC antibody tin 4 Antibody, anti-Trop-2 antibody, anti-CD142 antibody, anti-CA6 antibody, anti-GPR20 antibody, anti-CD174 antibody, anti-CD71 antibody, anti-Epha2 antibody, anti-LypD3 antibody, anti-FGFR2 antibody, anti-FGFR3 antibody, anti-Frα antibody, anti-Ceacams antibody, anti-GCC antibody, anti-IN antibody tegrin Av antibody, anti-Caix antibody, anti-P-Cadherin antibody, anti-GD3 antibody, anti-Cadherin antibody 6 Antibody, anti-LAMp1 antibody, anti-flT3 antibody, anti-BCMA antibody, anti-CD79B antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody or one or more anti-CD123 antibodies; preferably Trastuzumab (trade name Herceptin), Pertuzumab (also known as 2C4, trade name Perjeta), Nimotuzumab (Nimotuzumab, Trade name tai xin), Enoblituzumab Emibetuzumab, Inotuzumab, Pinatuzumab Brentuximab, Gemtuzumab Bivatuzumab, Lorvotuzumab cBR96 and Glematumamab.

The term "solvate" or "solvent compound" means that the ligand-drug conjugate of the present invention forms a medically-usable solvate with one or more solvent molecules, non-restrictive examples of which include water, ethanol, acetonitrile, isopropyl alcohol, DMSO, ethyl acetate.

The term "drug load" refers to the average number of cytotoxic drugs loaded on each antibody in the Formula I molecule, and can also be expressed as the ratio of drug dose to antibody dose. The drug load can range from 0 to 12 for each antibody (Ab), preferably 1 to 10 cytotoxic drugs (D). In the embodiments of the invention, the drug load is expressed as n, which, exemplary, can be the mean of 1, 2, 3, 4, 5, 6, 7, 8, 9, 10. Conventional methods such as UV/visible light spectroscopy, mass spectrometry, ELISA assay, and HPLC characterization were used to identify the average amount of drug for each ADC molecule after coupling reaction.

In one embodiment of the present invention, a cytotoxic drug is coupled to the n-terminal amino group of a ligand and/or the ε-amino group of a lysine residue by a linking unit. In general, the number of drug molecules that can be coupled to an antibody in the coupling reaction will be less than the theoretical maximum.

The following non-limiting methods can be used to control the load of ligand cytotoxic drug conjugates, including:
(1) Control the molar ratio of linking reagent and monoclonal antibody,
(2) Control reaction time and temperature,
(3) Select different reaction reagents.

See Chinese Pharmacopoeia for the preparation of conventional pharmaceutical compositions.

The term "pharmaceutically acceptable salt" or "medicinal salt" means the salt of the ligand-drug conjugate of the invention, or the salt of a compound described in the invention, which is safe and effective when used in mammals and has the expected biological activity. The ligand-drug conjugate of the invention contains at least one carboxyl group and can therefore form a salt with a base. Non-restrictive examples of pharmaceutically acceptable salts include: sodium salts, potassium salts, calcium salts or magnesium salts.

The term "pharmaceutically acceptable salt" or "medicinal salt" refers to a salt of a ligand-drug conjugate of the present invention, or a salt of a compound described in the present invention, which is safe and effective when used in mammals and has the expected biological activity. The ligand-drug conjugate compound of the present invention contains at least one amino group and can therefore form salts with acids. Non-restrictive examples of pharmaceutically acceptable salts include: hydrochloride, hydrobromide, hydroiodate, sulfate, bisulfate, citrate, acetate, succinate, ascorbate, oxalate, nitrate, pearate, hydrogen phosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

"Acidic amino acid" means that the isoelectric point of amino acid is less than 7, and acidic amino acid molecules often have one or more acidic groups such as carboxyl groups, which can effectively ionize into negative ion form and increase hydrophilicity in the structure. Acidic amino acids can be natural or unnatural.

"Natural amino acids" refer to amino acids that are biosynthesized. Natural amino acids are generally L-type, but there are a few exceptions, such as glycine, both natural and biosynthetic.

"Unnatural amino acids" refer to amino acids obtained by synthetic means.

The present invention is further elaborated below in conjunction with specific embodiments, it should be understood that these embodiments are only used to illustrate the present invention, and are not intended to limit the scope of the present invention. The following embodiments do not indicate specific conditions of the test method, usually in accordance with conventional conditions or in accordance with the conditions recommended by the manufacturer. Unless otherwise stated, all percentages, proportions, ratios, or servings are by weight.

Unless otherwise defined, all professions and sciences used herein are used in the same sense as those familiar to those skilled in the art.

Further, any method and material similar or equal to the content described may be applied to the method of the present invention. The best practices and materials described herein are for demonstration purposes only.

### Example 1: Synthesis of Compound 1 and iso-1

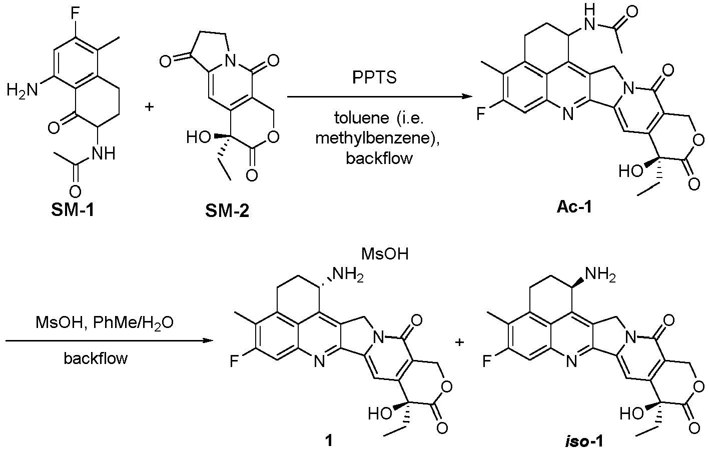

Add Compound SM-1 (N-(8-amino-6-fluoro-5-methyl-1-oxo-1,2,3,4-tetrahydronaphthal-2-yl-acetamide, purchased) (6.25g, 25.0mmol), Compound SM-2((S)-4-ethyl-4-hydroxy-7,8-dihydro-1H-pyran O[3,4-F]indolazine-3,6,10(4H)-ketone, purchased) (7.90g, 30.0mmol), and Pyridine p-toluenesulfonate salt (1.26g, 5.0mmol) into a 1L round-bottom flask. Add 500mL of toluene, heat reflux for water distribution, react for 6 hours, HPLC monitoring reaction until the disappearance of SM-2, stop the reaction. Cool, stir in ice bath and crystallization-precipitate for 2h, filtrate and vacuum dry to obtain compound **Ac-1:** 10.68g, 92%. LC-MS yields [M+H]⁺:478.2.

**Ac-1** (11.02g, 23.0mmol), mesylate (55mL), water (110mL) and toluene (55mL) were mixed into a 1L round-bottomed flask and heated for reflux. 6 hours later, TLC monitoring the reaction to ensure the raw material disappears. At room temperature, 500mL methanol was slowly added to the system and continuously stir until the drip was finished. Stir overnight. The reaction system was directly filtered, the filter cake was washed with methanol, the filtrate was collected, and the filter cake was bake-dried to obtain gray-white solid Compound I (6.58g, 54%). LC-MS to obtain [M+H]⁺:436.2. De-pressure and concentrate the filtrate, prepare and purify the liquid phase ((MeCN/H2O as mobile phase), lyophilize to obtain light brown solid compound ***iso***-1 (3.71g, 37%) was obtained by liquid phase preparation and purification (MeCN/H2O as mobile phase). LC-MS to obtain [M+H]⁺:436.2.

### Example 2: Synthesis of Compound D-D1

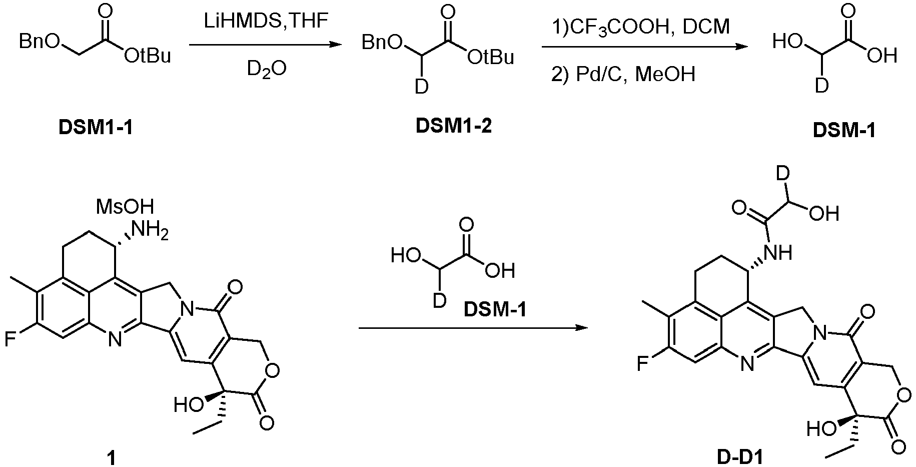

Replace the No. 1 1000ml three-necked bottle with nitrogen and protect with a nitrogen balloon, and install a constant pressure dropping funnel device. Use a syringe to add heavy water (25ml, 1.25mol) into the bottle, and stir at 0°C for later use. Put the No. 2 500ml three-necked bottle under Nitrogen balloon protection, add 1.0M LiHMDS THF solution (300ml, 0.30mol), place at 0°C and stir to cool down. Slowly add dropwise DSM1-1 (12.00g, 54.0 mmol), react for 5min after the dropwise addition, withdraw the liquid by the syringe, slowly add all to the No. 1 bottle with constant pressure dropping funnel and drop the liquid into the cooled heavy water for about 15 minutes. After the dropwise addition, stir for 5 minutes, add 300ml of saturated ammonium chloride, extracted 3 times with ethyl acetate, combine the organic phase, wash with water once, dried with anhydrous sodium sulfate, concentrate and then separate by column chromatography. 8.33g of colorless oily liquid DSM1-2 was isolated with a yield of 69%. ¹H-NMR for detection, the deuterated status is determined by the integral number of carbonyl α-position hydrogen, and it is confirmed that the undeuterated part is about 1.7%.

Add 50ml of dichloromethane and DSM1-2 (8.33g, 37.3mmol) into a 250ml single-necked bottle, stir at 0°C, add 50ml of TFA, bring to room temperature and stir for about 1h, TLC detects that the reaction is complete, and reduce pressure at 30°C Concentrate the reaction solution, and use DCM to take out the residual TFA, and dry it under reduced pressure with an oil pump for 15 minutes to obtain a crude oil product, which is directly used in the next step. Add 100ml of methanol to the crude product to dissolve, add 10% Pd/C (5.00g), and replace with hydrogen and add a hydrogen balloon device, put it at room temperature to react for about 2 hours, TLC detects that the raw materials disappear, filter with suction, and wash the filter cake with methanol twice. Combine the filtrates, concentrate under reduced pressure, and dry under reduced pressure with an oil pump to obtain light yellow oily liquid DSM-1 (2.85 g, 99%) to be used directly.

Compound 1 was added into a 25ml round bottom flask (100.3mg, 0.19mmol), DSM-1 (43.0mg, 0.56mmol), HATU (107.2mg, 0.28mmol), HOBt (38.1mg, 0.28mmol) and 5ml ultra Dry DMF, seal the bottle with liquid. Stir in an ice-water bath for 10 minutes, then add DIEA (95uL, 0.57mmol), and bring it up to room temperature and stir. HPLC detects the reaction progress, the reaction is about 3h, the raw material is less than 5%, and the reaction system liquid phases were prepared and separated, and lyophilized to obtain a yellow solid D-D1: 64.0mg, with a yield of 69%.
LC-MS results show [M+H]+: 495.2.
¹H NMR (400 MHz, DMSO-d6) δ 8.38 (d, J = 9.2 Hz, 1H), 7.64 (d, J = 11.2 Hz, 1H), 7.25 (s, 1H), 6.51 (s, 1H), 5.60-5.41 (m, 2H), 5.39 (s, 2H), 5.09 (d, J = 18.8 Hz, 1H), 4.92 (d, J = 18.8 Hz, 1H), 3.97(s, 1H), 3.22-2.98 (m, 2H), 2.29 (s, 3H), 2.22-2.08 (m, 2H), 1.95-1.73 (m, 2H), 0.86 (t, J = 7.2 Hz, 3H).

### Example 3: Synthesis of Compound D-D2

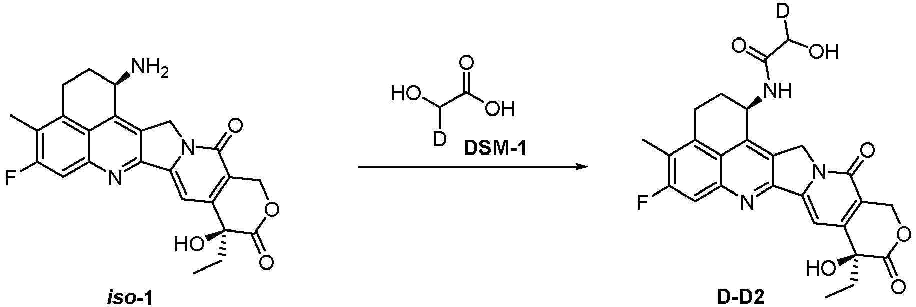

Add Compound iso-1 (82.5mg, 0.19mmol), DSM-1 (43.1mg, 0.56mmol), HATU (107.2mg, 0.28mmol), HOBt (38.2mg, 0.28mmol) and 5ml ultra-dry DMF, liquid seal the bottle mouth. Stir it under ice-water bath for 10 minutes, then add DIEA (95uL, 0.57mmol), and stir at room temperature. HPLC detects the reaction progress, the reaction is about 3h, the raw material is less than 5%. The reaction system liquid phase was prepared and separated, and freeze-dried to obtain a yellow solid D-D2: 67.8 mg, with a yield of 73%.
LC-MS results show [M+H]+: 495.1.
¹H NMR (400 MHz, DMSO-d6) δ 8.38 (d, J = 9.0 Hz, 1H), 7.65 (d, J = 10.9 Hz, 1H), 7.25 (s, 1H), 6.51 (s, 1H), 5.53 (dt, J = 9.1, 6.0 Hz, 1H), 5.41 (s, 2H), 5.12 (d, J = 19.1 Hz, 1H), 4.92 (d, J = 18.9 Hz, 1H), 3.99 (s, 1H), 3.10 (qt, J = 16.7, 6.1 Hz, 2H), 2.29 (d, J = 1.8 Hz, 3H), 2.16 (q, J = 6.4 Hz, 2H), 2.00 - 1.81 (m, 2H), 0.91 (t, J = 7.3 Hz, 3H).

### Example 4: Synthesis of Compound D-D3

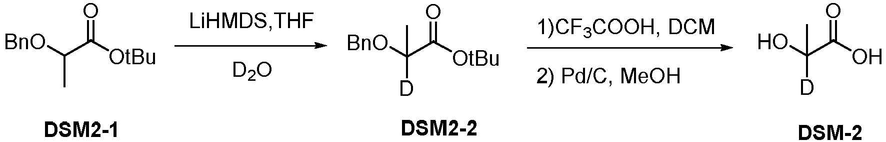

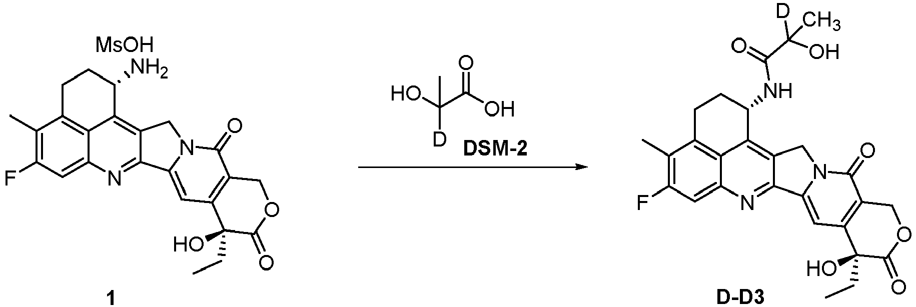

Replace the No. 1 1000ml three-necked bottle with nitrogen and protect it with a nitrogen balloon, and equip a constant pressure dropping funnel. Use a syringe to add heavy water (20ml, 1.00mol) into the bottle, and stir at 0°C for later use. Put the No. 2 500ml three-necked bottle with nitrogen ball protection, add 1.0M LiHMDS THF solution (200ml, 0.20mol), place at 0°C and stir to cool down. Slowly add DSM2-1 (10.00g, 42.3 mmol), react for Smin after the dropwise addition, and draw out the reactions with the syringe, slowly add to the No. 1 bottle using the constant pressure dropping funnel to add dropwise to the cooled heavy water for about 12 minutes. After the completion of drop-wise addition, stir for 5 minutes, and add 200ml of saturated ammonium chloride, ethyl acetate extract for 3 times, combine the organic phase, wash the organic phase once with water, dry over with anhydrous sodium sulfate, concentrate and separate by column chromatography. 5.07g of colorless oily liquid DSM2-2 was isolated, and the yield was 50%. ¹H-NMR detection, determining the deuterium replacement by the integral number of carbonyl α-position hydrogen. The carbonyl α-position hydrogen almost disappeared confirming that the undeuterated part is about 1%.

Add 40ml of dichloromethane and DSM2-2 (5.07g, 21.4mmol) into a 150ml single-necked bottle, stir at 0°C, add 20ml of TFA, bring to room temperature and stir for about 1h, TLC detects that the reaction is complete, and reduce pressure at 30°C. Concentrate the reaction solution, and use DCM to take out the residual TFA, and dry it under reduced pressure with an oil pump for 15 minutes to obtain a crude oil product, which is directly used in the next step. Add 50ml of methanol to the crude product to dissolve, add 10% Pd/C (2.00g), and replace with hydrogen, add a hydrogen balloon device, put it at room temperature for about 2 hours, TLC detects that the raw materials disappear, filter with suction, and wash the filter cake with methanol twice. Combine the filtrates, concentrate under reduced pressure, and dry under reduced pressure with an oil pump to obtain light yellow oily liquid DSM-2 (1.84 g, 94%) to be used directly.

Into a 25ml round bottom flask was added Compound 1 (100.4mg, 0.19mmol), DSM-2 (51.3mg, 0.56mmol), HATU (107.1mg, 0.28mmol), HOBt (38.3mg, 0.28mmol) and 5ml ultra dry DMF, seal the bottle with liquid. Stir it in an ice-water bath for 10 minutes, then add DIEA (95uL, 0.57mmol), and bring it up to room temperature and stir. HPLC detects the reaction progress, the reaction is about 3h, the raw material is less than 5%, and the reaction system liquid phases were prepared and separated, and lyophilized to obtain 75.9 mg of a yellow solid, with a yield of 79%.
LC-MS results show [M+H]⁺: 509.2.
¹H NMR (400 MHz, DMSO-d6) δ 8.35 (d, *J* = 8.8 Hz, 1H), 7.64 (d, *J* = 10.8 Hz, 1H), 7.26 (s, 1H), 6.53 (s, 1H), 5.56 (d, *J* = 5.2 Hz, 1H), 5.48(dd, *J* = 14.0, 6.4 Hz, 1H), 5.40(s, 2H), 5.09 (d, J = 18.8 Hz, 1H), 4.89 (d, J = 18.8 Hz, 1H), 3.22-3.11 (m, 1H), 3.11-3.00 (m, 1H), 2.29 (s, 3H), 2.22-2.09 (m, 2H), 1.92-1.78 (m, 2H), 1.32 (s, 3H), 0.87 (t, J = 7.2 Hz, 3H).

### Example 5: Synthesis of Compound D-D4

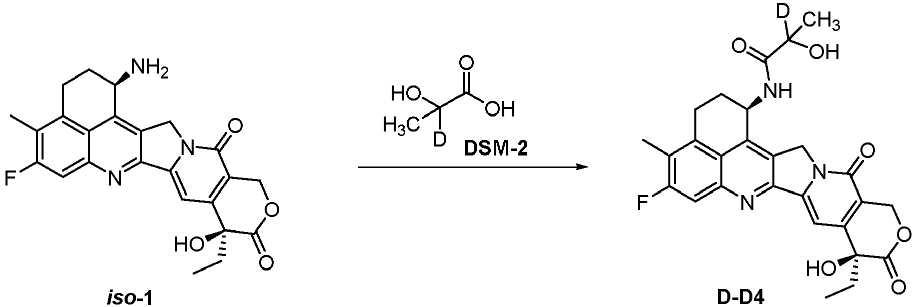

Add Compound iso-1 (82.8mg, 0.19mmol), DSM-2 (50.9mg, 0.56mmol), HATU (107.4mg, 0.28mmol), HOBt (38.0mg, 0.28mmol) and 5ml ultra- Dry DMF, seal the bottle with liquid. Stir in an ice-water bath for 10 minutes, then add DIEA(95uL, 0.57mmol), and bring up to room temperature and stir. HPLC detects the reaction progress, the reaction is about 3h, the raw material is less than 5%, the reaction system liquid phase were prepared and separated, and lyophilized to obtain 70.7 mg of a yellow solid, with a yield of 74%.
LC-MS results show [M+H]+: 509.3.
¹HNMR (400 MHz, DMSO-d6) δ 8.46 (d, J = 9.2 Hz, 1H), 7.71 (d, J = 10.9 Hz, 1H), 7.29 (s, 1H), 6.53 (s, 1H), 5.68 (d, J = 4.9 Hz, 1H), 5.54 (q, J = 7.3 Hz, 1H), 5.42 (s, 2H), 5.20 (d, J = 19.0 Hz, 1H), 4.93 (d, J = 18.9 Hz, 1H), 3.25 - 3.01 (m, 2H), 2.32 (s, 3H), 2.14 (q, J = 6.5 Hz, 2H), 1.96 - 1.80 (m, J = 7.1 Hz, 2H), 1.42 (s, 3H), 0.90 (t, J = 7.2 Hz, 3H).

### Example 6: Synthesis of Compound D-D5

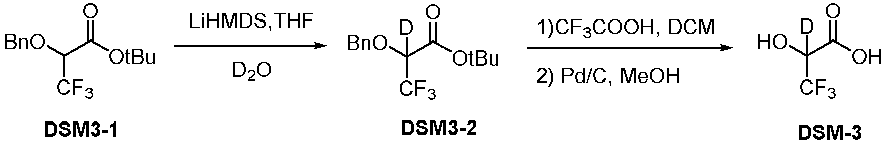

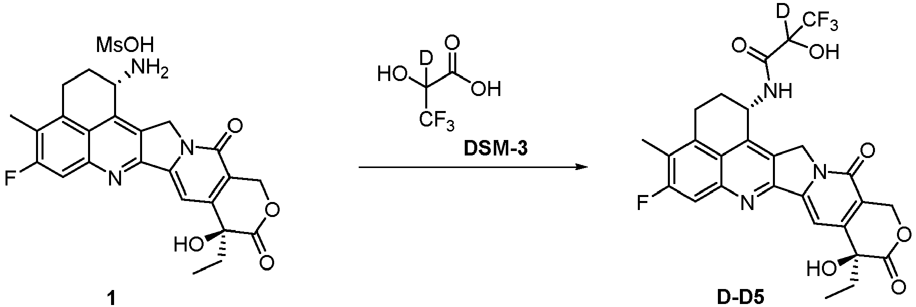

Replace the No. 1 1000ml three-necked bottle with nitrogen and protect it with a nitrogen balloon, and equip a constant pressure dropping funnel. Add heavy water (30ml, 1.50mol) into the bottle with a syringe, and stir at 0°C for later use. Put the No. 2 500ml three-necked bottle with nitrogen ball protection, add 1.0M LiHMDS THF solution (350ml, 0.35mol), place at 0°C and stir to cool down. Slowly add DSM3-1 (20.32g, 70.0 mmol), react for Smin after the dropwise addition, and draw out reaction system with the syringe, slowly add to the No. 1 bottle via the constant pressure dropping funnel and add dropwise to the cooled heavy water for about 12 minutes. After the dropwise addition was completed, stirred for 5 minutes, and added 350ml of saturated ammonium chloride, extracts 3 times with ethyl acetate, combine the organic phase, wash the organic phase once with water, dried over anhydrous sodium sulfate, concentrate and separate by column chromatography. 7.15g of light-yellow oily liquid DSM3-2 was isolated with a yield of 35%. ¹H-NMR detection, measure the deuterium by the integral number of carbonyl α-position hydrogen, it is found that the carbonyl α-position hydrogen almost disappears, and it is confirmed that the undeuterated part is about 1%.

Add 40ml of dichloromethane and DSM3-2 (7.15g, 24.5mmol) into a 150ml single-necked bottle, stir at 0°C, add 20ml of TFA, bring to room temperature and stir for about 1h, TLC detects that the reaction is complete, and reduce pressure at 30°C Concentrate the reaction solution, and use DCM to take out the residual TFA, and dry it under reduced pressure with an oil pump for 15 minutes to obtain a crude oil product, which is directly used in the next step. Add 50ml of methanol to the crude product to dissolve, add 10% Pd/C (3.50g), and replace with hydrogen and add a hydrogen balloon device, put it at room temperature to react for about 2 hours, TLC detects that the raw materials disappear, filter with suction, and wash the filter cake twice with methanol. Combine the filtrates, concentrate under reduced pressure, and dry under reduced pressure with an oil pump to obtain light yellow oily liquid DSM-3 (3.54 g, 97%) to use directly.

Add Compound 1 (100.8mg, 0.19mmol), DSM-3 (81.3mg, 0.56mmol), HATU (106.8mg, 0.28mmol), HOBt (38.5mg, 0.28mmol) and 5ml ultra-dry DMF to a 25ml round bottom flask, the bottle mouth is liquid-sealed. Stir in an ice-water bath for 10 minutes, then add DIEA (95uL, 0.57mmol), and bring it to room temperature and stir. HPLC detects the reaction progress, the reaction is about 3h, the raw material is less than 5%, and the reaction system liquid phase is prepared and separated, and freeze-dried to obtain 63.1 mg of yellow solid with yield 59%.
LC-MS results show [M+H]+: 562.1.

### Example 7: Synthesis of Compound D-D6

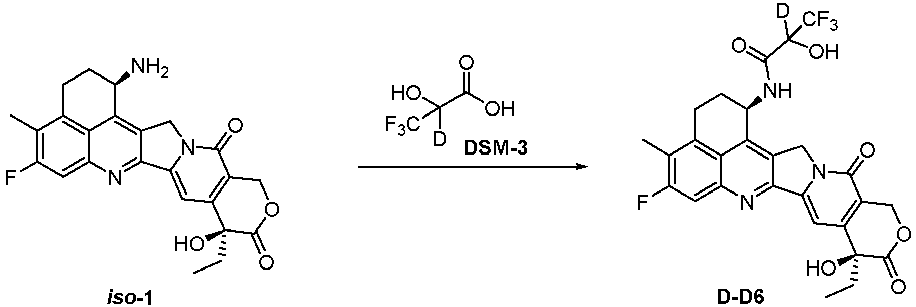

Add Compound iso-1 (82.3mg, 0.19mmol), DSM-3 (81.5mg, 0.56mmol), HATU (107.2mg, 0.28mmol), HOBt (38.3mg, 0.28mmol) and 5ml ultra- Dry DMF, seal the bottle with liquid. Stir it in an ice-water bath for 10 minutes, then add DIEA (95uL, 0.57mmol), and bring up to room temperature and stir. HPLC detects the reaction progress, the reaction is about 3h, the raw material is less than 5%, the reaction system liquid phase was prepared and separated, and freeze-dried to obtain 66.1 mg of a yellow solid, with a yield of 62%.
LC-MS results show [M+H]+:562.2.

### Example 8: Synthesis of Compound D-D7

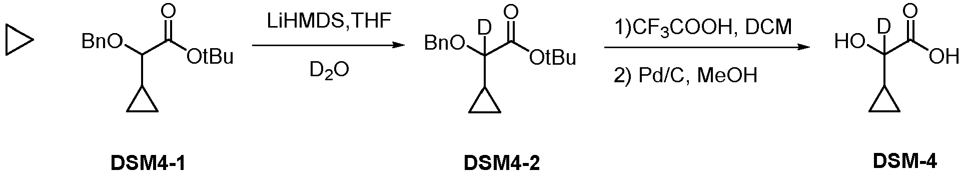

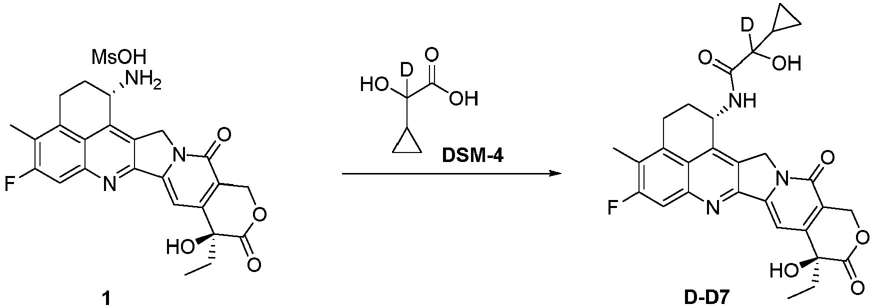

Replace the No. 1 1000ml three-necked bottle with nitrogen and protect it with a nitrogen balloon, and equip a constant pressure dropping funnel. Add heavy water (30ml, 1.50mol) into the bottle with a syringe, and stir at 0°C for later use. Put the No. 2 500ml three-necked bottle with nitrogen Ball protection, add 1.0M LiHMDS THF solution (350ml, 0.35mol), place at 0°C and stir to cool down. Slowly add DSM4-1 (18.36g, 70.0 mmol) to wherein, react for Smin after the dropwise addition, and draw out the reactions with the syringe, slowly add to the No. 1 bottle via the constant pressure dropping funnel and add dropwise to the cooled heavy water for about 12 minutes. After the dropwise addition was completed, stir for 5 minutes, and add 350ml of saturated ammonium chloride, extract 3 times with ethyl acetate, combine the organic phases, wash the organic phase once with water, dry over anhydrous sodium sulfate, concentrate and separate by column chromatography. 7.58g of light-yellow oily liquid DSM4-2 was isolated, and the yield was 41%. Using ¹H-NMR detection, measuring the deuterium by the integral number of carbonyl α-position hydrogen, it was found that the carbonyl α-position hydrogen almost disappeared, and the undeuterated portion was confirmed to be about 0.7%.

Add 60ml of dichloromethane and DSM4-2 (7.58g, 28.8mmol) into a 150ml single-necked bottle, stir at 0°C, add 20ml of TFA, bring to room temperature and stir for about 1h, TLC detects that the reaction is complete, and reduce pressure at 30°C. Concentrate the reaction solution, and use DCM to take out residual TFA, and dry it under reduced pressure with an oil pump for 15 minutes to obtain a crude oil product, which is directly used in the next step. Add 50ml of methanol to the crude product to dissolve, add 10% Pd/C (4.01g), and replace with hydrogen and add a hydrogen balloon device, put it at room temperature to react for about 2 hours, TLC detects that the raw materials disappear, filter with suction, and wash the filter cake with methanol twice. Combine the filtrates, concentrate under reduced pressure, and dry under reduced pressure with an oil pump to obtain almost colorless oily liquid DSM-4 ( 3.27g, 97%) used directly.

Add Compound 1 (100.7mg, 0.19mmol), DSM-4 (65.9mg, 0.56mmol), HATU (107.8mg, 0.28mmol), HOBt (38.9mg, 0.28mmol) and 5ml ultra-dry DMF to a 25ml round bottom flask, the bottle mouth is liquid-sealed. Stir it in an ice-water bath for 10 minutes, then add DIEA (95uL, 0.57mmol), and bring it to room temperature and stir. HPLC detects the reaction progress, the reaction is about 3h, the raw material is less than 5%, and the reaction system liquid phase is prepared and separated and freeze-dried to obtain 71.3 mg of yellow solid with yield of 70%.
LC-MS results show [M+H]+: 535.2.

### Example 9: Synthesis of Compound D-D8

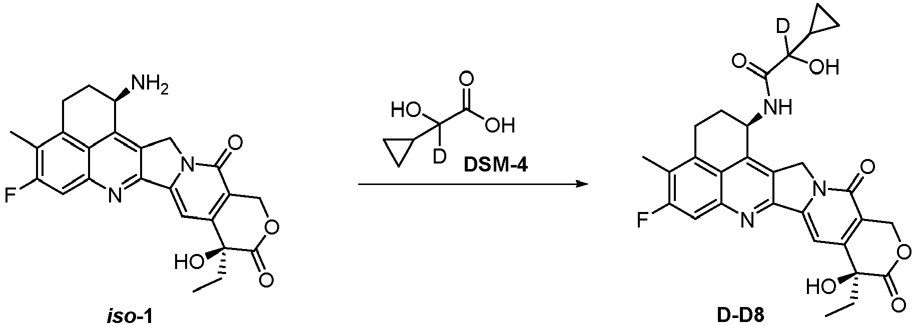

Add Compound iso-1 (82.7mg, 0.19mmol), DSM-4 (65.6mg, 0.56mmol), HATU (107.3mg, 0.28mmol), HOBt (38.9mg, 0.28mmol) and 5ml ultra- Dry DMF to a 25ml round bottom flask, seal the flask with liquid. Stir it in an ice-water bath for 10 minutes, then add DIEA (95uL, 0.57mmol), and bring it up to room temperature and stir. HPLC detects the reaction progress, the reaction is about 3h, the raw material is less than 5%, the reaction system liquid phases are prepared and separated, and lyophilized to obtain 65.3 mg of a yellow solid, with a yield of 64%.
LC-MS results show [M+H]⁺: 535.2.

### Example 10: Synthesis of Compound D-D9

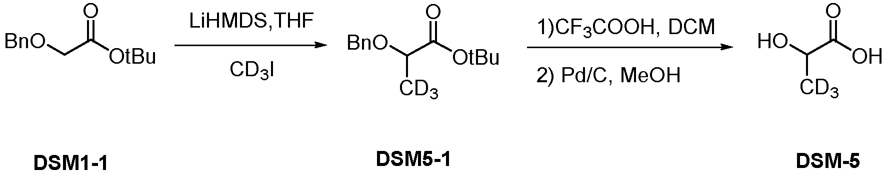

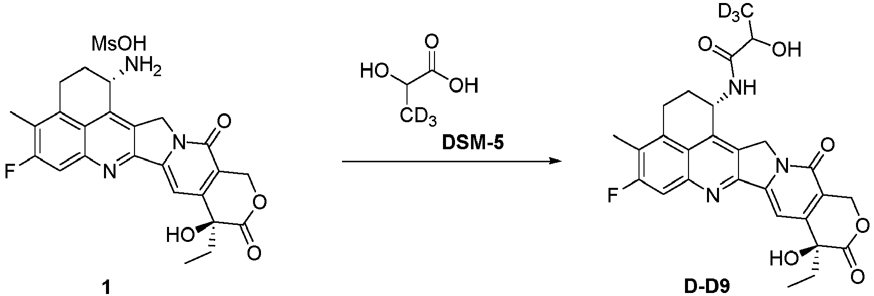

Protect the 1L three-neck flask with a nitrogen balloon, add 300mL of newly distilled THF and 1.0M LiHMDS THF solution (200ml, 0.20mol), place it at -78°C and stir to cool down. Slowly add DSM1-1 (22.23g, 0.10 mol), react for 30 minutes after the dropwise addition, add CD3I (10mL, 0.20mol). After the dropwise addition, stir for 15min, raise the reaction to room temperature and stir for another 15min, add 300ml of saturated ammonium chloride to the reaction system to quench the reaction, extract 3 times with ethyl acetate, combine the organic phases, dried over anhydrous sodium sulfate, concentrate and separate by column chromatography. 13.88g of light-yellow oily liquid DSM5-1 was isolated, with a yield of 58%.

Add 40ml of dichloromethane and DSMS-1 (4.79g, 20.0mmol) to a 150ml single-necked bottle, stir at 0°C, add 20ml of TFA, bring to room temperature and stir for about 1h, TLC detects that the reaction is complete, and reduce pressure at 30°C Concentrate the reaction solution, and use DCM to take out residual TFA, and dry it under reduced pressure with an oil pump for 15 minutes to obtain a crude oil product, which is directly used in the next step. Add 50ml of methanol to the crude product to dissolve, add 10% Pd/C (2.56g), and replace with hydrogen and add a hydrogen balloon device, put it at room temperature to react for about 2 hours, TLC detects that the raw materials disappear, filter with suction, and wash the filter cake with methanol twice. Combine the filtrates, concentrate under reduced pressure, and dry under reduced pressure with an oil pump to obtain almost colorless oily liquid DSM-5 ( 1.83g, 98%) for direct use.

Add Compound 1 (100.5mg, 0.19mmol), DSM-5 (52.6mg, 0.56mmol), HATU (107.6mg, 0.28mmol), HOBt (38.7mg, 0.28mmol) and 5ml ultra-dry DMF to a 25ml round bottom flask , the bottle mouth was sealed with liquid. Stir it in an ice-water bath for 10 minutes, then add DIEA (95uL, 0.57mmol), and bring up to room temperature and stir. HPLC detects the reaction progress, the reaction is about 3h, the raw material is less than 5%, and the reaction system liquid phase is prepared and separated and freeze-dried to obtain 66.9mg of yellow solid, with yield of 69%.
LC-MS results show [M+H]+: 511.2.

### Example 11: Synthesis of Compound D-D10

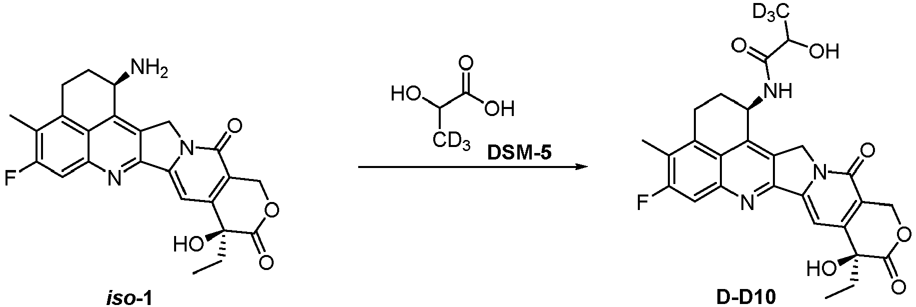

Add Compound iso-1 (82.6mg, 0.19mmol), DSM-5 (52.3mg, 0.56mmol), HATU (107.4mg, 0.28mmol), HOBt (38.2mg, 0.28mmol) and 5ml ultra- Dry DMF, seal the bottle with liquid. Stir in an ice-water bath for 10 minutes, then add DIEA (95uL, 0.57mmol), and bring up to room temperature and stir. HPLC detects the reaction progress, the reaction is about 3h, the raw material is less than 5%, the reaction system liquid phases were separated and freeze-dried to obtain 70.8 mg of a yellow solid, with a yield of 73%.
LC-MS results show [M+H]⁺: 511.1.

### Example 12: Synthesis of Compound D-D11

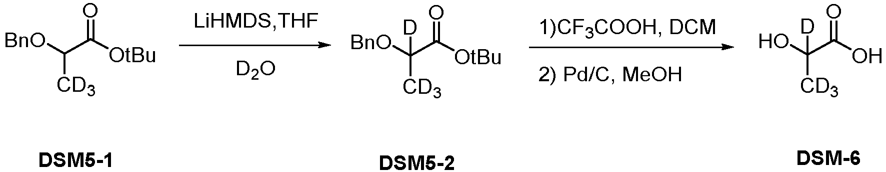

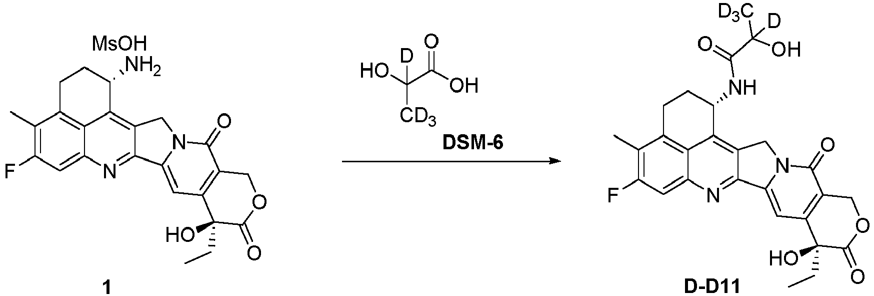

To a No. 1 500ml three-necked bottle, perform nitrogen replacement, protect it with a nitrogen balloon, and equip a constant pressure dropping funnel. Add heavy water (deuteroxide) (20ml, 1.0mol) into the bottle with a syringe, and stir at 0°C for later use. Put the No. 2 250ml three-necked bottle with nitrogen ball protection, add 1.0M LiHMDS THF solution (125ml, 0.125mol), place at 0°C and stir to cool down. Slowly add DSMS-1 (5.99g, 25.0 mmol) to wherein, react for Smin after the dropwise addition, and draw out the reactions from the No. 2 bottle with the syringe, slowly add to the No. 1 bottle via the constant pressure dropping funnel and add dropwise to the cooled heavy water for about 12 minutes. After the dropwise addition is completed, stir for 5 minutes, add 150ml saturated ammonium chloride, use ethyl acetate to extract 3 times, combine the organic phases, wash the combined organic phase once with water, dry over anhydrous sodium sulfate, concentrate and separate by column chromatography. 3.11g of light-yellow oily liquid DSM5-2 was isolated, and the yield was 52%. ¹H-NMR detection, measuring the deuterium replacement by the integral number of carbonyl α-position hydrogen, it was found that the carbonyl α-position hydrogen almost disappeared, and the undeuterated portion was confirmed to be about 1.5%.

Add 30ml of dichloromethane and DSMS-2 (3.11g, 12.6mmol) into a 100ml single-necked bottle, stir at 0°C, add 10ml of TFA, bring to room temperature and stir for about 1h, TLC detects that the reaction is complete, and reduce pressure at 30°C. Concentrate the reaction solution, and use DCM to take out residual TFA, and dry it under reduced pressure with an oil pump for 15 minutes to obtain a crude oil product, which is directly used in the next step. Add 30ml of methanol to the crude product to dissolve, add 10% Pd/C (1.50g), and replace with hydrogen and add a hydrogen balloon device, put it at room temperature to react for about 2 hours, TLC detects that the raw materials disappear, filter with suction, and wash the filter cake with methanol twice. Combine the filtrates, concentrate under reduced pressure, and dry under reduced pressure with an oil pump to obtain almost colorless oily liquid DSM-6 ( 1.15g, 97%) to be used directly.

Add Compound 1 (101.1mg, 0.19mmol), DSM-6 (52.7mg, 0.56mmol), HATU (108.1mg, 0.28mmol), HOBt (38.7mg, 0.28mmol) and 5ml ultra-dry DMF to a 25ml round bottom flask, the bottle mouth is liquid-sealed. Stir it in an ice-water bath for 10 minutes, then add DIEA (95uL, 0.57mmol), and bring it to room temperature and stir. HPLC detects the reaction progress, the reaction is about 3h, the raw material is less than 5%, and the reaction system liquid phase is prepared and separated and freeze-dried to obtain 66.1 mg of yellow solid with yield of 68%.
LC-MS results show [M+H]⁺: 512.2.

### Example 13: Synthesis of Compound D-D12

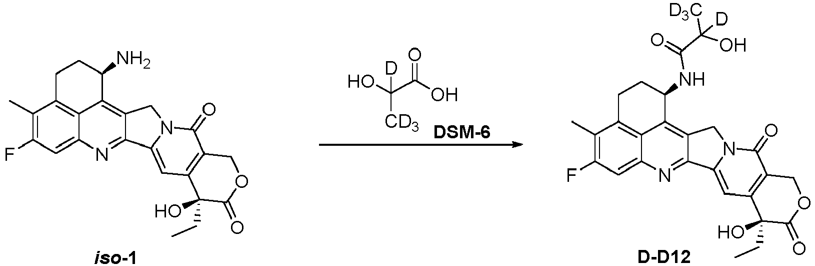

Add Compound iso-1 (82.7mg, 0.19mmol), DSM-6 (52.9mg, 0.56mmol), HATU (107.4mg, 0.28mmol), HOBt (38.2mg, 0.28mmol) and 5ml ultra- Dry DMF, seal the bottle with liquid. Stir it in an ice-water bath for 10 minutes, then add DIEA (95uL, 0.57mmol), and bring it up to room temperature and stir. HPLC detects the reaction progress, the reaction is about 3h, the raw material is less than 5%, the reaction system liquid phases were prepared and separated and freeze-dried to obtain 70.8 mg of a yellow solid, with a yield of 73%.
LC-MS results show [M+H]⁺: 512.2.

### Example 14: Synthesis of Compound D-D13

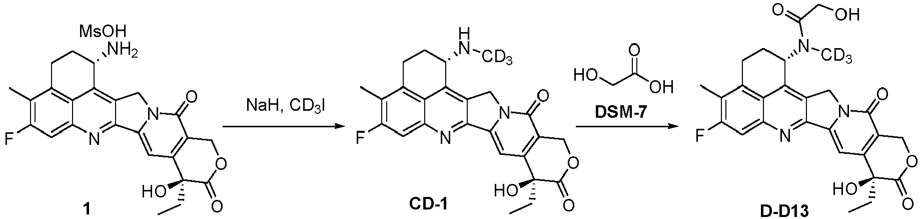

Weigh Compound 1 (319.1mg, 0.60mmol) into a 25mL round-bottomed flask, under argon protection, add 8mL of freshly distilled THF, and cool at -78°C. Dissolve 14.8mg of NaH in 2.0mL of freshly distilled THF, and slowly add dropwise to the low-temperature solution of Compound 1, stir at low temperature for 5 minutes, add CD₃I (50 uL, 0.80 mmol), after the addition, bring the reaction to room temperature and stir for 1 hour. HPLC monitors that the reaction no longer makes obvious progress, and the reaction is stopped. Add 5 mL of HCl (3M) solution, stirred overnight at room temperature, concentrated directly, and purify the liquid phase to obtain a yellow solid CD-1: 172.0mg, with 63% yield.
LC-MS results show [M+H]+: 453.2.

Add Compound CD-1 (67.5mg, 0.15mmol), DSM-7 (35.2mg, 0.45mmol), HATU (114.2mg, 0.30mmol), HOBt (41.0mg, 0.30mmol) and 5ml ultra- Dry DMF into a 25mL round-bottomed flask, seal the flask with liquid. Stir it in an ice-water bath for 10 minutes, then add DIEA (78uL, 0.45mmol), and stir at room temperature. HPLC detects the reaction progress, the reaction is about 6h, the raw material is less than 5%, the reaction system liquid phases are prepared and separated, and freeze-dried to obtain 35.7 mg of a yellow solid, with a yield of 47%.
LC-MS results show [M+H]+: 511.2.

### Example 15: Synthesis of Compound D-D14

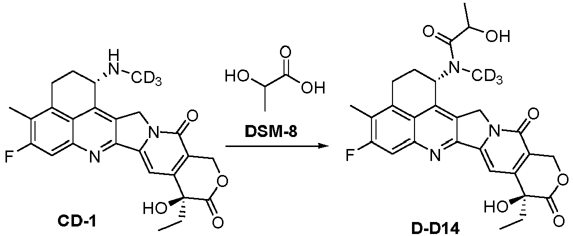

Add Compound CD-1 (67.9mg, 0.15mmol), DSM-8 (40.5mg, 0.45mmol), HATU (113.9mg, 0.30mmol), HOBt (41.4mg, 0.30mmol) and 5ml ultra- Dry DMF into a 25mL round-bottomed flask, seal the flask with liquid. Stir it in an ice-water bath for 10 minutes, then add DIEA (78uL, 0.45mmol), and bring it up to room temperature and stir. HPLC detects the reaction progress, reacts overnight, the raw material is less than 5%, the liquid phase of the reaction system The preparation was separated and lyophilized to obtain 38.9 mg of yellow solid, with a yield of 49%.
LC-MS results show [M+H]+: 525.2.

### Example 16: Synthesis of Compound D-D15 and D-D16

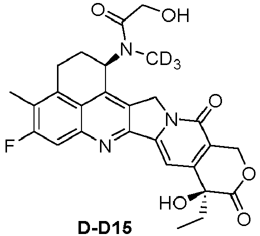

Referring to the synthetic route and method of Embodiment 14, Compound **D-D15** (33.5mg) is obtained. LC-MS results show [M+H]⁺: 511.2.

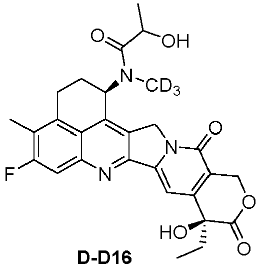

Referring to the synthetic route and method of Embodiment, Compound **D-D16** (37.5mg) is obtained.

LC-MS results show [M+H]+: 525.1.

### Example 17: Synthesis of Compound D-D17

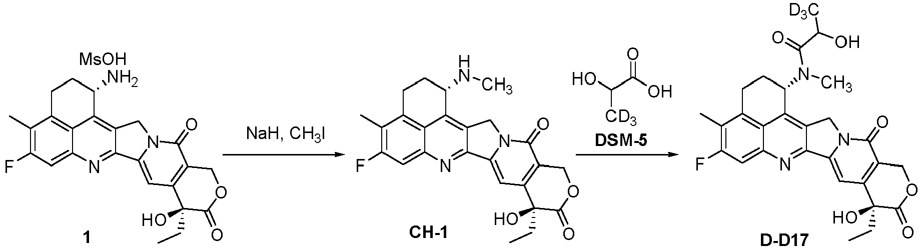

Weigh Compound 1 (320.5mg, 0.60mmol) into a 25mL round-bottomed flask, under argon protection, add 8mL of freshly distilled THF, and cool at -78°C. Dissolve 15.5mg of NaH in 2.0mL of freshly distilled THF, and slowly add dropwise to the low-temperature solution of Compound 1, stir at low temperature for 5 minutes, add CH₃I (50 usL, 0.80 mmol), after the addition is complete, bring the reaction to room temperature and stir for 1 hour. HPLC monitoring until the reaction no longer significantly progresses. The reaction is stopped. Add to the system 5mL HCl (3M) solution, stirred at room temperature overnight, concentrated directly, and prepare and purify the liquid phase to obtain yellow solid CH-1: 174.6mg, 65% yield .
LC-MS results show [M+H]+: 450.2.

Add Compound CH-1 (66.8mg, 0.15mmol), DSM-5 (42.2mg, 0.45mmol), HATU (114.2mg, 0.30mmol), HOBt (41.0mg, 0.30mmol) and 5ml ultra- Dry DMF into a 25mL round bottom flask, seal the flask with liquid. Stir it in an ice-water bath for 10 minutes, then add DIEA (78uL, 0.45mmol), and stir at room temperature. HPLC detects the reaction progress, the reaction is about 5h, the raw material is less than 5%, the reaction system liquid phases were prepared and separated, and freeze-dried to obtain 38.7 mg of a yellow solid, with a yield of 49%.
LC-MS results show [M+H]+: 525.2.

### Example 18: Synthesis of Compound D-D18, D-D19 and D-D20

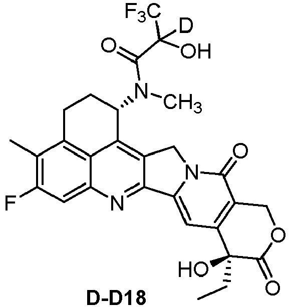

Referring to the synthetic route and method of Embodiment 17, Compound **D-D18** (39.5mg) is obtained, LC-MS results show [M+H]+: 576.1.

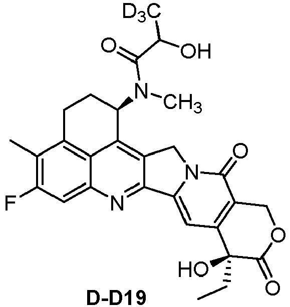

Referring to the synthetic route and method of Embodiment 17, Compound **D-D19** (37.5mg) is obtained, LC-MS results show [M+H]+: 525.1.

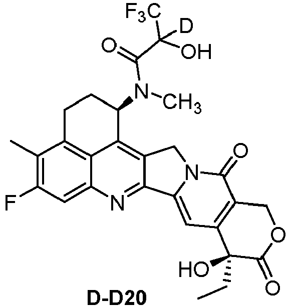

Referring to the synthetic route and method of Embodiment 17, Compound **D-D16** (38.9mg) is obtained, LC-MS results show [M+H]+:576.2.

### Example 19: Synthesis of Compound M1

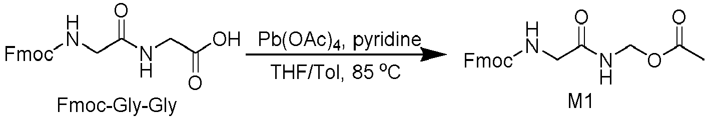

Add N-fluorenylmethoxycarbonyl-glycine-glycine (100 g, 282 mmol, 1.0 eq, outsourced), lead tetraacetate (175 g, 553 mmol, 1.4 eq), 2000 mL dry THF and 670 mL of toluene to a 5000 mL single mouth bottle, stirred evenly, under nitrogen protection, heated to 85 °C for 2.5 h. TLC monitoring, after the reaction of the raw materials, cool to room temperature, filter, the filtrate was concentrated under reduced pressure, and the residue was purified by column chromatography to obtain Compound M1 (87 g). LC-MS: [M+NH4]⁺386.0.

### Embodiment 20: Synthesis of Compound M2

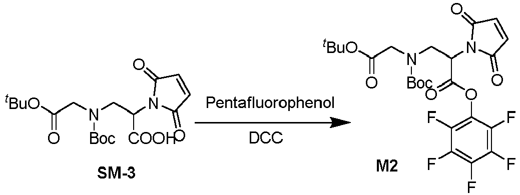

In a 1000 mL one-mouth bottle, add Compound SM-3 (49.9 g, 100.0 mmol, 1.0 eq, synthesized by referring to the method disclosed in patent application CN108452321), pentafluorophenol (18.5 g, 110.0 mmol, 1.1 eq), DCC (20.64 g, 110.0 mmol, 1.1 eq) and THF (500 mL), react at room temperature for 1 h (monitor the complete reaction by TLC), filter to remove insoluble matter, record this filtrate as filtrate A, store at 2-8°C for later use (Take the solvent directly, use according to 0.2M calculation). LC-MS: [M+H]⁺ 565.2.

### Example 21: Synthesis of Compound L-D1

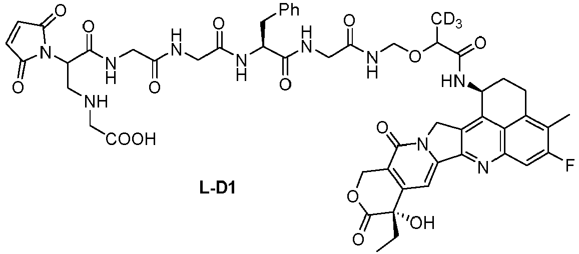

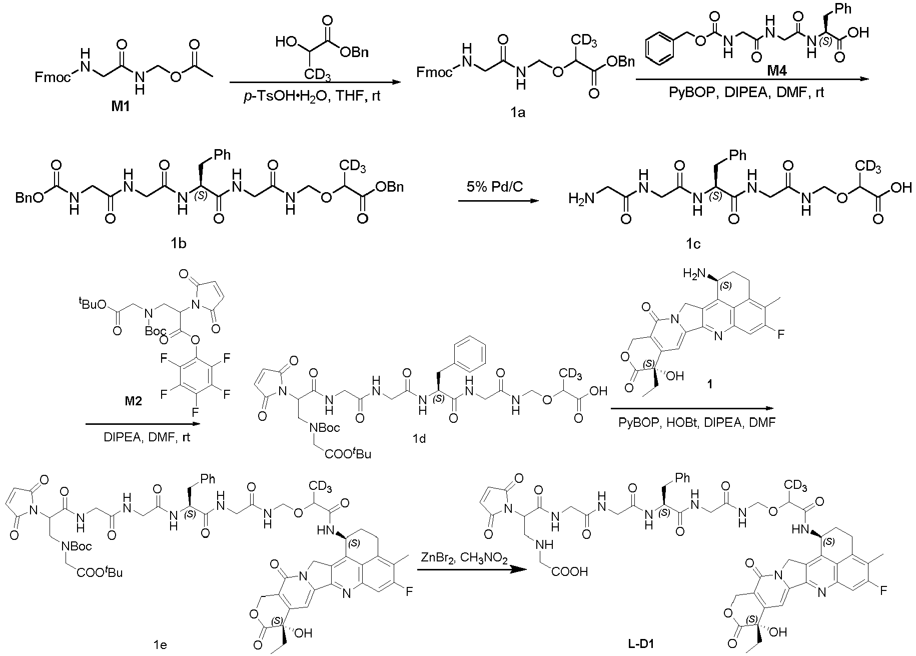

### Step 1: Synthesis of Compound 1a

In a 250 mL single-necked bottle, add M1 (7.37 g, 20.0 mmol), 100 mL THF, p-toluenesulfonic acid monohydrate (0.38 g, 2.00 mmol), stir and cool to 0 °C, add dropwise the synthesis intermediate of DSM-5 Benzyl ester (7.33 g, 40.0 mmol), after dropping, naturally warm to room temperature for reaction (reaction about 2-4 h), TLC monitoring. After the reaction is completed, add saturated NaHCOs solution to quench the reaction, extract with ethyl acetate, washed with saturated sodium chloride solution, dried over anhydrous sodium sulfate, filtered, concentrated, and the residue was purified by column chromatography (PE:EA=10:1-5:1-1:1) to obtain 1a (5.32g), yield 54%. LC-MS: [M+H]+492.2∘

### Step 2: Synthesis of Compound 1b

In a 50 mL single-necked bottle, add 1a (5.00 g, 10.2 mmol), 20 mL DMF, stir at 0 °C, add DBU (1.64 mL, 11.0 mmol), and react for 1 h. Monitored by TLC until the deprotection of Fmoc is finished, lay still and wait for use;

Add M4 (4.55 g, 11.0 mmol, purchased), PyBOP (6.25 g, 12.0 mmol), HOBt (1.62 g, 12.0 mmol) and 10 mL DMF to another 50 mL single-necked bottle, add DIEA(2.00 mL, 12.0 mmol) under ice bath, continue to stir for 30 min, add the above-mentioned standing reaction solution to the reaction bottle, and raise reaction temperature to room temperature. After the reaction is finished as monitored by HPLC, the reaction solution liquid phase is prepared and purified to obtain the product preparation solution, which is freeze-dried to obtained solid 1b (4.61 g), with yield of 68%. LC-MS:[M+H]⁺ 665.3.

### Step 3: Synthesis of Compound 1c

Add 1b (4.32g, 6.50 mmol) to a 50 mL single-necked bottle, dissolve in 15 mL of DMF, add 4.2 g of 5% Pd/C, and hydrogenate for 2 h. After the reaction is complete, filter to obtain the filtrate and obtain the crude product 1c to be used directly for the next reaction.

### Step 4: Synthesis of Compound 1d

Put the crude product **1c** in an ice-water bath, add DIPEA (1.22mL, 7.00 mmol), and then add Compound M2 (35mL, 7.0 mmol), raise to room temperature and react for 1 h. Use HPLC to monitor the reaction until completion, and purify the liquid phase to obtain the preparation solution, lyophilize the preparation solution to obtain **1d** (3.62g). LC-MS: [M+H]+ 821.4.

### Step 5: Synthesis of Compound 1e

Add **1d** (500.0 mg, 0.60 mmol), Compound 1 (310.6 mg, 0.58 mmol), PyBOP (448.3 mg, 0.94 mmol), HOBt (127.0 mg, 0.94 mmol) and 15 mL DMF into a 50 mL single-neck bottle, Add DIEA (418 uL, 2.40 mmol) in an ice-water bath, raise temperature to room temperature and react for 3 h. Monitor the reaction by HPLC until finishing, purify the reaction solution by liquid phase preparation to obtain the preparation solution of Compound **1e,** which is lyophilized to obtain **1e** (497.9mg , 67%). LC-MS:[M+H]⁺ 1238.6.

### Step 6: Synthesis of Compound L-D1

Add **1e** (100 mg, 0.081 mmol), zinc bromide (368 mg, 1.63 mmol) and 5 mL nitromethane into a 25 mL single-necked bottle, and react at 40 °C for 1 h. After the reaction was finished as monitored by HPLC, concentrate under reduced pressure to remove solvent to get the crude product. The crude product liquid phase is prepared and purified to obtain the product preparation liquid, which is freeze-dried to obtain the solid Compound **L-D1**(63.1 mg). LC-MS:[M+H]⁺ 1082.3.

### Example 22: Synthesis of Compound L-D2

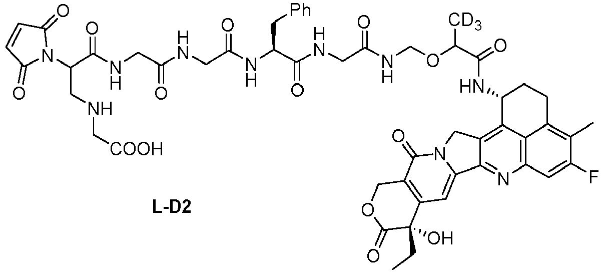

Refer to the synthetic route and method of Embodiment 21 to obtain Compound **L-D2** (59.8mg). LC-MS: [M+H]⁺ 1082.4.

### EXample 23: Synthesis of Compound L-D3

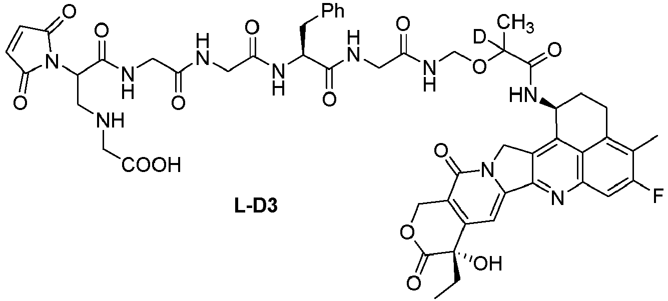

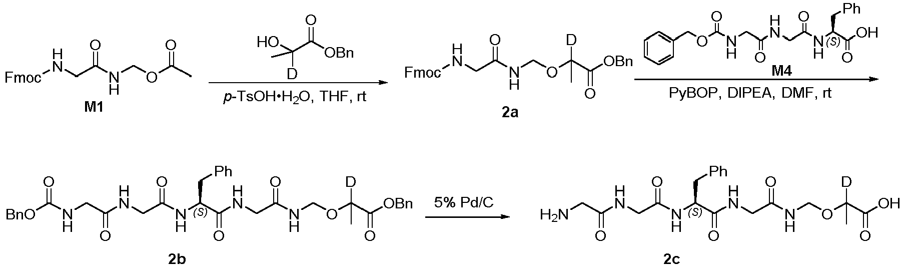

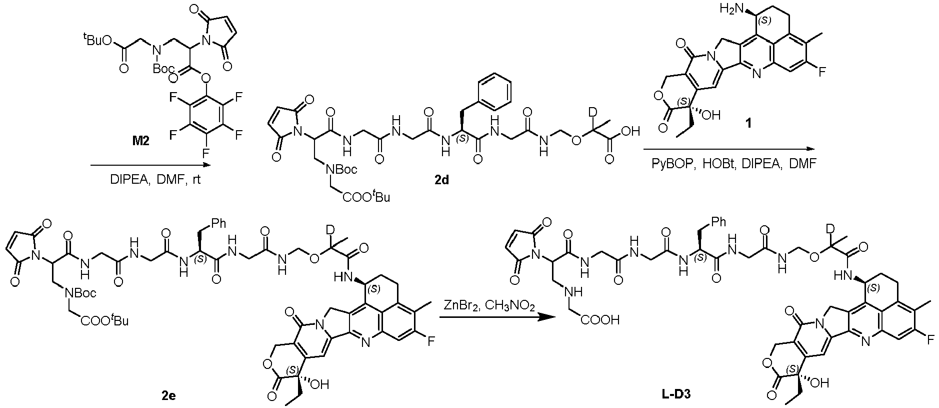

### Step 1: Synthesis of Compound 2a

In a 250 mL single-necked bottle, add **M1** (7.36 g, 20.0 mmol), 100 mL THF, p-toluenesulfonic acid monohydrate (0.38 g, 2.00 mmol), stir and cool to 0 °C, add dropwise the synthesis intermediate of DSM-2 Benzyl ester (7.25g, 40.0 mmol), after dripping, naturally warmed to room temperature and react (reaction about 2-4 h), monitored by TLC. After the reaction was completed, saturated NaHCOs solution was added to quench the reaction, extracted with ethyl acetate, washed with saturated sodium chloride solution, dried over anhydrous sodium sulfate, filtered, concentrated, and the residue was purified by column chromatography (PE:EA=10:1-5:1-1:1) to obtain **2a** (5.12g), yield 52%. LC-MS: [M+H]⁺ 490.2.

### Step 2: Synthesis of Compound 2b

In a 50 mL single-necked bottle, add **2a** (5.00 g, 10.2 mmol), 20 mL DMF, stir at 0 °C, add DBU (1.64 mL, 11.0 mmol), and react for 1 h. Monitor by TLC until the deprotection of Fmoc is completed. Lay still and wait for use;
Add **M4** (4.56 g, 11.0 mmol, purchased), PyBOP (6.26 g, 12.0 mmol), HOBt (1.64 g, 12.0 mmol) and 10 mL DMF to another 50 mL single-necked bottle, add DIEA(2.00 mL, 12.0 mmol) in an ice-water bath, continue to stir for 30 min, add the above-mentioned reaction solution to the reaction bottle, and raise to room temperature for reaction. After the HPLC monitoring the end of the reaction, the reaction solution is purified by the preparative liquid phase to obtain the product preparation solution, extract with dichloromethane, wash with saturated sodium chloride solution, dry over anhydrous sodium sulfate, filter, and concentrate under reduced pressure to obtain solid **2b** (4.58 g), with yield of 68%. LC-MS: [M+H]⁺ is 663.3.

### Step 3: Synthesis of Compound 2c

Add **2b** (4.31g, 6.50 mmol) into a 50 mL single-necked bottle, dissolve into 15 mL of DMF, add 4.0 g of 5% Pd/C, hydrogenate for 2 h. After the reaction is completed, filter to obtain the filtrate, and directly obtain the crude product 2c for the next reaction.

### Step 4: Synthesis of Compound 2d

Put the crude product **2c** in an ice-water bath, add DIPEA (1.22mL, 7.00 mmol), and then add Compound **M2** (35mL, 7.0 mmol), raise reaction temperature to room temperature and react for 1 h. HPLC monitor until the completion of the reaction, and perform liquid phase purification to obtain the preparation solution, freeze-dried to obtain **2d** (3.57g). LC-MS: [M+H]+ 819.4.

### Step 5: Synthesis of Compound 2e

Add **2d** (500.0 mg, 0.61 mmol), Compound 1 (320.1 mg, 0.60 mmol), PyBOP (448.5 mg, 0.94 mmol), HOBt (127.3 mg, 0.94 mmol) and 15 mL DMF into a 50 mL single-necked bottle, add DIEA (418 uL, 2.40 mmol) in ice-water bath, raise to room temperature and the reaction was carried out at room temperature for 3 h. After the reaction was completed as monitored by HPLC, the reaction solution was prepared and purified by liquid phase to obtain the preparation solution of Compound **2e**, which was freeze-dried to obtain **2e** (482.3 mg, 64 %). LC-MS: [M+H]⁺ 1236.5.

### Step 6: Synthesis of Compound L-D3

Add **2e** (100 mg, 0.081 mmol), zinc bromide (370 mg, 1.63 mmol) and 5 mL nitromethane into a 25 mL single-necked bottle, and react at 40 °C for 1 h. After the reaction was completed as monitored by HPLC, concentrate under reduced pressure to remove solvent to obtain crude product. The crude product was prepared and purified by liquid phase to obtain product preparation solution, which was freeze-dried to obtain solid Compound **L-D3** (48.3 mg). LC-MS:[M+H]⁺ 1080.4.

### Example 24: Synthesis of Compound L-D4

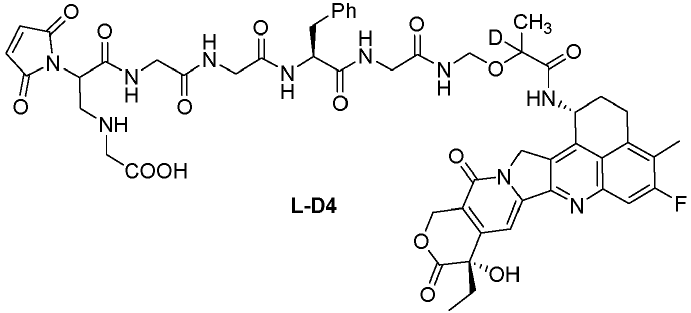

With reference to the synthetic route and method of Embodiment 23, obtain Compound **L-D4** (49.8mg). LC-MS:[M+H]⁺ 1080.4.

### Example 25: Synthesis of Compound L-D5

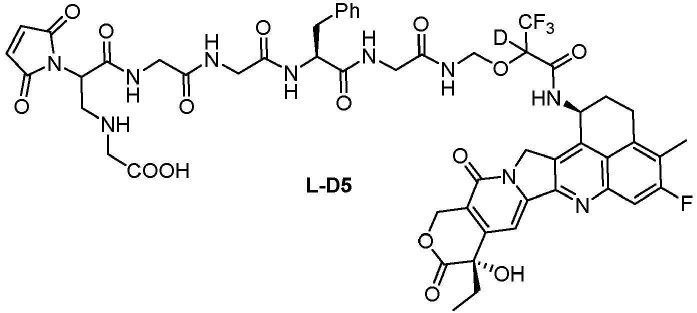

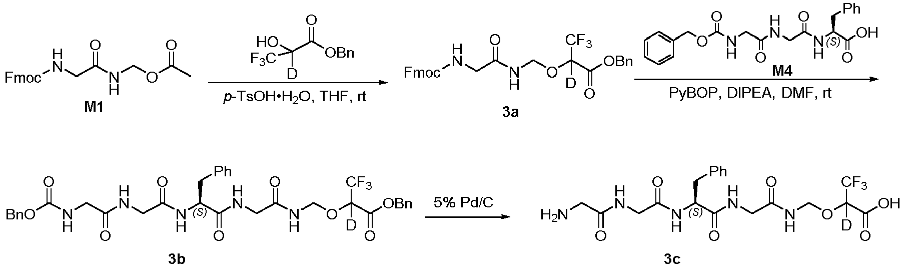

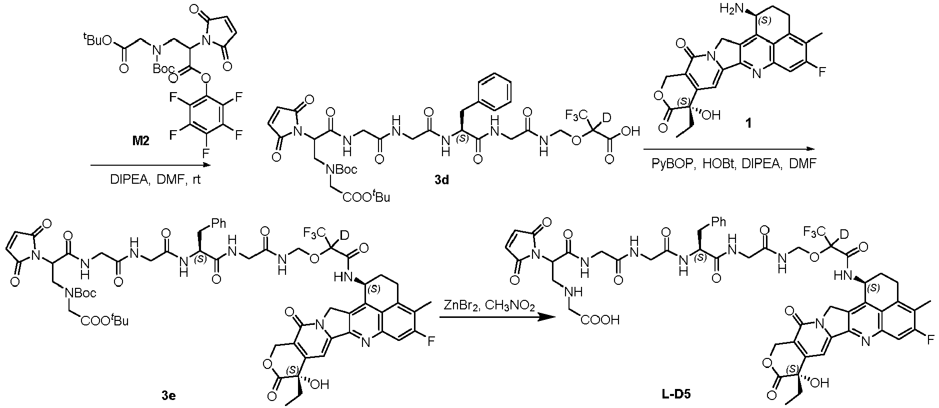

### Step 1: Synthesis of Compound 3a

In a 250 mL single-necked bottle, add **M1** (11.05 g, 30.0 mmol), 150 mL THF, p-toluenesulfonic acid monohydrate (0.57 g, 3.00 mmol), stir and cool to 0 °C, add dropwise the synthesis intermediate of **DSM-3** Benzyl ester (9.41g, 40.0 mmol), after dripping, naturally warm to room temperature for reaction (reaction about 2-4 h), TLC monitoring. After the reaction is completed, add saturated NaHCOs solution to quench the reaction, extract with ethyl acetate, wash with saturated sodium chloride solution, dry over anhydrous sodium sulfate, filter, concentrate, and the residue was purified by column chromatography (PE:EA=10:1-5:1-1:1) to obtain **3a** (8.04g), yield 37% . LC-MS:[M+H]⁺ 544.2.

### Step 2: Synthesis of Compound 3b

In a 50 mL single-necked bottle, add **3a** (5.54 g, 10.2 mmol), 20 mL DMF, stir at 0 °C, add DBU (1.64 mL, 11.0 mmol), and react for 1 h. After the completion of Fmoc deprotection as monitored by TLC, lay still and wait for use;
Add **M4** (4.55 g, 11.0 mmol, purchased), PyBOP (6.27 g, 12.0 mmol), HOBt (1.66 g, 12.0 mmol) and 10 mL DMF into another 50 mL single-necked bottle, add DIEA (2.00 mL , 12.0 mmol) in an ice-water bath, continue to stir for 30 min, add the above-mentioned standing reaction solution to the reaction bottle, and raise to room temperature for reaction. After the reaction is completed with HPLC monitoring, the reaction solution is purified by the preparative liquid phase to obtain the product preparation solution, which is extracted with dichloromethane, washed with saturated sodium chloride solution, dried over anhydrous sodium sulfate, filtered, and concentrated under reduced pressure to obtain solid 3b (3.52 g), yield 48%. LC-MS: [M+H]⁺ 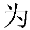 717.3.

### Step 3: Synthesis of Compound 3c

Add 3b (3.22g, 4.50 mmol) into a 50 mL single-necked bottle, dissolve it in 10 mL DMF, add 3.0 g 5% Pd/C, hydrogenate for 2 h, after the reaction is completed, filter to obtain the filtrate, and directly obtain the crude product **3c** for the next reaction.

### Step 4: Synthesis of Compound 3d

The crude product 3c is placed in an ice-water bath, DIPEA(1.22mL, 7.00 mmol) is added, and Compound **M2** (25mL, 5.0 mmol) was added, and the reaction was completed at room temperature for 1 h. HPLC monitor the completion of the reaction, and liquid phase was purified to obtain the preparation solution, freeze-dried to obtain **3d**(3.17g). LC-MS:[M+H]⁺ 873.3.

### Step 5: Synthesis of Compound 3e

Add **3d** (500.2 mg, 0.57 mmol), **Compound 1** (319.5 mg, 0.60 mmol), PyBOP (447.3 mg, 0.94 mmol), HOBt (126.9 mg, 0.94 mmol) and 15 mL DMF into a 50 mL single-necked bottle, Add DIEA (418 uL, 2.40 mmol) under ice-water bath, and the reaction temperature was raised to room temperature for 3 h. After the reaction was completed as monitored by HPLC, the reaction solution was purified by liquid phase preparation to obtain the preparation solution of Compound **3e**, which was freeze-dried to obtain **3e** (492.3 mg, 67 %). LC-MS:[M+H]⁺ 1290.5.

### Step 6: Synthesis of Compound L-D5

Add **3e** (100 mg, 0.077 mmol), zinc bromide (370 mg, 1.63 mmol) and 5 mL nitromethane into a 25 mL single-necked bottle, and react at 40 °C for 1 h. After the reaction is completed as monitored by HPLC, concentrate under reduced pressure to remove solvent to obtain crude product. The crude product was prepared and purified by liquid phase to obtain the product preparation solution, which was lyophilized to obtain a solid Compound **L-D5** (42.4mg, 48%). LC-MS: [M+H]⁺ 1134.4.

### Example 26: Synthesis of Compound L-D6

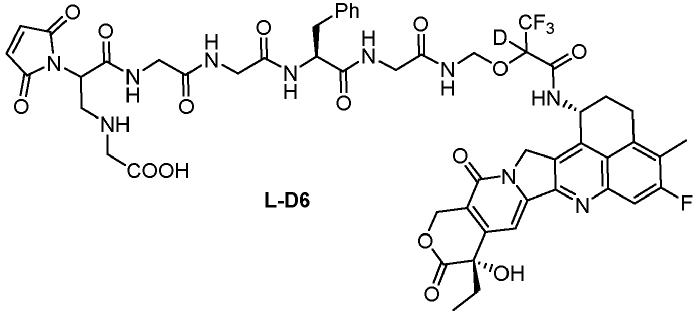

Referring to the synthetic route and method of Embodiment 25, obtain Compound **L-D6** (41.8mg). LC-MS:[M+H]⁺ 1134.4.

### Example 27: Synthesis of Compound L-D7

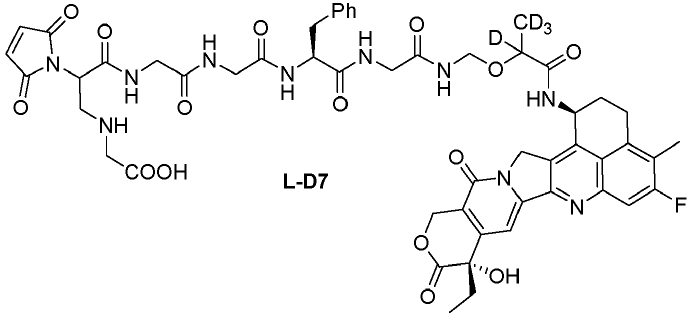

Referring to the synthetic route and method of Embodiment 23, obtain Compound **L-D7**(45.7mg). LC-MS:[M+H]⁺ 1083.4.

### Example 28: Synthesis of Compound L-D8

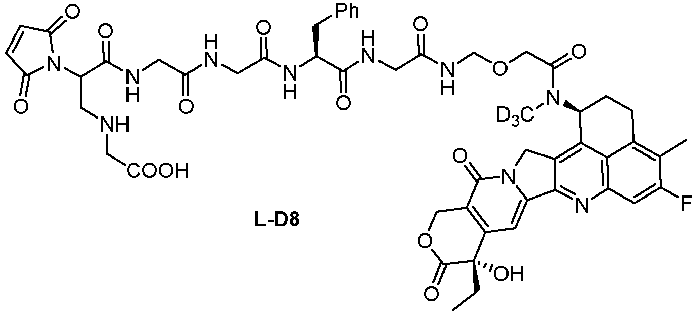

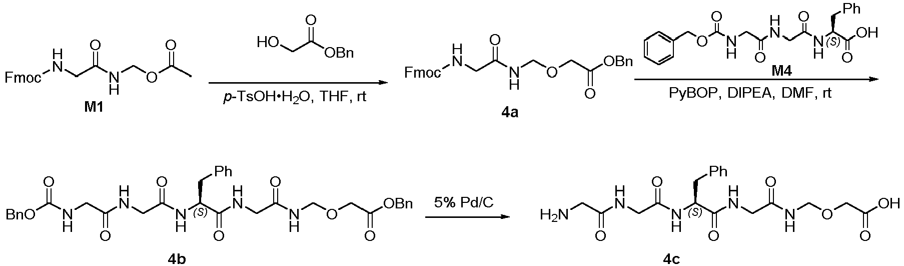

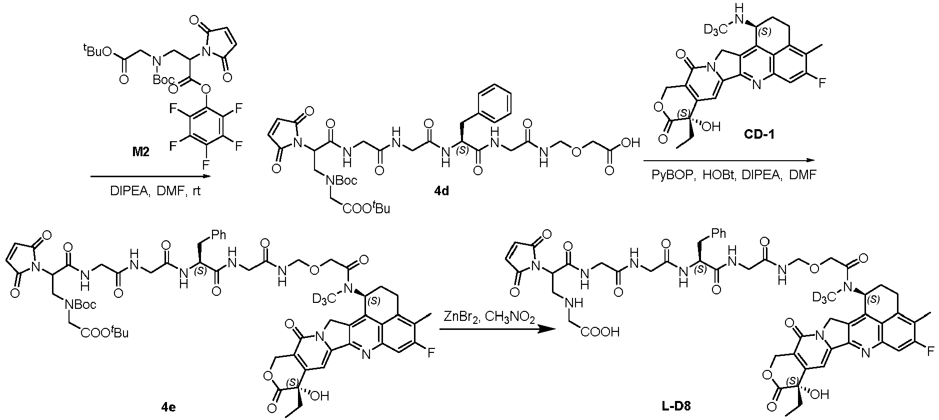

### Step 1: Synthesis of Compound 4a

In a 250 mL single-necked bottle, add **M1** (7.36 g, 20.0 mmol), 100 mL THF, p-toluenesulfonic acid monohydrate (0.38 g, 2.00 mmol), stir and cool to 0 °C, dropwise add benzyl glycolate (6.65 g, 40.0 mmol), after dripping, naturally warm to room temperature for reaction (reaction about 2-4 h), TLC monitoring. After the reaction is completed, add saturated NaHCOs solution to quench the reaction, extract with ethyl acetate, wash with saturated sodium chloride solution, dry over anhydrous sodium sulfate, filter, concentrate, and the residue is purified by column chromatography (PE:EA=10:1-5:1-1:1) to obtain **4a** (5.29g), with a yield of 56%. LC-MS:[M+H]⁺ 475.2.

### Step 2: Synthesis of Compound 4b

Add **4a** (5.00 g, 10.5 mmol), 20 mL DMF, stir at 0 °C, add DBU (1.64 mL, 11.0 mmol), and react for 1 h. After TLC monitoring the completion of Fmoc deprotection, let stand and wait for use. To a 50 mL single-mouth vial, add M4 (4.57 g, 11.0 mmol, purchased), PyBOP (6.26 g, 12.0 mmol), HOBt (1.65 g, 12.0 mmol) and 10 mL DMF, and DIEA (2.00 mL, 12.0 mmol) under ice water bath. Continue stirring for 30 min, add the above static reaction solution to the reaction flask and raise the reaction to room temperature. After monitoring the reaction by HPLC, purify the reaction solution by the preparative liquid phase to obtain the product preparation solution, and then extract with dichloromethane, wash with saturated sodium chloride solution, dry with anhydrous sodium sulfate, filter and concentrate under reduced pressure to obtain solid 4b (4.68 g) in 71% yield.

In a 50 mL single-necked bottle, add 4a (5.00 g, 10.5 mmol), 20 mL DMF, stir at 0 °C, add DBU (1.64 mL, 11.0 mmol), and react for 1 h. Deprotection of Fmoc is completed as monitored by TLC, lay still and wait for use;
Add M4 (4.57 g, 11.0 mmol, purchased), PyBOP (6.26 g, 12.0 mmol), HOBt (1.65 g, 12.0 mmol) and 10 mL DMF into another 50 mL single-necked bottle, add DIEA (2.00 mL, 12.0 mmol) under an ice-water bath, continue to stir for 30 min, add the above-mentioned standing reaction solution to the reaction bottle, raise to room temperature for reaction. After reaction is completed with HPLC monitoring, the reaction solution is purified by the preparative liquid phase to obtain the product preparation solution, The prepared solution is extracted with dichloromethane, washed with saturated sodium chloride solution, dried over anhydrous sodium sulfate, filtered, and concentrated under reduced pressure to obtain solid **4b** (4.68 g), yield 71%. LC-MS:[M+H]⁺ 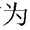 648.3.

### Step 3: Synthesis of Compound 4c

Add **4b** (3.00g, 4.63 mmol) into a 50 mL single-necked bottle, dissolve it in 15 mL DMF, add 2.0 g 5% Pd/C, hydrogenate for 2 h, after the reaction is completed, filter to obtain the filtrate, and directly obtain the crude product **4c** for the next reaction.

### Step 4: Synthesis of Compound 4d

The crude product **4c** was placed in an ice-water bath, DIPEA (1.22mL, 7.00 mmol) is added, and Compound **M2** (25mL, 5.0 mmol) is added, and the reaction is completed at room temperature for 1 h. The completion of the reaction was monitored by HPLC, and liquid phase was purified to obtain preparation solution. The preparation solution is lyophilized to obtain **4d** (2.57g). LC-MS:[M+H]⁺ 804.3.

### Step 5: Synthesis of Compound 4e

Add **4d** (500.0 mg, 0.62 mmol), Compound **CD-1** (289.1 mg, 0.64 mmol), PyBOP (448.5 mg, 0.94 mmol), HOBt (127.3 mg, 0.94 mmol) and 15 mL DMF into a 50 mL a single-neck bottle, add DIEA (418 uL, 2.40 mmol) in an ice-water bath, raise to room temperature and react for 3 h. After the reaction is completed as monitored by HPLC, the reaction solution is prepared and purified by liquid phase to obtain the preparation solution of Compound **4e**, which is lyophilized to obtain **4e**(412.3mg, 48%). LC-MS:[M+H]⁺ 1238.5.

### Step 6: Synthesis of Compound L-D8

Add **4e** (100 mg, 0.081 mmol), zinc bromide (370 mg, 1.63 mmol) and 5 mL nitromethane into a 25 mL single-necked bottle, and react at 40 °C for 1 h. After the reaction was completed as monitored by HPLC, concentrate under reduced pressure to remove solvent to obtain crude product. The crude product was prepared and purified by liquid phase to obtain product preparation solution, which was freeze-dried to obtain solid Compound **L-D8** (48.7mg). LC-MS:[M+H]⁺ 1082.4.

### Example 29: Synthesis of Compound L-D9

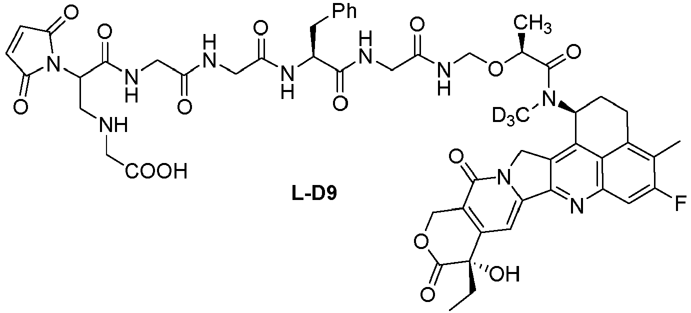

With reference to the synthetic route and method of Embodiment 21, 28, obtain Compound **L-D9**(45.1mg). LC-MS:[M+H]⁺ 1096.4.

### Example 30: Synthesis of Compound L-D10

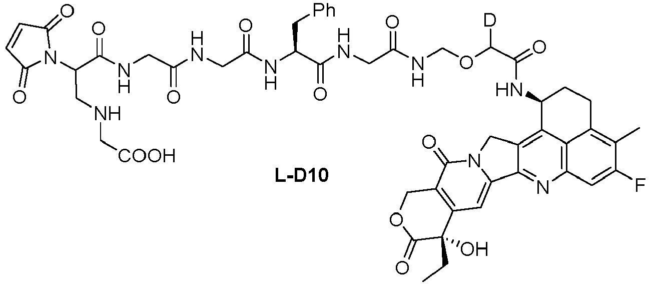

With reference to the synthetic route and method of Embodiment 21, obtain Compound **L-D10** (45.9mg). LC-MS:[M+H]⁺ 1066.4.

### Example 31: Synthesis of Compound L-D11

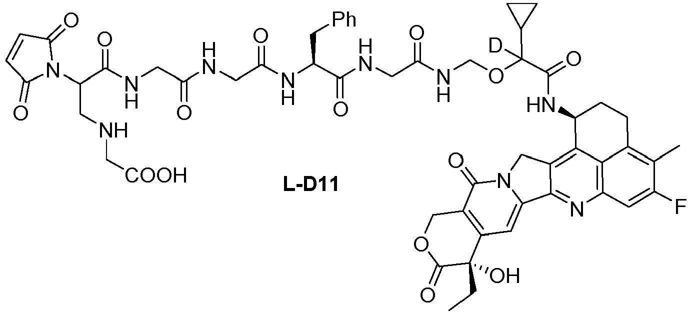

With reference to the synthetic route and method of Embodiment 23, obtain Compound **L-D11**(55.2mg). LC-MS:[M+H]⁺ 1106.4.

### Example 32: Synthesis of Compounds used as controls

With reference to the methods disclosed in CN111689980 and WO2020063676, the following compounds were obtained:

| **Compound Synthesis serial number** | **Structure of the Compound** |
|---|---|
| **2** | 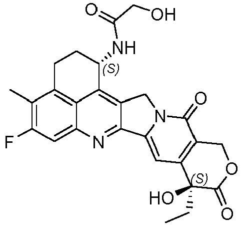 |
| **3** | 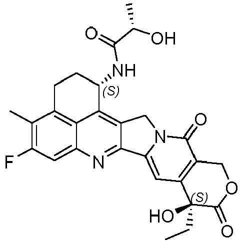 |
| **4** | 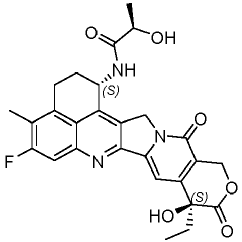 |
| **5** | 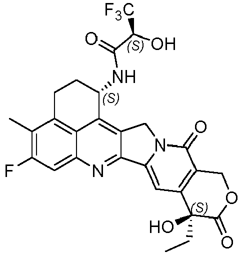 |
| **6** | 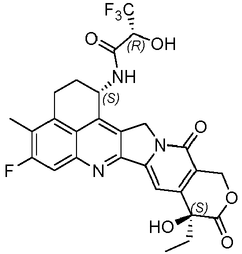 |
| **7** | 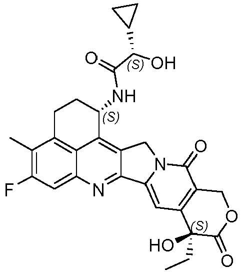 |
| **8** | 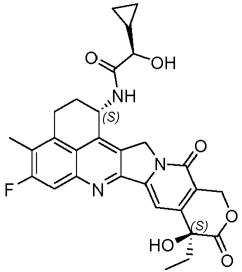 |

### Example 33: Synthesis of Compound L-H1

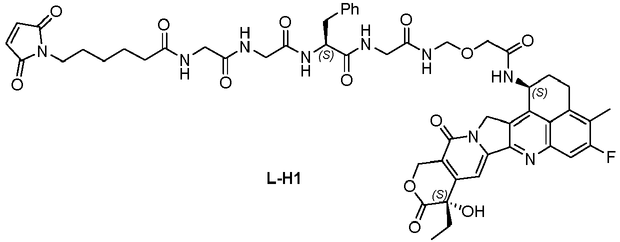

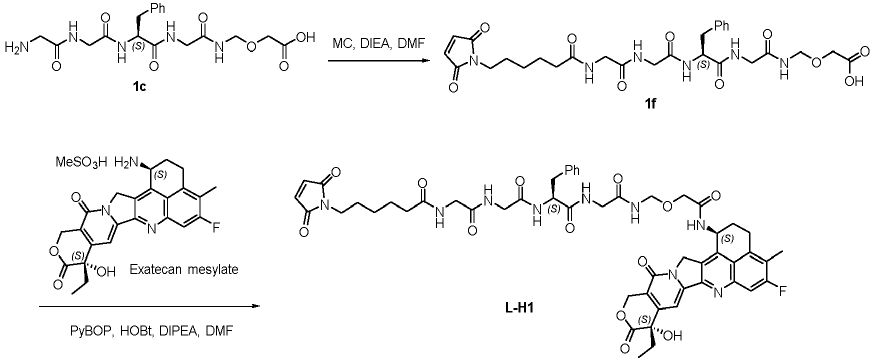

### Step 1: Synthesis of Compound 1f

Add **1b** (500.0mg, 0.772mmol), 5% Pd/C (500.0mg, 100% m) and 10ml DMF into a 50 mL one-mouth bottle, add a hydrogen balloon and replace the hydrogen, and react at room temperature for about 3 hours. HPLC monitors that the reaction is complete. ultrasonically remove hydrogen. Filtrate to obtain the filtrate, and obtain the crude product **1c**. Under ice-water bath, add MC (280.1mg, 0.9mmol) and DIEA (235mg, 1.8mmol) to the filtrate successively, under nitrogen protection, raise temperature to react at room temperature for 1 hour, monitored by HPLC, purified by liquid phase preparation, and lyophilized to obtain Compound **1f** (268.9mg, 86%), MS:[M+H]⁺617 .2.

### Step 2: Synthesis of Compound L-H1

Add **1f** (268.9mg, 0.44mmol), Eatecan mesylate (233.8mg, 0.44mmol, purchased), HATU (190.5mg, 0.50mmol), HOBt (67.8mg, 0.50mmol) into a 10ml one-mouth bottle and 5ml of DMF, placed in an ice-water bath and stirred for 10min, added dropwise DIEA (140uL, 0.84mmol) to continue the reaction, HPLC monitored the reaction to completion and the preparation was separated, and the preparation solution was freeze-dried to obtain 254.3mg of a yellow solid, with a yield of 56%. LC-MS:[M+H]⁺ 1034.4.

### Example 34: Synthesis of the Following Compounds According to the Synthesis Route and Method of Example 21:

| **Serial Number of Compounds (payload)** | **Payload Structure** |
|---|---|
| **L-H2** | 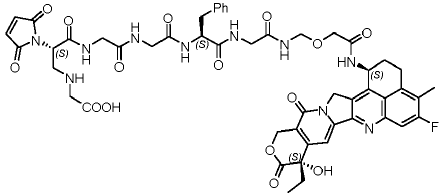 |
| **L-H3** | 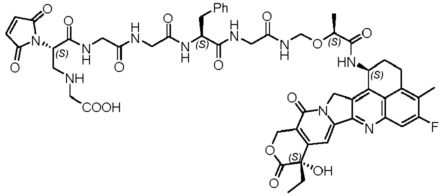 |
| **L-H4** | 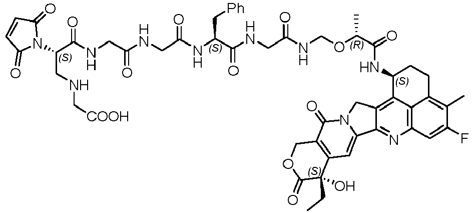 |
| **L-H5** | 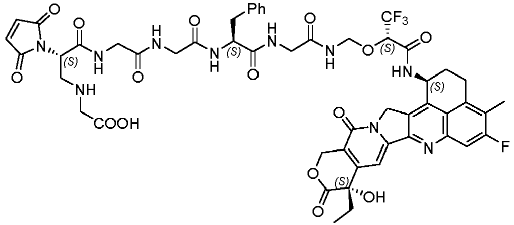 |
| **L-H6** | 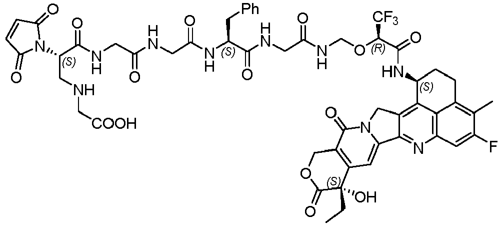 |
| **L-H7** | 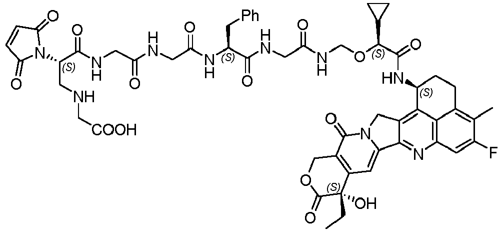 |
| **L-H8** | 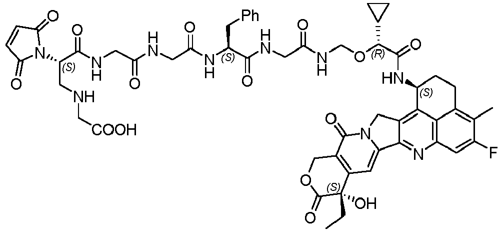 |

### Example 35: General method for preparation of ADC drug by conjugation.

After the initial purification of the antibody molecule Ab so that the monomer rate is greater than 95%, The Ab undergoes a buffer replacement by an ultrafiltration centrifugation to arrive at a phosphate buffer at a concentration of 10 mg/ml. Add TCEP at a ratio of 20 times the number of molar molecules of the Ab, and react at room temperature for 4h to open the disulfide bond between the antibody chains. Add payload at a ratio of 20 times the number of molar molecules of the Ab, and react at room temperature for 2h. Use an ultrafiltration centrifuge tube with a molecular weight cut-off of 30KDa to replace into PBS buffer and remove uncoupled payload. The ADC sample after buffer replacement is filtered through a 0.22 micron sterile filter for later use.

Using this conjugation method (Embodiment 35), conjugate the following Payload Compound, e.g., **L-D1**, **L-D2**, **L-D3**, **L-D4**, **L-D5**, **L-D6**, **L-D7**, **L-D8**, **L-D9**, **L-D10**, **L-D11**, **L-H1, L-H2, L-H3, L-H4, L-H5, L-H6, L-H7** and **L-H8** with antibodies, e.g., A, Trastuzumab and B, Cetuximab. The conjugation products are measured for their average drug/antibody ratio (DAR) value using reversed-phase high performance liquid chromatography. The conjugated ADC drugs and the payload compounds are listed below:

| **Payload Serial #** | **Conjugates ADC#** | **Ab** | **ADC Structure** | **DAR** |
|---|---|---|---|---|
| **L-D1** | **ADC-1** | A | 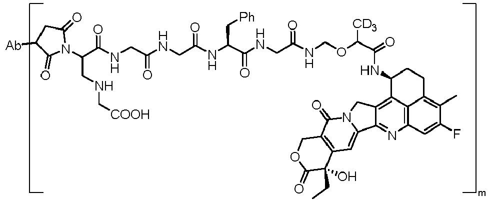 | 7.7 |
| **L-D2** | **ADC-2** | A | 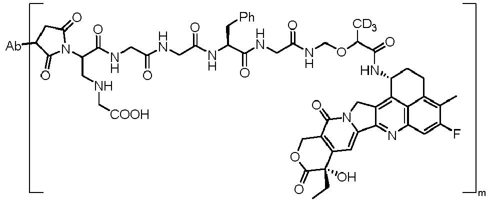 | 7.7 |
| **L-D3** | **ADC-3** | A | 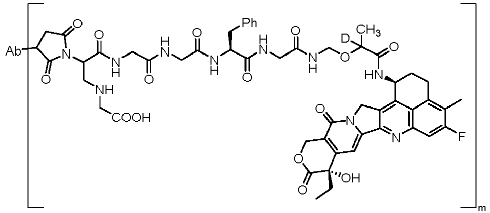 | 7.6 |
| **L-D4** | **ADC-4** | A | 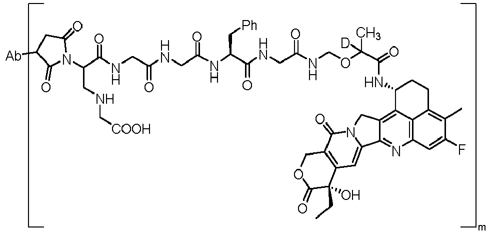 | 7.7 |
| L-D5 | ADC-5 | A | 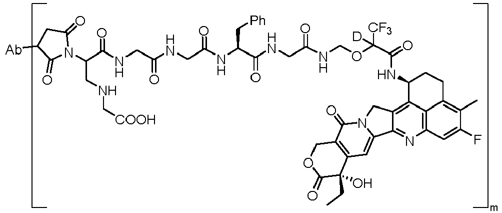 | 7.4 |
| L-D6 | ADC-6 | A | 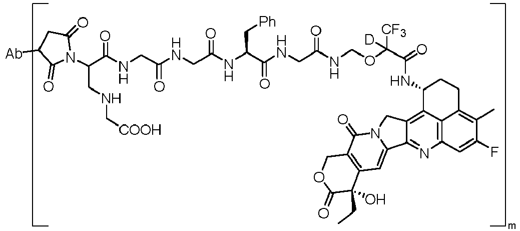 | 7.3 |
| L-D7 | ADC-7 | A | 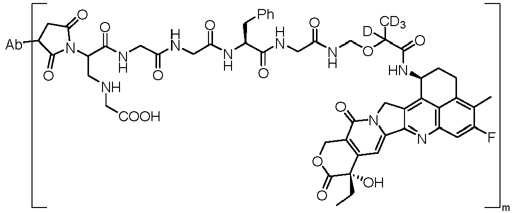 | 7.6 |
| L-D8 | ADC-8 | A | 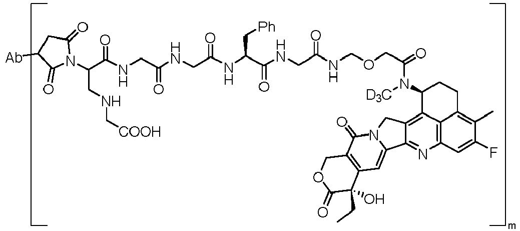 | 7.5 |
| L-D9 | ADC-9 | A | 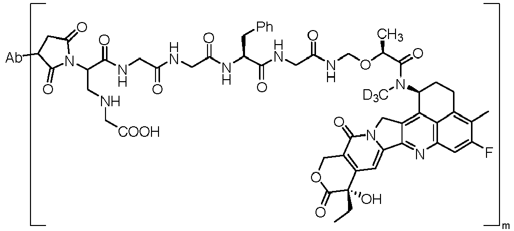 | 7.3 |
| L-D10 | ADC-10 | A | 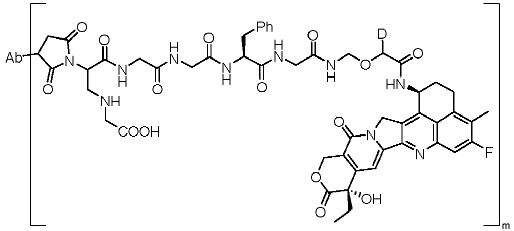 | 7.8 |
| **L-H1** | **ADC-11** | A | 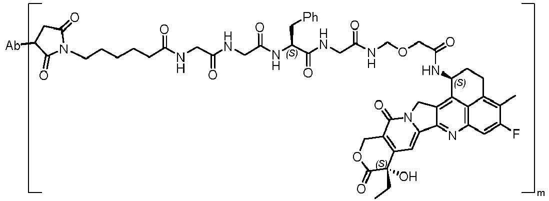 | 7.6 |
| **L-H2** | **ADC-12** | A | 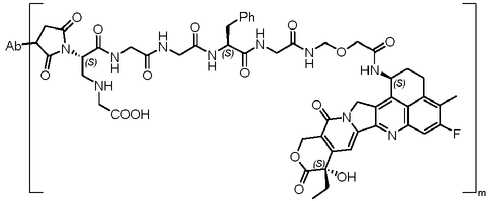 | 7.4 |
| **L-H3** | **ADC-13** | A | 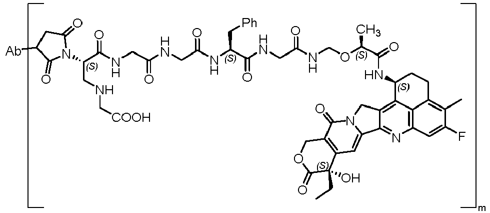 | 7.3 |
| **L-H4** | **ADC-14** | A | 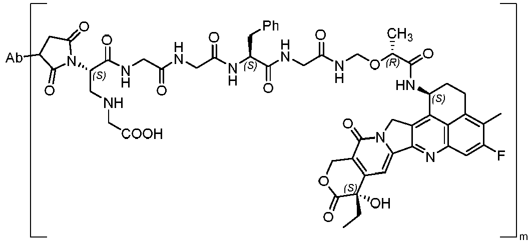 | 7.3 |
| **L-H5** | **ADC-15** | A | 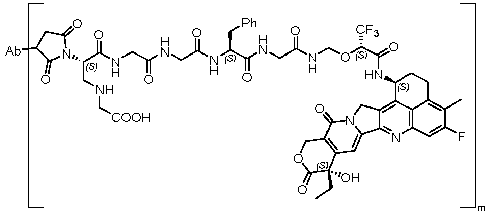 | 7.5 |
| **L-H6** | **ADC-16** | A | 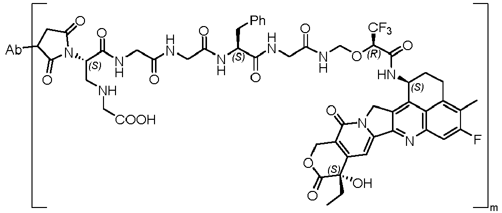 | 7.4 |
| **L-H7** | **ADC-17** | A | 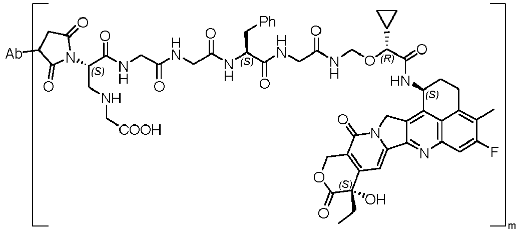 | 7.5 |
| **L-H8** | **ADC-18** | A | 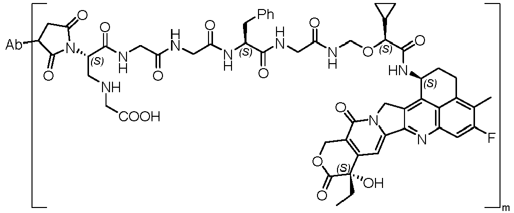 | 7.4 |
| **L-D3** | **ADC-19** | B | 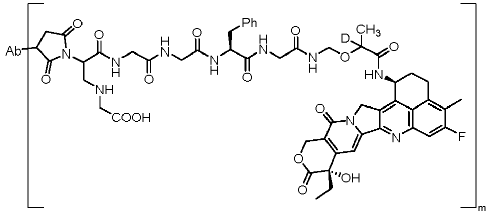 | 7.3 |
| **L-D11** | **ADC-20** | B | 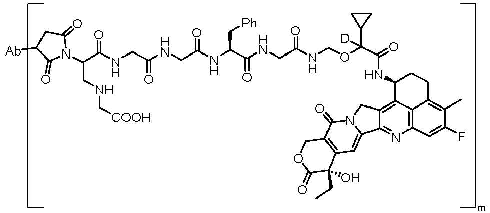 | 7.2 |

### Embodiment 36: Clearance half-life test of deuterated camptothecin drugs

### Material

### liver microsomes:

### Human liver microsomes (supplied by Xenotech))

### Chemical reagents:

| **Name** | **Supplier** | **Batch #** |
|---|---|---|
| NADPH | Sigma | SLCC8498 |
| KH₂PO₄ | Sinopharm Chemical Reagent Co., Ltd. | 20180816 |
| K₂HPO₄• 3H₂O | Sinopharm Chemical Reagent Co., Ltd. | P1359845 |
| MgCl₂·6H₂O | Sinopharm Chemical Reagent Co., Ltd. | 20180813 |
| formic acid (FA) | Aladdin | L1903097 |
| DMSO | Sigma | BCBW5664 |
| Acetonitrile (ACN) | Merck | I0928430749 |
| Methanol (MeOH) | Merck | I1074607006 |

### Equipment

| | |
|---|---|
| Liquid phase system | Waters ACQUITY UPLC I-Class |
| MassSpec: | Waters Xevo G2-XS QTOF |
| Water bath: | BSG-24 |
| Centrifuge: | Thermo Fisher ST40R |
| Waterpurification: | Milli-Q IQ7000 |

### INCUBATION SYSTEM

| | |
|---|---|
| Liver microsomal protein concentration | 1.0 mg/mL |
| Species | Human |
| Test article and positive control concentration | 10 µM |
| NADPHConcentration | 1.0 mM |
| Magnesium chloride | 3.0 mM |
| Incubation Medium | 100 mM Potassium phosphate buffer (pH = 7.4) |
| Incubation conditions | 37 °C |
| Incubation volume | 200 µl |

### EXPERIMENTAL FLOW AND PROCEEDURE

### Preparation of Phosphate Buffer

Preparation of phosphate buffer: Weigh appropriate amount of KH₂PO₄ (MW= 136.09), K₂HPO₄ • 3H₂O (MW=228.22) and MgCl₂ • 6H₂O (MW=203.3) and fully dissolve in appropriate volume of deionized water to prepare 100 mM pH = 7.4 ± 0.05 potassium phosphate buffer containing 3 mM MgCl₂, if necessary, use HCl to adjust the pH.

### Stock solution and working solution preparation

Preparation of stock solution: Weigh an appropriate amount of the test product or positive control Compound into an appropriate container, and prepare a 10mM stock solution with DMSO (or other suitable organic solvents).

Preparation of working solution: dilute 10mM stock solution with acetonitrile (or other suitable solvent) to 1.0 mM as working solution, set aside.

### Preparation of Microsomal Test/Positive Control Working Solution

Liver microsome stock solution (20 mg/mL) was diluted with 100 mM potassium phosphate buffer (pH 7.4) to the concentration required for incubation of samples (1.12 mg/mL).

### Preparation of NADPH solution

The NADPH coenzyme solution contains 10 mM NADPH, which is obtained by dissolving and diluting NADPH in 100 mM potassium phosphate buffer solution, and is ready for use before incubation.

### Preparation of stop solution

Methanol and acetonitrile were mixed in equal volumes (MeOH:ACN=1:1, v:v) as a stop solution and stored in a 4°C refrigerator for later use.

### Sample Incubation and Processing

1. Add the working solution of the test substance to the liver microsome working solution and pre-incubate at 37 °C for 5 minutes, then add the prepared NADPH working solution and mix well. Make the reaction volume of the incubation system reach 200 µL, and the liver microsomes concentration in the incubation system is 1.0 mg/mL , the test substance concentration is 10 µM. The incubation tube is incubated in a water bath at 37 °C for 120 minutes. In the incubation system of the test substance, total organic solvents (DMSO and acetonitrile or other organic The minimum volume content of the solvent) is ≤1%, and the DMSO content is ≤0.1%. All operations are performed on wet ice.
2. For the blank sample, add the corresponding volume of phosphate buffer solution instead of the working solution of the test substance. For the 0 min sample, first add the stop solution and the liver microsome solution and mix well, and then add the working solution of the test substance.
3. At the end of the incubation, add 400 µL of stop solution to terminate the reaction, and remove the incubation sample from the water bath.
4. After the reaction was terminated, the sample was shaken and centrifuged at a centrifugal force of 4700 g for 10 minutes. After centrifugation, 200 µL of the supernatant was pipetted into a 96-well plate and directly used for LC-MS analysis to analyze metabolites and main drug ratio.

### Test Results:

| **Drug Serial #** | **Drug structure** | **Ratio of main drug after incubation** |
|---|---|---|
| **D-D1** | 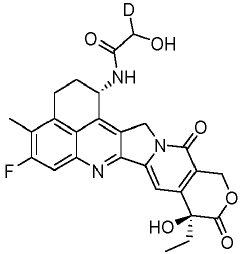 | 98% |
| **D-D3** | 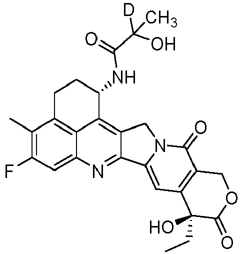 | 97% |
| **D-D5** | 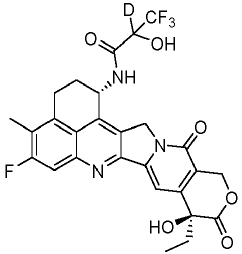 | 99% |
| **D-D7** | 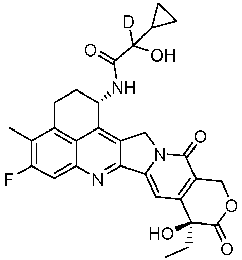 | 96% |
| **D-D11** | 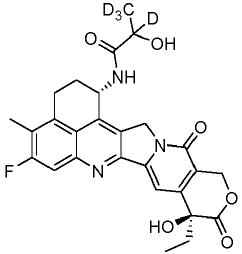 | 96% |
| **D-D15** | 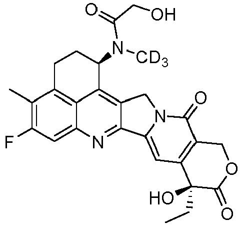 | 91% |
| **D-D19** | 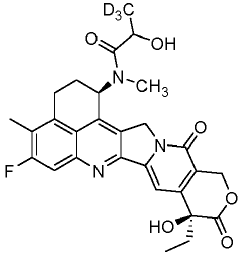 | 97% |
| **2** | 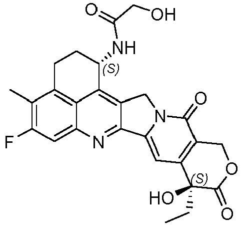 | 65% |
| **3** | 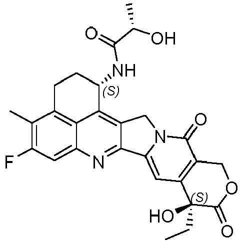 | 56% |
| **5** | 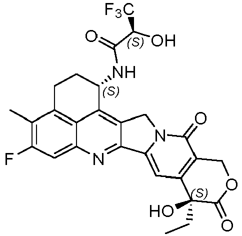 | 73% |
| **7** | 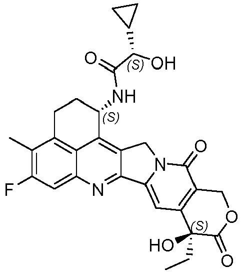 | 85% |

The experimental results show that the metabolic stability of deuterated drugs in liver microparticles is significantly improved.

### Embodiment 37: ADC drug anti-tumor cell activity test

A431, Fadu, Bxpc-3, SW620 and N87 tumor cell lines are used as the research system for in vitro drug efficacy test in the present invention. An appropriate number of tumor cell are uniformly inoculated in a 96-well plate, and incubated in a carbon dioxide incubator. After 24 hours, the cells are confirmed to be in normal state under microscope, and drug dosing treatment is carry out. Dilute the drug with the medium (the initial concentration of ADC drug is 500nM, the dilution factor is 7 times, a total of 8 concentration points is used, the theoretical coupling ratio (DAR) of toxin and antibody is 8 :1, the actual coupling ratio is roughly 7.5:1, so the initial concentration of the toxin is 4.0µM, 7 times concentration gradient dilution, 8 concentration points), mix well and add to the corresponding cell well, wherein the last two columns are respectively Control group (i.e. cells + medium, no drug treatment) and blank group (i.e. no cells, only containing medium, used to subtract the background), put them in a carbon dioxide incubator and incubate at 37°C for 5 days. After 5 days, add 20 µL to each well MTS (Promega, G3581) and react for 2 hours, and the absorbance reading at a wavelength of 490nm was read with a microplate reader (Molecular Device, model: SpectraMAX190). By detecting the activity of dehydrogenase in mitochondria, IC50 was calculated to evaluate the inhibitory effect of ADC drugs on tumor cell proliferation. The results are as follows

| **ADC Serial #** | **Cell Activity (nm)** | | | | |
|---|---|---|---|---|---|
| | **Fadu** | **BXPC-3** | **A431** | **SW620** | **N87** |
| **ADC-1** | 8.03 | 28.12 | 36.91 | 6.22 | 7.37 |
| **ADC-2** | 11.31 | 32.13 | 41.67 | 10.98 | 9.82 |
| **ADC-3** | 3.21 | 10.32 | 22.24 | 2.97 | 4.06 |
| **ADC-4** | 5.89 | 19.32 | 28.77 | 6.43 | 7.89 |
| **ADC-5** | 0.008 | 1.32 | 10.98 | 0.42 | 0.02 |
| **ADC-6** | 0.07 | 21.90 | 6.09 | 1.23 | 1.15 |
| **ADC-7** | 4.01 | 11.22 | 16.35 | 2.24 | 4.52 |
| **ADC-8** | 15.23 | 87.78 | 62.25 | 16.55 | 35.31 |
| **ADC-9** | 13.56 | 98.10 | 48.96 | 14.53 | 26.79 |
| **ADC-10** | 3.44 | 9.85 | 27.72 | 3.01 | 2.46 |
| **ADC-11** | 13.57 | 35.66 | 43.81 | 9.03 | 11.86 |
| **ADC-12** | 18.92 | 49.85 | 56.70 | 15.09 | 13.70 |
| **ADC-13** | 7.81 | 25.72 | 43.16 | 3.71 | 18.32 |
| **ADC-14** | 15.98 | 23.21 | 36.49 | 13.08 | 21.20 |

As demonstrated in the above ADC cell activity test, after the deuterated camptothecin drug of the present invention is coupled with the antibody through the linking unit L, the drug shows great anti-tumor activity in multiple antigen-positive tumor cell lines, and indicates great clinical application value.

### Embodiment 38: ADC Drug Efficacy Test In Vivo

An A431 tumor-bearing nude mouse model was established to evaluate the in vivo efficacy of deuterated camptothecin derivative ADC conjugated drugs and non-deuterated ADC conjugates in the present invention. E.g., 3×10⁶ A431 cells were subcutaneously injected into the right shoulder of BALB/c nude mice aged 4-6 weeks. After the mean tumor volume of the mice grows to 140-150mm³, they are divided into random groups, 5 mice in each group, injected intravenously on days 0, 7, 14, and 21 with Blank control (buffer solution blank) and 10mg/kg dose of antibody drug conjugates ADC-1, ADC-3, ADC-5, ADC-10, ADC-12, ADC-13 and ADC-15. On days 0, 7 , 14, 21, 24, 28, 32, and 36, the tumor volume was measured, and the measurement data are shown as the average tumor volume at the time of measurement. At the same time, the change of the mouse body weight was recorded to observe the preliminary toxicity of the ADC drug in vivo. The results are as follows:

| Type | average body weight (g) | | | | | | | |
|---|---|---|---|---|---|---|---|---|
| | Day 0 | Day7 | Day 14 | Day21 | Day24 | Day28 | Day32 | Day36 |
| Blank Control | 19.82 | 20.41 | 20.57 | 21.13 | 21.94 | 22.44 | - | - |
| ADC-1 | 20.03 | 20.67 | 21.02 | 21.68 | 22.05 | 22.85 | 22.69 | 23.05 |
| ADC-3 | 19.78 | 20.14 | 20.68 | 21.34 | 21.96 | 22.32 | 21.99 | 22.45 |
| ADC-5 | 19.89 | 19.67 | 19.93 | 20.68 | 21.03 | 21.54 | 20.78 | 21.55 |
| ADC-10 | 19.57 | 20.10 | 20.53 | 21.12 | 21.57 | 21.83 | 21.94 | 22.13 |
| ADC-12 | 20.02 | 19.79 | 20.45 | 21.09 | 21.57 | 21.77 | 21.32 | 22.07 |
| ADC-13 | 19.99 | 20.33 | 20.75 | 21.42 | 22.00 | 22.03 | 21.54 | 22.06 |
| ADC-15 | 20.07 | 19.56 | 20.69 | 20.70 | 21.46 | 21.89 | 20.83 | 22.14 |

| type | mean tumor volume (mm³) | | | | | | | |
|---|---|---|---|---|---|---|---|---|
| | Day 0 | Day7 | Day 14 | Day21 | Day24 | Day28 | Day32 | Day36 |
| Blank control | 143.2 | 421.7 | 666.8 | 1421.5 | 1984.5 | 2983.5 | - | - |
| ADC-1 | 148.9 | 217.8 | 137.7 | 81.5 | 49.3 | 52.3 | 58.9 | 72.8 |
| ADC-3 | 157.8 | 209.8 | 127.2 | 92.3 | 33.6 | 35.2 | 43.7 | 69.9 |
| ADC-5 | 145.6 | 201.5 | 117.8 | 86.9 | 49.7 | 48.6 | 54.6 | 89.2 |
| ADC-10 | 150.3 | 208.7 | 121.9 | 99.9 | 60.8 | 57.2 | 62.9 | 93.4 |
| ADC-12 | 153.2 | 213.3 | 166.8 | 118.5 | 89.5 | 93.9 | 135.4 | 199.3 |
| ADC-13 | 158.9 | 217.6 | 137.8 | 111.4 | 90.5 | 98.7 | 156.2 | 231.2 |
| ADC-15 | 146.3 | 206.0 | 158.35 | 123.4 | 109.5 | 120.4 | 189.3 | 321.5 |

After the above drug efficacy experiments in ADC mice, it was proved that the deuterated camptothecin drug disclosed in the present invention showed clear anti-tumor activity in tumor-bearing mice after coupling with the antibody through the linking unit L, and the average tumor body weight was significantly lower than that of the blank control, and the inhibitory effect was obvious. No obvious rebound was observed during the observation period, and the drug effect lasted. There was no significant change in the body weight of the mice during the administration period. Except for the blank control, the body weight of the mice in the group had no significant change , no mice died, and the deuterated camptothecin drug of the present invention are safe.

## Claims

**1.** A deuterated camptothecin derivative shown in Formula D or a pharmaceutically acceptable salt or solvate thereof: 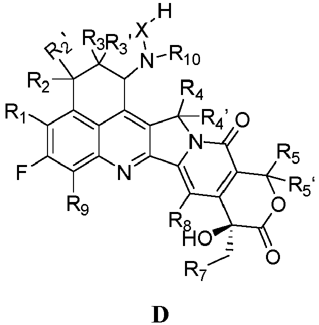 wherein:
R₁, R₇, R₁₀ are independently selected from hydrogen, deuterium, C₁₋₆ alkyl, one or more deuterated C₁₋₆ alkyl, and substituted alkyl;
R₂, R_{2'}, R₃, R_{3'}, R₄, R_{4'}, R₅, R_{5'}, R₈, and R₉ are hydrogen or deuterium;
X is selected from -C(O)-CR^{a}R^{b}-(CR^{c}R^{d})ₙ-O-, -C(O)-CR^{a}R^{b}-(CR^{c}R^{d})ₙ-NH- and -C(O)-CR^{a}R^{b}-(CR^{c}R^{d})ₙ-S-;
R^{a} and R^{b} are independently selected from hydrogen, deuterium, halogen, alkyl, deuterated alkyl, substituted alkyl, cycloalkyl, one or more deuterated cycloalkyl, cycloalkylalkyl, one or more deuterated cycloalkylalkyl, alkoxyalkyl, one or more deuterated alkoxyalkyl, heterocyclyl, aryl, substituted aryl and heteroaryl;
alternatively, R^{a}, R^{b} together with their connected carbon atoms form a C₃₋₆ cycloalkyl group or one or more deuterated C₃₋₆ cycloalkyl, cycloalkylalkyl, one or more deuterated cycloalkylalkyl, heterocyclic group, or one or more deuterated heterocyclic group;
R^{c}, R^{d} are identical or different, and are independently selected from hydrogen, deuterium, halogen, C₁₋₆ alkyl, halogen alkyl, one or more deuterated C₁₋₆ alkyl, alkoxy, one or more deuterated alkoxy, hydroxyl, amino, cyanide, nitro, hydroxyalkyl, cycloalkylalkyl, one or more deuterated cycloalkylalkyl, heterocyclic, and one or more deuterated heterocyclic group;
alternatively, R^{c}, R^{d} together with their connected carbon atoms form a C₃₋₆ cycloalkyl, one or more deuterated C₃₋₆ cycloalkyl, cycloalkyl, one or more deuterated cycloalkyl, heterocyclic group, or one or more deuterated heterocyclic group;
n is an integer from 0-4; and
R₁, R₂, R_{2'}, R₃, R_{3'}, R₄, R_{4'}, R₅, R_{5'}, R₇, R₈, R₉, R₁₀ and X contain at least one deuterium.

**2.** The deuterated camptothecin derivative according to claim 1 or a pharmaceutically acceptable salt or solvate thereof, wherein X is -C(O)-CR^{a}R^{b}-(CR^{c}R^{d})ₙ-O-;
R^{a} is selected from hydrogen, deuterium, alkyl, deuterated alkyl, substituted alkyl, cycloalkyl, one or more deuterium substituted cycloalkyl, cycloalkylalkyl, one or more deuterated cycloalkylalkyl, alkoxyalkyl, one or more deuterated alkoxyalkyl, heterocyclic group, aryl, substituted aryl or heteroaryl;
R^{b} is selected from hydrogen, deuterium, alkyl, deuterated alkyl, substituted alkyl, cycloalkyl, one or more deuterated cycloalkyl, cycloalkylalkyl, one or more deuterated cycloalkylalkyl, alkoxyalkyl, one or more deuterated alkoxyalkyl, heterocyclic group, aryl, substituted aryl or heteroaryl;
alternatively, R^{a}, R^{b} together with their connected carbon atoms form a C₃₋₆ cycloalkyl, one or more deuterated C₃₋₆ cycloalkyl, cycloalkylalkyl, one or more deuterated cycloalkylalkyl or heterocyclic group, or one or more deuterated heterocyclic group;
R^{c}, R^{d} are identical or different, and are independently selected from hydrogen, deuterium, alkyl, one or more deuterated alkyl, alkoxy group, one or more deuterated alkoxy group, hydroxyl, amino, cyano, nitro, hydroxyalkyl, cycloalkyl, or heterocyclic group;
alternatively, R^{c}, R^{d} together with their connected carbon atoms form C₃₋₆ cycloalkyl, one or more deuterated C₃₋₆ cycloalkyl, cycloalkylalkyl, or one or more deuterated cycloalkylalkyl; and
n is 0 or 1.

**3.** The deuterated camptothecin derivative according to claims 1 or 2, or a pharmaceutically acceptable salt or solvate thereof, wherein the deuterated camptothecin derivative comprises the structure shown in Formula D₂: 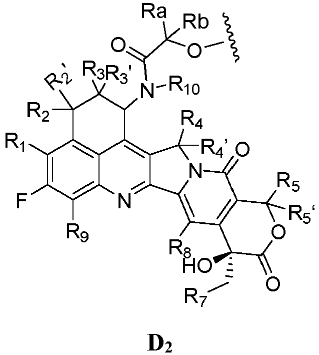 wherein:
R₁₀ is selected from hydrogen, or one or more deuterated C₁₋₆ alkyl;
R₂, R_{2'}, R₃, R_{3'}, R₄, R_{4'}, R₅, R_{5'}, R₈, R₉ are hydrogen or deuterium;
R^{a} is selected from hydrogen, deuterium, alkyl, and one or more deuterated alkyl;
R^{b} is selected from hydrogen, deuterium, alkyl, and one or more deuterated alkyl;
alternatively, R^{a}, R^{b} together with their connected carbon atoms form a C₃₋₆ cycloalkyl, one or more deuterated C₃₋₆ cycloalkyl, cycloalkylalkyl, one or more deuterated cycloalkylalkyl, a heterocyclic group, or one or more deuterated heterocyclic group; and
wherein, the wavy line in Formula **D₂** represents hydrogen, or a covalent linkage to a connector unit or to a ligand unit having a binding affinity to an antigen expressed on a target cell.

**4.** The deuterated camptothecin derivative according to claim 1 or a pharmaceutically acceptable salt or solvate thereof, wherein X comprises: 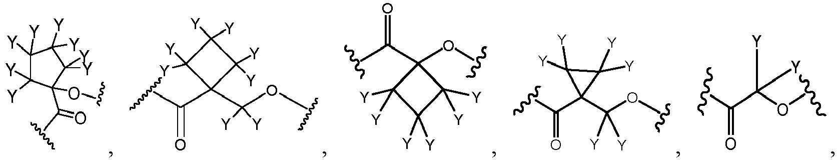 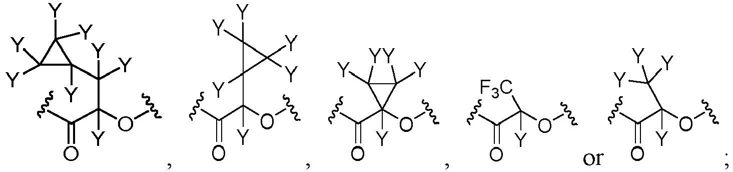 wherein Y is hydrogen or deuterium.

**5.** The deuterated camptothecin derivative according to claim 1 or a pharmaceutically acceptable salt or solvate thereof, further comprising its tautomer, racemate, diastereomer, or a mixture thereof.

**6.** The deuterated camptothecin derivative according to claim 1 or a pharmaceutically acceptable salt or solvate thereof, further comprising the following compounds or their pharmaceutically accepted salts or solvates thereof.

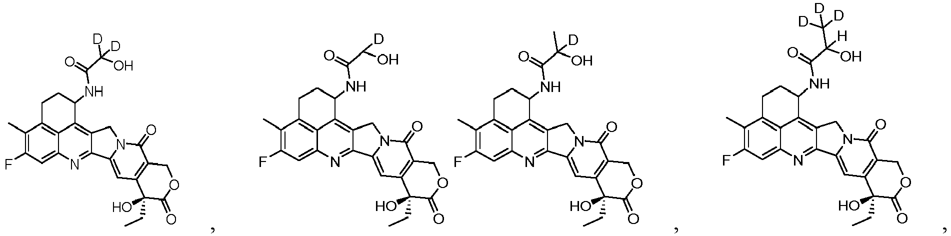

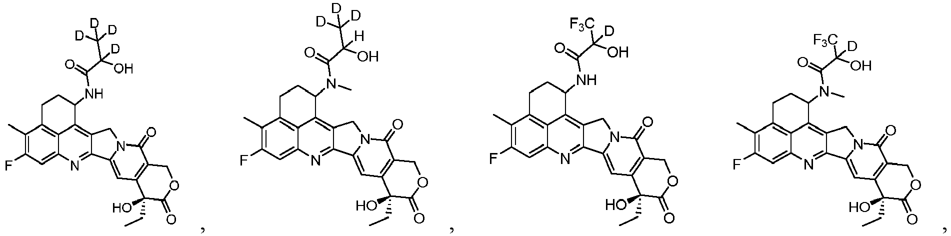

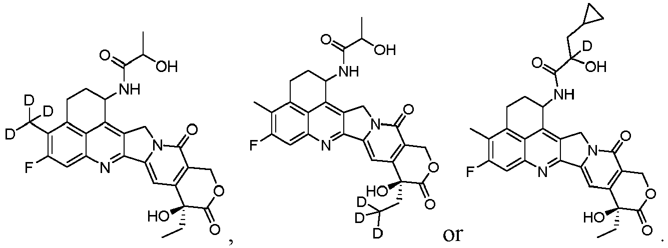

**7.** A drug-linker compound as shown in the Formula -L-X-D2, or a pharmaceutically acceptable salt or solvate thereof, 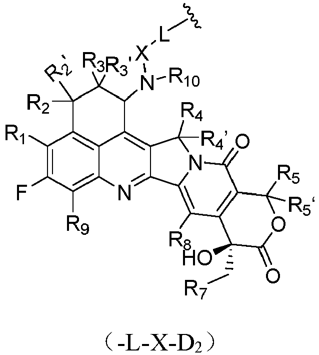 wherein:
R₁, R₇, R₁₀ are selected from hydrogen, deuterium, one or more deuterated C₁₋₆ alkyls, C₁₋₆ alkyl, substituted alkyl, aryl, one or more deuterated aryl, heteroaryl, and one or more deuterated heteroaryl ;
R₂, R_{2'}, R₃, R_{3'}, R₄, R_{4'}, R₅, R_{5'}, R₈, R₉ are hydrogen or deuterium;
X is -C(O)-CR^{a}R^{b}-(CR^{c}R^{d})ₙ-O-;
R^{a}, R^{b} are independently selected from hydrogen, deuterium, halogen, alkyl, deuterated alkyl, substituted alkyl, cycloalkyl, one or more deuterated cycloalkyl, cycloalkylalkyl, one or more deuterated cycloalkylalkyl, alkoxy alkyl, one or more deuterated alkoxy alkyl, heterocyclic group, one or more deuterated heterocyclic, aryl, one or more deuterated aryl, substituted aryl, heteroaryl, and one or more deuterated heteroaryl;
alternatively, R^{a}, R^{b} together with their connected carbon atoms form a C₃₋₆ cycloalkyl, one or more deuterated C₃₋₆ cycloalkyl, cycloalkylalkyl, one or more deuterated cycloalkylalkyl, heterocyclic, or one or more deuterated heterocyclic group;
R^{c}, R^{d} are identical or different, and independently selected from hydrogen, deuterium, halogen, alkyl, haloalkyl, C₁₋₆ alkyl, one or more deuterated or fully deuterated C₁₋₆ alkyl, alkoxy, one or more deuterated alkoxy, hydroxyl, amino, cyano, nitro, hydroxyalkyl, cycloalkyl, one or more deuterated cycloalkyl, heterocyclic, and one or more deuterated heterocyclic;
alternatively, R^{c}, R^{d} together with their connected carbon atoms form a C₃₋₆ cycloalkyl, one or more deuterated C₃₋₆ cycloalkyl, cycloalkylalkyl, one or more deuterated cycloalkylalkyl, heterocyclic, and one or more deuterated heterocyclic;
n is an integer from 0-4;
R₁, R₂, R_{2'}, R₃, R_{3'}, R₄, R_{4'}, R₅, R_{5'}, R₇, R₈, R₉, R₁₀ and X contain at least one deuterium;
L is a linker unit; and
wherein the wavy line in Formula -L-X-D₂ represents hydrogen or a covalent linkage to a connector unit or to an antibody having binding affinity to an antigen expressed by target cells.

**8.** The drug-linker compound according to claim 7 or pharmaceutically acceptable salt or solvate thereof, wherein the O-terminal of the X is connected to the linker unit L.

**9.** The drug-linker compound according to claims 7 or 8 or a pharmaceutically acceptable salt or solvate thereof, wherein: the linker unit L is -L₁-L₂-L₃-L₄-, wherein the L₁ end is connected to an antibody and the L₄ end is connected to the X;
wherein:
L₁ is selected from -(Succinimido-3-yl-*N*)-Y-C(O)-, -CH₂-C(O)-NR⁵-Y-C(O)- or -C(O)-Y-C(O)-;
wherein Y is selected from C₁₋₈ alkyl, C₁₋₈ alkyl-cycloalkyl or 1-8 atoms linear or linear-cyclic heteralkyl; wherein the heteroalkyl comprises 1-3 atoms selected from N, O or S, and wherein the C₁₋₈ alkyl, cycloalkyl, linear or linear-cyclic heteralkyl are independently substituted by one or more substituents selected from deuterium, halogen, hydroxyl, cyano, nitro, amino, alkyl, carboxy, heteralkyl, substituted alkyl, alkoxy or cycloalkyl;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or a chemical bond, wherein p is an integer from 0-20;
L₃ is a peptide residue having 2-7 amino acids, wherein the amino acids are further substituted by one or more substituents selected from deuterium, halogen, hydroxyl, cyano, amino, nitro, alkyl, substituted alkyl, alkoxy, cycloalkyl and substituted cycloalkyl;
L₄ is selected from -NR⁷(CR⁸R⁹)_{q}-, -C(O)NR⁷, -C(O)NR⁷(CH₂)_{q}- or chemical bond, wherein q is an integer from 0-6;
R⁵, R⁶ and R⁷ are identical or different, and are independently selected from hydrogen, deuterium, halogen, alkyl, deuterated alkyl, haloalkyl, cycloalkyl, cycloalkylalkyl, alkoxyalkyl, heterocyclic, aryl, substituted aryl or heteroaryl; and
R⁸ and R⁹ are identical or different, and are independently selected from hydrogen, deuterium, halogen, alkyl, deuterated alkyl, haloalkyl, cycloalkyl, cycloalkylalkyl, alkoxyalkyl, heterocyclic, aryl, substituted aryl or heteroaryl.

**10.** The drug-linker compound according to claim 9 or a pharmaceutically acceptable salt or solvent thereof, wherein:
L₁ is selected from -(Succinimido-3-yl-*N*)-Y-C(O)-, -CH₂-C(O)-NR⁵-Y-C(O)- or -C(O)-Y-C(O)-;
wherein Y is selected from C₁₋₈ alkyl, C₁₋₈ alkyl-cycloalkyl or 1-8 atom linear or linear-cyclic heteralkyl; wherein the heteroalkyl comprises 1-3 atoms selected from N, O or S, and the C₁₋₈ alkyl, cycloalkyl, linear or linear-cyclic heteralkyl are substituted by one or more substituents selected independently from deuterated, halogen, hydroxyl, cyano, nitro, amino, alkyl, carboxy, heteralkyl, substituted alkyl, alkoxy and cycloalkyl;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or chemical bond, wherein p is an integer from 0-20;
L₃ is a peptide residue comprising phenylalanine (F), glycine (G), valine (V), lysine (K), citrulline, serine(S), glutamic acid (E) or aspartic acid (D);
L₄ is -NR⁷CR⁸R⁹-;
R⁵, R⁶, R⁷ are identical or different, and are independently selected from hydrogen, deuterium, halogen, alkyl, deuterated alkyl, haloalkyl, cycloalkyl, cycloalkylalkyl, alkoxyalkyl, heterocyclic, aryl, substituted aryl and heteroaryl;
R⁸, R⁹ are identical or different, and are independently selected from hydrogen, deuterium, halogen, alkyl, deuterated alkyl, haloalkyl, cycloalkyl, cycloalkylalkyl, alkoxyalkyl, heterocyclic, aryl, substituted aryl and heteroaryl.

**11.** The drug-linker compound according to claim 9 or a pharmaceutically acceptable salt or solvent thereof, wherein L3 is preferably a peptide comprising one, two or more amino acids selected from phenylalanine and glycine; the most preferred is a tetrapeptide residue comprising glycine-glycine-phenylalanine-glycine.

**12.** The drug-linker compound according to claim 9 or a pharmaceutically acceptable salt or solvent thereof, wherein L4 is preferably -NHCH₂-.

**13.** A drug-linker compound having a general formula L-X-D₂, or a pharmaceutically acceptable salt or solvate thereof having the following formula; 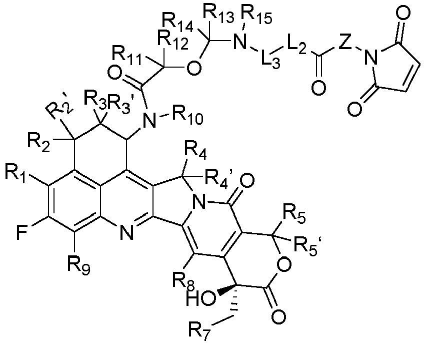 wherein:
Z is selected from -Y-C(O)-, -CH₂-C(O)-NR⁵-Y-C(O)- or -C(O)-Y-C(O)-, wherein Y comprises Ci-s alkyl, Ci-s alkyl-cycloalkyl or 1-8 atoms of linear or linear-cyclic heteralkyl, wherein the heteralkyl comprises 1-3 atoms selected from N, O or S, and the C₁₋₈ alkyl, cycloalkyl, linear or linear-cyclic heteralkyl is independently substituted by one or more substituents selected from deuterium, halogen, hydroxyl, cyano, nitro, amino, alkyl, heteralkyl, substituted alkyl, alkoxy, carboxyl or cycloalkyl;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or chemical bond, wherein p is an integer from 0-20, wherein the alkyl group in the structure is substituted by one or more deuterium;
L₃ is a peptide residue having 2-7 amino acids, wherein the amino acids are further substituted by one or more substituents selected from deuterium, halogen, hydroxyl, cyano, amino, nitro, alkyl, substituted alkyl, alkoxy, cycloalkyl and substituted cycloalkyl;
R is selected from deuterium, C₁₋₆ alkyl, substituted alkyl, aryl, substituted aryl or heteroaryl;
R₁₁ is selected from hydrogen, deuterium, halogen, alkyl, deuterated alkyl, haloalkyl, cycloalkyl, cycloalkylalkyl, heterocyclic, aryl, substituted aryl or heteroaryl;
R₁₂ is selected from hydrogen, deuterium, halogen, alkyl, haloalkyl, deuterated alkyl, cycloalkyl, cycloalkylalkyl, alkoxyalkyl, heterocyclic, aryl, substituted aryl or heteroaryl;
alternatively, R₁₁, R₁₂ together with their connected carbon atoms form C₃₋₆ cycloalkyl, cycloalkylalkyl or heterocyclic group;
R⁵ and R⁶ are selected from hydrogen, deuterium, halogen, alkyl, deuterated alkyl, haloalkyl, cycloalkyl, cycloalkylalkyl, alkoxyalkyl, heterocyclic group, aryl, substituted aryl or heteroaryl;
R₁, R₇, R₁₀, R₁₅ are selected from hydrogen, alkyl, or one or more deuterated C₁₋₄ alkyl;
R₂, R_{2'}, R₃, R_{3'}, R₄, R_{4'}, R₅, R_{5'}, R₈, R₉ are hydrogen or deuterium; and
R₁, R₂, R_{2'}, R₃, R_{3'}, R₄, R_{4'}, R₅, R_{5'}, R₇, R₈, R₉, R₁₀, R₁₁ and R₁₂ contain at least one deuterium.

**14.** The compound having a general formula (L-X-D₂) according to claim 13, or its pharmaceutically acceptable salt or solvate, comprising a compound of Formula (L_{b}-X-D₂), or a pharmaceutically acceptable salt or solvate thereof; 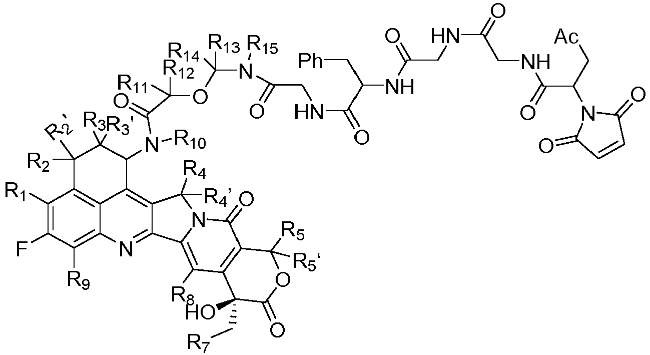 L_{b}-X-D₂
wherein:
R₁₁, R₁₂ are independently selected from hydrogen, deuterium, halogens, alkyl, deuterated alkyl, substituted alkyl, cycloalkyl, one or more deuterated cycloalkyl, cycloalkylalkyl, one or more deuterated cycloalkylalkyl, heterocyclic, one or more deuterated heterocyclic, aryl, one or more deuterated aryl, substituted aryl, heteroaryl, or one or more deuterated heteroaryl;
alternatively, R₁₁, R₁₂ together with the carbon atoms connected thereto form a C₃₋₆ cycloalkyl, one or more deuterated C₃₋₆ cycloalkyl, cycloalkylalkyl, one or more deuterated cycloalkylalkyl, heterocyclic group, or one or more deuterated heterocyclic group;
R₁, R₇, R₁₀, R₁₅ are selected from hydrogen, alkyl, one or more deuterated or fully deuterated C₁₋₄ alkyl or substituted alkyl;
R₂, R_{2'}, R₃, R_{3'}, R₄, R_{4'}, R₅, R_{5'}, R₈, and R₉ are selected from hydrogen or deuterium; and
Ac is a hydrophilic unit having the structure of Formula c, containing an amino group and a carboxyl group, X is a scaffold connecting the amino group and the carboxyl group, X is a C₁₋₁₀ hydrocarbylene or substituted hydrocarbylene, wherein the hydrocarbylene or substituted hydrocarbylene are substituted with one or more s deuterium; Ac is connected to the 2-position methylene carbon marked in the Formula L_{b}-X-D₂ through an amino functional group.

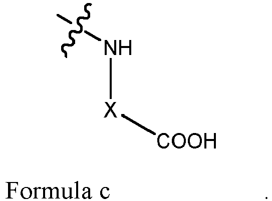

**15.** The compound having a general formula (L-X-D₂) according to claim 14, or its pharmaceutically acceptable salt or solvate, wherein Ac is glycine, α-alanine, β-alanine, or (D/L) glutamic acid.

**16.** The compound having a general formula (L-X-D₂) according to claim 13, or its pharmaceutically acceptable salt or solvate, further comprise a structure selected from: 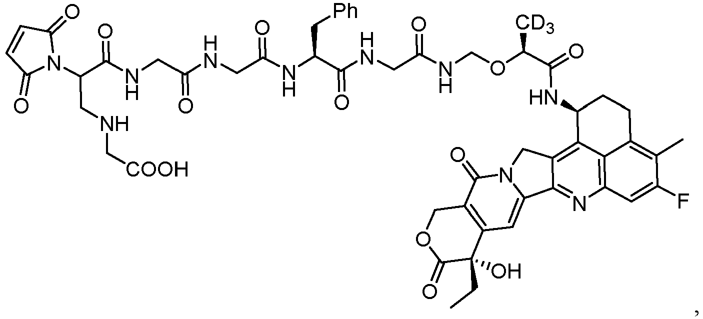 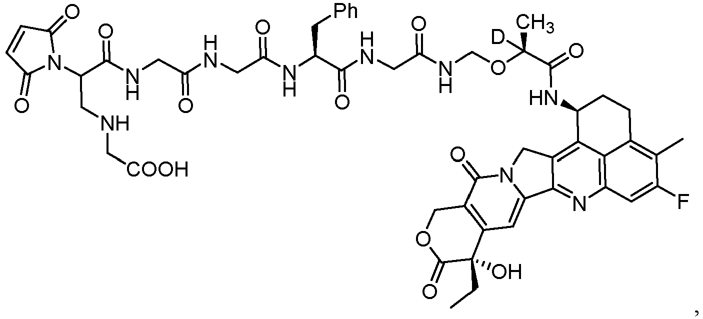 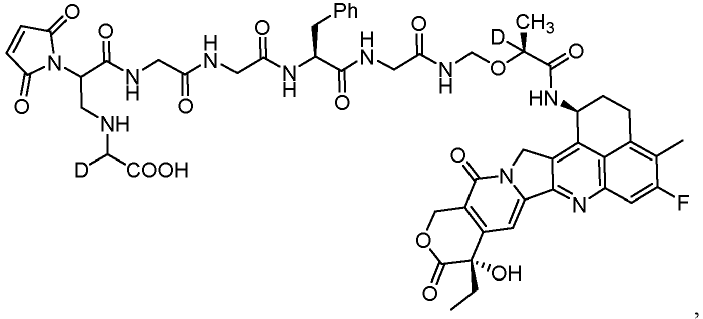 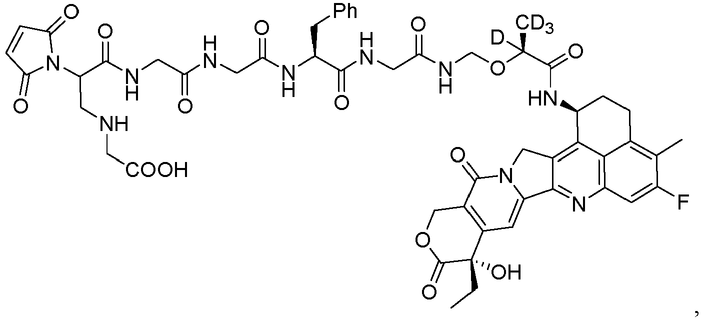 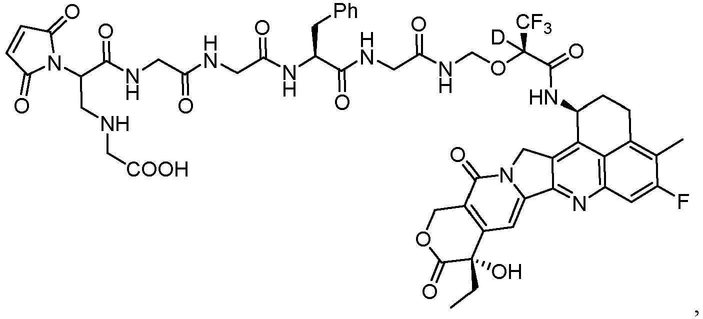 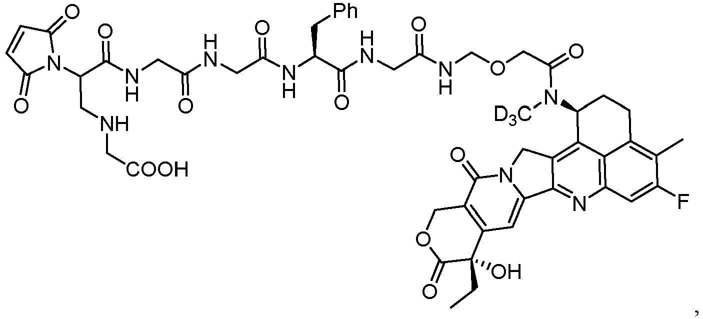 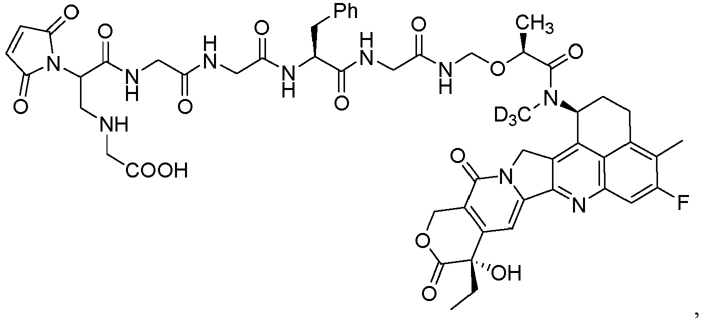 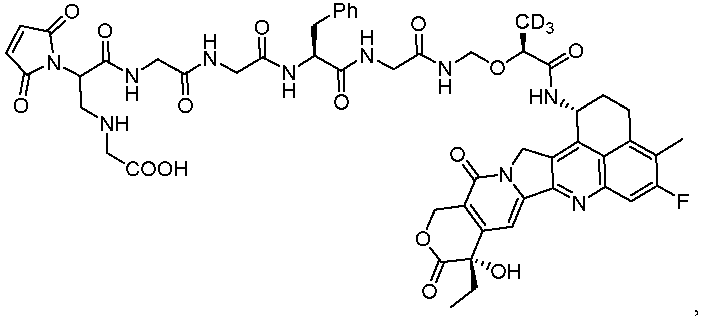 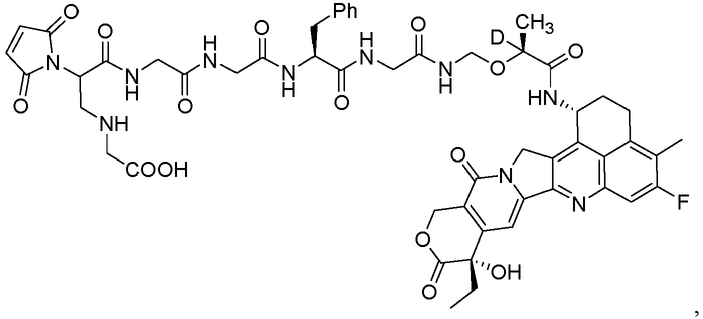 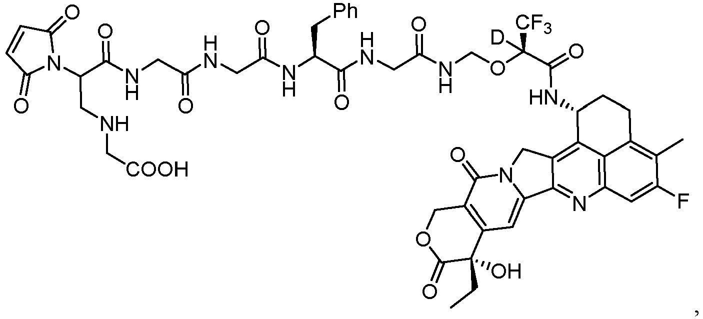 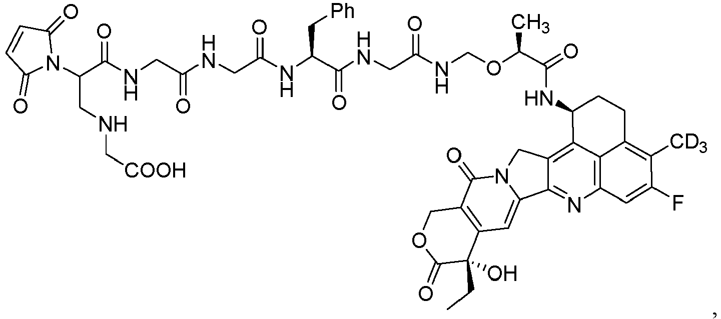 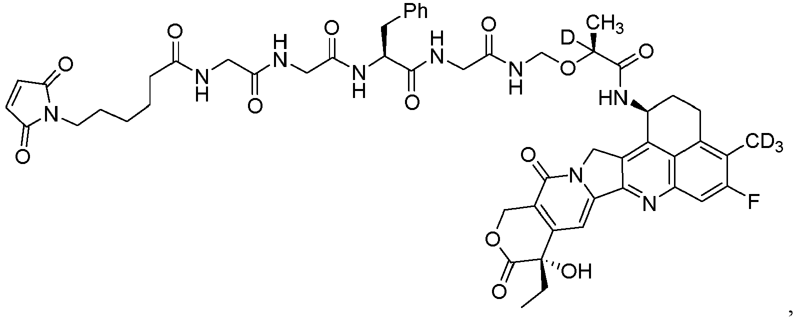 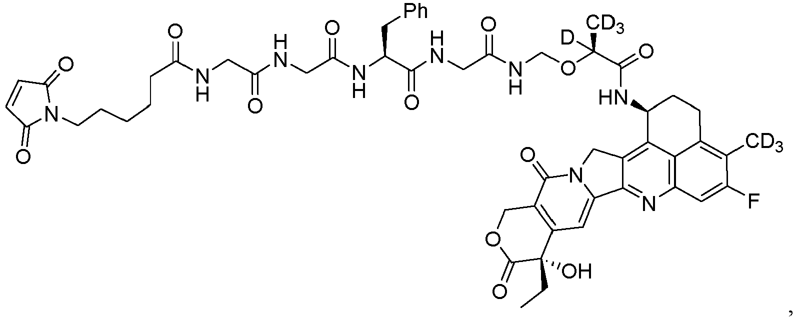 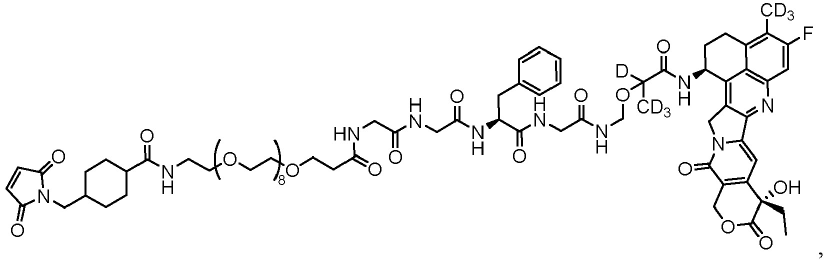 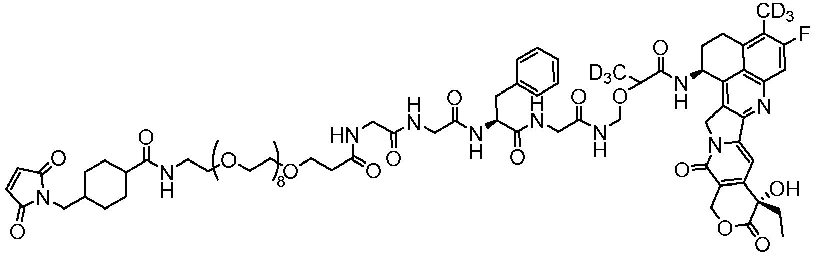 or

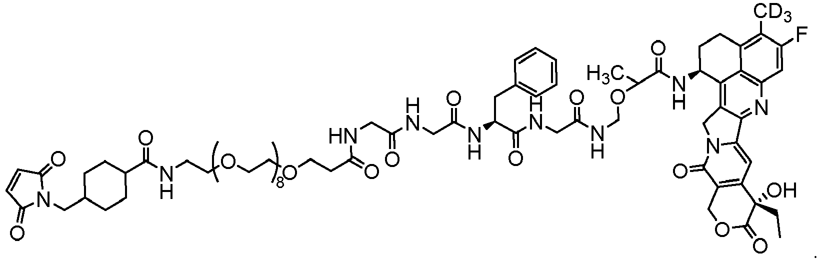

**16.** An antibody-drug conjugate having the Formula (Ab-L-X-Dr) or a pharmaceutically acceptable salt or solvate thereof, comprising the deuterated camptothecin derivatives or camptothecin-linker compounds according to claims 1-15 or their tautomer, mesomer, racemate, enantiomer, diastereoisomer or a mixture thereof, connected to a ligand unit to form the said antibody-drug conjugate, 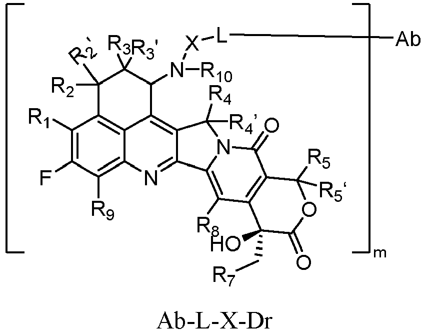 wherein:
R₁, R₇, R₁₀ are selected from hydrogen, deuterium, one or more deuterated C₁₋₆ alkyls, C₁₋₆ alkyl, substituted alkyl, aryl, one or more deuterated aryl, heteroaryl, or one or more deuterated heteroaryl;
R₂, R_{2'}, R₃, R_{3'}, R₄, R_{4'}, R₅, R_{5'}, R₈, R₉ are hydrogen or deuterium;
X is -C(O)-CR^{a}R^{b}-(CR^{c}R^{d})ₙ-O-;
R^{a}, R^{b} are independently selected from hydrogen, deuterium, halogens, alkyl, deuterated alkyl, substituted alkyl, cycloalkyl, one or more deuterium substituted cycloalkyl, cycloalkylalkyl, one or more deuterium substituted cycloalkylalkyl, alkoxyalkyl, one or more deuterium substituted alkoxyalkyl, heterocyclic, aryl, substituted aryl and heteroaryl;
alternatively, R^{a}, R^{b} together with the carbon atoms connected thereto form a C₃₋₆ cycloalkyl, or one or more deuterium atom substituted C₃₋₆ cycloalkyl, cycloalkylalkyl, one or more deuterated cycloalkylalkyl, heterocyclic group, or one or more deuterated heterocyclic group;
R^{c}, R^{d} are identical or different, and are independently selected from hydrogen, deuterium, halogen, C₁₋₆ alkyl, halogen alkyl, one or more deuterated C₁₋₆ alkyl, alkoxy, one or more deuterated alkoxy, hydroxyl, amino, cyanide, nitro, hydroxyalkyl, cycloalkyl, one or more deuterated cycloalkyl, heterocyclic, and one or more deuterated heterocyclic group;
alternatively, R^{c}, R^{d} together with their connected carbon atoms form C₃₋₆ cycloalkyl, or one or more deuterated C₃₋₆ cycloalkyl, cycloalkylalkyl, one or more deuterated cycloalkylalkyl, heterocyclic group, and one or more deuterated heterocyclic group;
n is an integer from 0-4;
L is a linker unit; and
wherein the wavy line in Formula -L-X-D₂ represents hydrogen, or a covalent linkage to a connector unit or an antibody having binding affinity to an antigen expressed on target cells.

**17.** The antibody-drug conjugate according to claim 16 or a pharmaceutically acceptable salt or solvate thereof, wherein the linker unit -L- comprises -L₁-L₂-L₃-L₄-, wherein the L₁ end is connected to an antibody and the L₄ end is connected to the X;
wherein:
L₁ is selected from -(Succinimido-3-yl-*N*)-Y-C(O)-, -CH₂-C(O)-NR⁵-Y-C(O)- or -C(O)-Y-C(O)-,
wherein Y is selected from C₁₋₈ alkyl, C₁₋₈ alkyl-cycloalkyl or 1-8 atom linear or linear-cyclic heteralkyl; wherein the heteroalkyl comprises 1-3 atoms selected from N, O or S, and wherein the C₁₋₈ alkyl, cycloalkyl, linear or linear-cyclic heteralkyl are independently substituted by one or more substituents selected from deuterated, halogen, hydroxyl, cyano, nitro, amino, alkyl, carboxy, heteralkyl, substituted alkyl, alkoxy and cycloalkyl;
L₂ is selected from -NR⁶(CH₂CH₂O)ₚCH₂CH₂C(O)-, -NR⁶(CH₂CH₂O)ₚCH₂C(O)-, - S(CH₂)ₚC(O)- or a chemical bond, wherein p is an integer from 0-20;
L₃ comprises a peptide residue comprising 2-7 amino acids, wherein the amino acids are optionally further substituted by one or more substituents selected from deuterium, halogen, hydroxyl, cyano, amino, nitro, alkyl, substituted alkyl, alkoxy, cycloalkyl or substituted cycloalkyl;
L₄ is selected from -NR⁷(CR⁸R⁹)_{q}-, -C(O)NR⁷, -C(O)NR⁷(CH₂)_{q}- or chemical bond, and q is an integer from 0-6;
R⁵, R⁶ and R⁷ are identical or different, and are independently selected from hydrogen, deuterium, halogens, alkyl, deuterated alkyl, haloalkyl, cycloalkyl, cycloalkylalkyl, alkoxyalkyl, heterocyclic group, aryl, substituted aryl or heteroaryl group;
R⁸ and R⁹ are identical or different, and are independently selected from hydrogen, deuterium, halogens, alkyl, deuterated alkyl, haloalkyl, cycloalkyl, cycloalkylalkyl, alkoxyalkyl, heterocyclic, aryl, substituted aryl or heteroaryl.
m is an integer or a decimal between 1-10;
Ab comprises an antibody, antibody fragment, a target protein or Fc-fusion protein; and
L is a linker unit.

**18.** The antibody-drug conjugate according to claim 17 or a pharmaceutically acceptable salt or solvate thereof, wherein: Ab is an antibody linked to a linker unit through its heteroatom, the antibody is selected from murine antibodies, chimeric antibodies, humanized antibodies, fully human antibodies, antibody fragments, bispecific antibodies and multispecific antibodies.

**19.** The antibody-drug conjugate or its pharmaceutically acceptable salt or solvate according to claims 17 or 18, wherein the antibody is an antibody targeting one or more of the following targets: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody or anti-CD123 antibody, and anti-CD47 antibody.

**20.** The antibody-drug conjugate according to claims 17 or 18 or a pharmaceutically acceptable salt or solvate thereof, comprising one of the following structures: 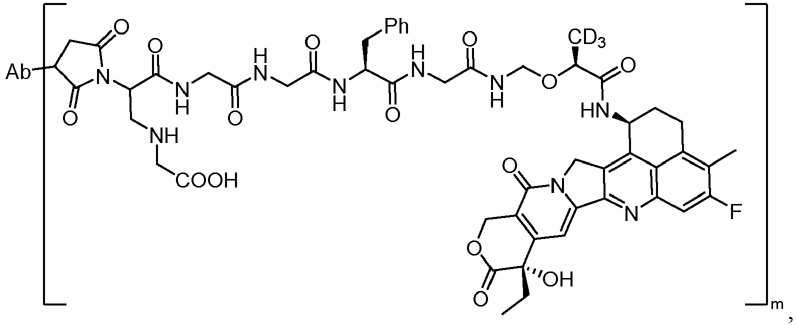 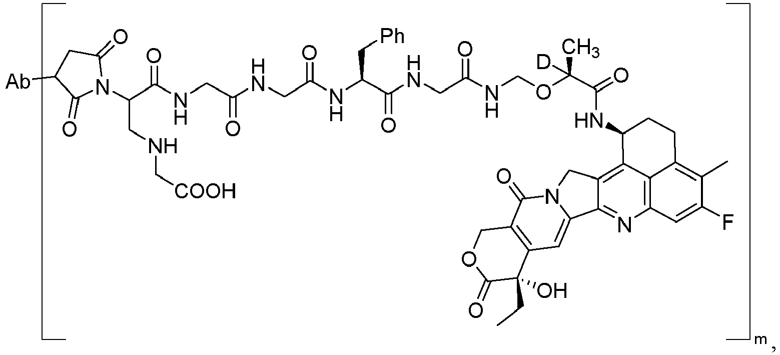 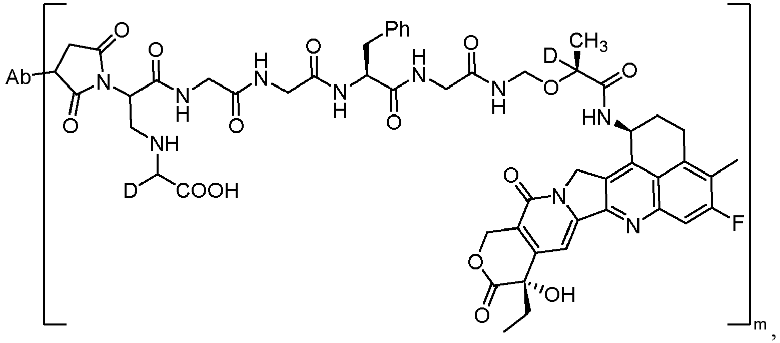 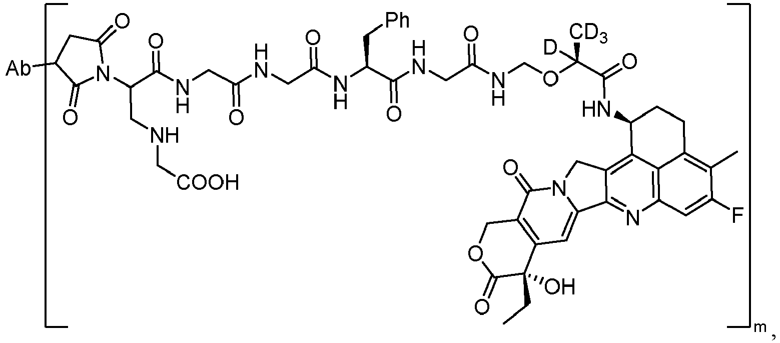 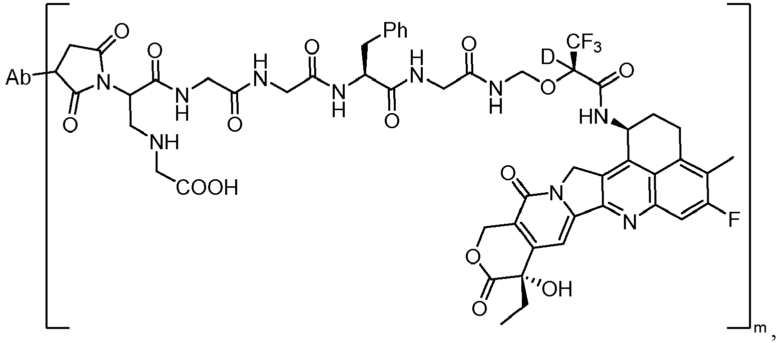 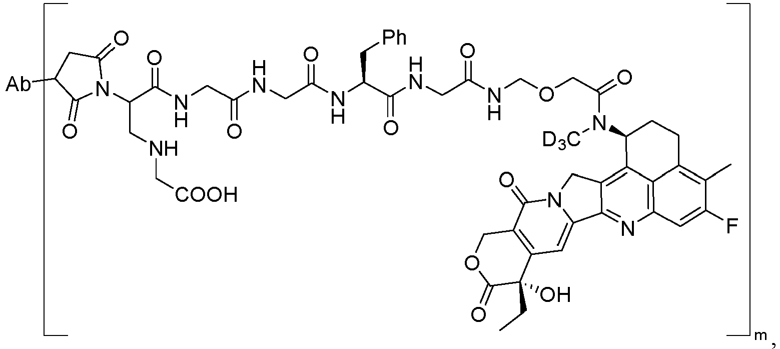 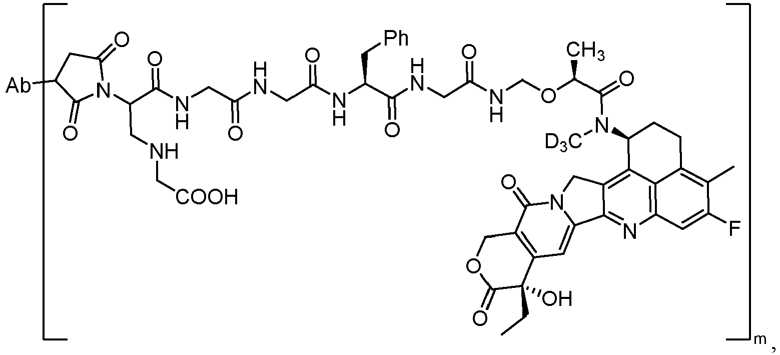 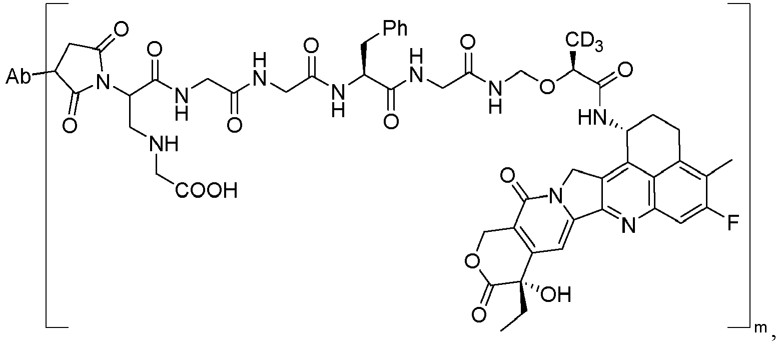 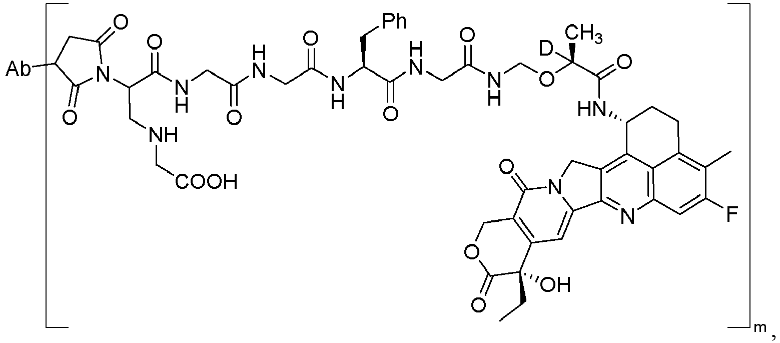 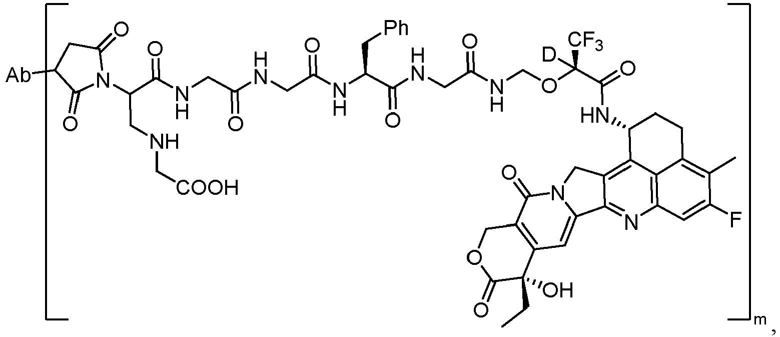 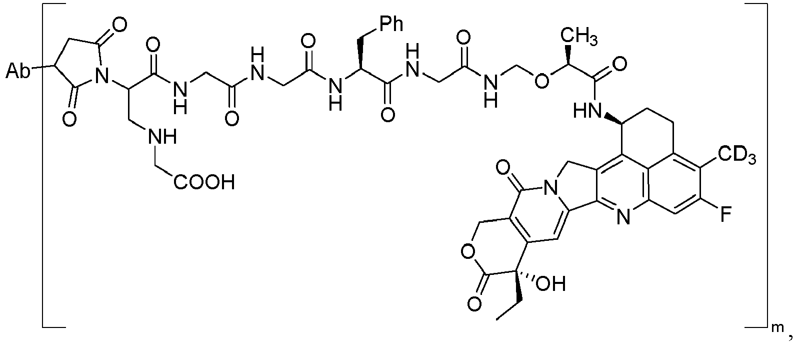 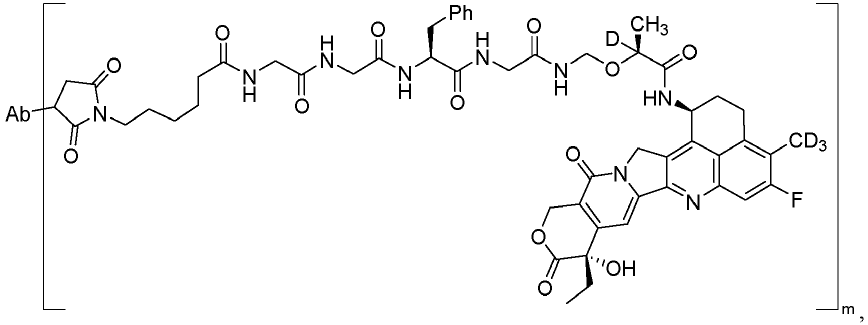 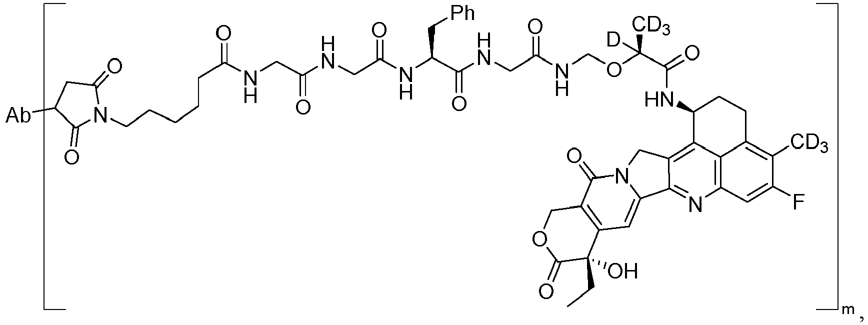 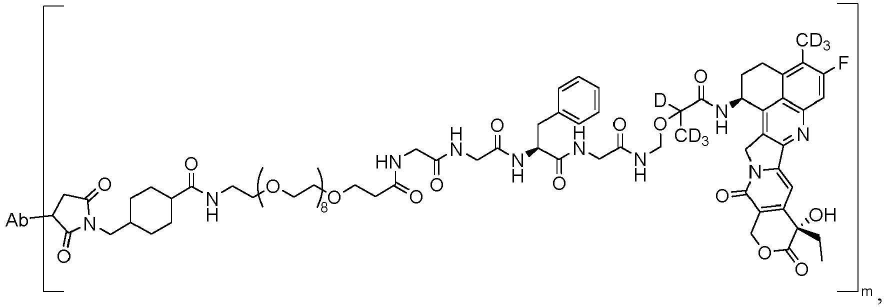 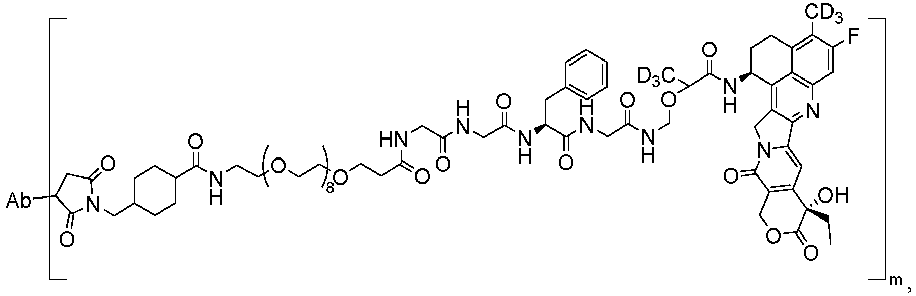 or 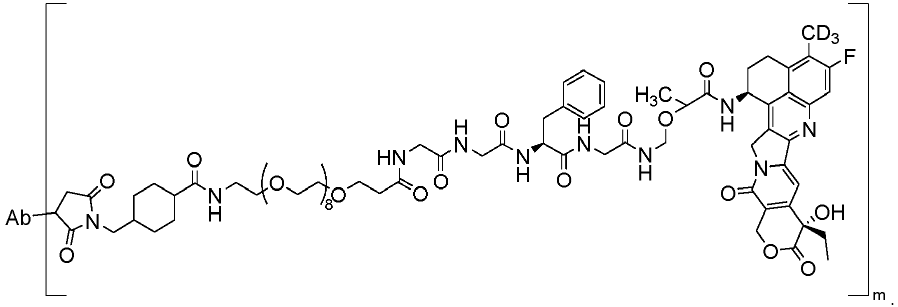 wherein:
m is an integers or a decimal between 1-10; and
Ab is an antibody, antibody fragment, a target protein, or a Fc-fusion protein.

**21.** A method for preparing the compound of claim 14 (L-X-D₂), comprising the following steps:

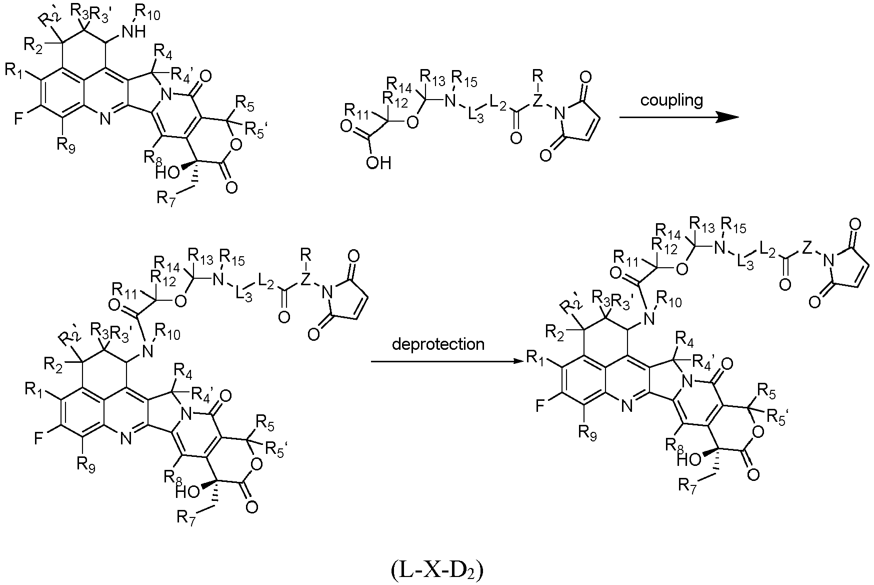

**22.** A method for preparing an antibody-drug conjugate of Formula (Ab-Lₐ-X-Dr) according to claims 17-20 or a pharmaceutically acceptable salt or solvate thereof, comprising the following steps: 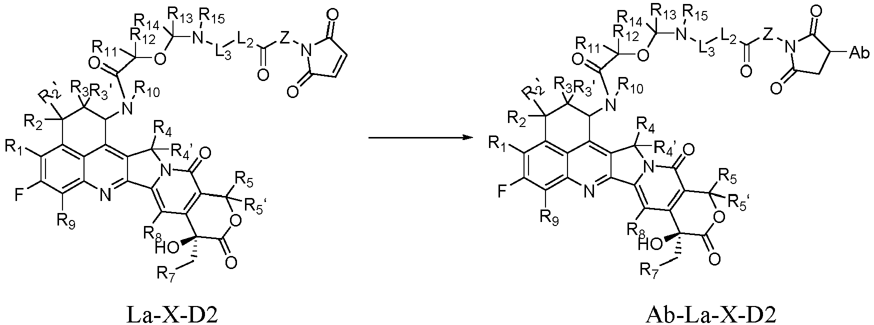 generating a compound of Formula Ab-Lₐ-X-D₂ by conjugating antibodies, antibody fragments, target proteins, or Fc-fusion proteins, to a compound of Formula (La-X-D₂).

**23.** A pharmaceutical composition comprising a therapeutically effective amount of the antibody-drug conjugate or a pharmaceutically acceptable salt or solvate thereof according to claims 1-20, and a pharmaceutically acceptable carrier, diluent or excipient.

**24.** The use, for purpose of preparing drugs for the treatment or prevention of tumor, of the deuterated camptothecin derivative or antibody-drug conjugate of claims 1-20, or their tautomers, mesomers, racemates, enantiomers, diastereoisomers or a mixture thereof, or a pharmaceutically acceptable salt or solvent compound thereof, and a pharmaceutically acceptable carrier, diluent, or excipient.

**25.** The use according to claim 24, wherein the tumor comprise solid tumor and hematological tumor.

**26.** The use according to claim 25, wherein the tumor comprises breast cancer, ovarian cancer, cervical cancer, uterine cancer, prostate cancer, kidney cancer, urethral cancer, bladder cancer, liver cancer, stomach cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma, sarcoma, lymphoma or leukemia
